# EUROPEAN PATENT APPLICATION

(11) **EP 1 388 586 A2**
(43) Date of publication of application: **11.02.2004**
(21) Application number: 03018217.4
(22) Date of filing: 28.05.1999
(51) Int. Cl.: C12N 7/00, A61K 39/42, A61K 39/395, A61K 39/21, C07K 16/00

(54) **Identification of broadly reactive DR restricted epitopes**

(30) Priority: 29.05.1998 US 87192 P
(62) Divisional of application: 99926084.7
(71) Applicant: Epimmune Inc., San Diego, California 92121 (US)
(72) Inventor: Sette, Alessandro, La Jolla, CA 92037 (US); Southwood, Scott, Santee, CA 92071 (US); Sidney, John, San Diego, CA 92130 (US)
(74) Representative: Cripps, Joanna Elizabeth

(57) **Abstract**

The present invention is based, at least in part, on the discovery and validation of specific immunogenic peptide epitopes for various HLA class II DR molecules, representative of the worldwide population. Such peptides comprise an epitope, or analog thereof, which binds to an HLA class II molecule at an IC₅₀ of less than or equal to 1,000 nM.

## Description

### CROSS-REFERENCE TO RELATED APPLICATIONS

The present application claims the benefit of Provisional U.S.S.N. 60/087,192 filed 5/29/98. The application is also related to U.S.S.N. 09/009953, filed January 21, 1998, U.S.S.N. 60/036,713, filed January 23, 1997, and U.S.S.N. 60/037,432 filed February 7, 1997.

### BACKGROUND OF THE INVENTION

Helper T lymphocytes (HTL) play several important functions in immunity to pathogens. Firstly, they provide help for induction of both CTL and antibody responses. By both direct contact and by secreting lymphokines such as IL2 and IL4, HTL promote and support the expansion and differentiation of T and B cell precursors into effector cells. In addition, HTL can also be effectors in their own right, an activity also mediated by direct cell contact and secretion of lymphokines, such as IFNγ and TNFα. HTL have been shown to have direct effector activity in case of tumors, as well as viral, bacterial, parasitic, and fungal infections.

HTL recognize a complex formed between class II MHC molecules and antigenic peptides, usually between 10 and 20 residues long, and with an average size of between 13 and 16 amino acids. Peptide-class II interactions have been analyzed in detail, both at the structural and functional level, and peptide motifs specific for various human and mouse class II molecules have been proposed.

In the last few years, epitope based vaccines have received considerable attention as a possible mean to develop novel prophylactic vaccines and immunotherapeutic strategies. Selection of appropriate T and B cell epitopes should allow to focus the immune system toward conserved epitopes of pathogens which are characterized by high sequence variability (such as HIV, HCV and Malaria).

In addition, focusing the immune response towards selected determinants could be of value in the case of various chronic viral diseases and cancer, where T cells directed against the immunodominant epitopes might have been inactivated while T cells specific for subdominant epitopes might have escaped T cell tolerance. The use of epitope based vaccines also allows to avoid "suppressive" T cell determinants which induce TH₂ responses, in conditions where a TH₁ response is desirable, or vice versa.

Finally, epitope based vaccines also offer the opportunity to include in the vaccine construct epitopes that have been engineered to modulate their potency, either by increasing MHC binding affinity, or by alteration of its TCR contact residues, or both. Inclusion of completely synthetic non-natural or generically unrelated to the pathogen epitopes (such as TT derived "universal" epitopes), also represents a possible mean of modulating the HTL response toward a TH₁, or TH₂ phenotype.

Once appropriate epitope determinants have been defined, they can be assorted and delivered by various means, which include lipopeptides, viral delivery vectors, particles of viral or synthetic origin, naked or particle absorbed cDNA.

However, before appropriate epitopes can be defined, one major obstacle has to be overcome, namely the very high degree of polymorphism of the MHC molecules expressed in the human population. In fact, more than two hundred different types of HLA class I and class II molecules have already been identified. It has been demonstrated that in the case of HLA class I molecules, peptides capable of binding several different HLA class I molecules can be identified. Over 60% of the known HLA class I molecules can, in fact, be grouped in four broad HLA supertypes, characterized by similar peptide binding specificities (HLA supermotifs).

In the case of class II molecules, it is also known that peptides capable of binding multiple HLA types and of being immunogenic in the context of different HLA molecules do indeed exist. Specific immunogenic peptide have not been readily identified, particularly those reaactive with a large number of allelic products.

The present invention addresses these and other needs.

### SUMMARY OF THE INVENTION

The present invention is based, at least in part, on the discovery and validation of specific immunogenic peptide epitopes for various HLA class II DR molecules, representative of the worldwide population. Such peptides comprise an epitope, or analog thereof, which binds to an HLA class II molecule at an IC₅₀ of less than or equal to 1,000 nM. Epitopes of the invention have been identified in a variety of antigens including tumor associated antigens such as carcinoembryonic antigen (CEA), p53, MAGE-2, MAGE-3, or Her2/neu; viral antigens such as those from HIV, HBV, or HCV; and parasites such as *Plasmodium falciparum.*

The HLA class II binding peptides of the invention may further comprise an epitope having an amino acid that is Y, F, W, L, I, V, or M at the first position from the N-terminus of the epitope and an amino acid of S, T, C, A, P, V, I, L, or M at the sixth position from the N-terminus of the epitope.

A peptide epitope of the invention, or a nucelic acid that encodes a peptide of the invention, may be used, *inter alia,* as a pharmaceutical composition to induce a helper T cell response in a patient by contacting a helper T cell with the epitope. One or more peptide epitopes of the invention may be included in such a composition. In a preferred embodiment, one or more epitopes is presented to a helper T cell by an antigen-presenting cell that has been pulsed with the peptide *ex vivo.*

### Definitions

The term "peptide" is used interchangeably with "oligopeptide" in the present specification to designate a series of residues, typically L-amino acids, connected one to the other typically by peptide bonds between the alpha-amino and carbonyl groups of adjacent amino acids. The oligopeptides of the invention are typically less than about 50 residues in length and usually consist of between about 10 and about 30 residues, more usually between about 12 and 25, and often 15 and about 20 residues.

An "immunogenic peptide" is a peptide which comprises an allele-specific motif such that the peptide will bind an MHC molecule and induce an HTL response. Immunogenic peptides of the invention are capable of binding to an appropriate HLA molecule and inducing HTL response against the antigen from which the immunogenic peptide is derived.

With regard to a particular amino acid sequence, an "epitope" is a set of amino acid residues which is involved in recognition by a particular immunoglobulin, or in the context of T cells, those residues necessary for recognition by T cell receptor proteins and/or Major Histocompatibility Complex (MHC) receptors. In an immune system setting, *in vivo* or *in vitro,* an epitope is the collective features of a molecule, such as primary, secondary and tertiary peptide structure, and charge, that together form a site recognized by an immunoglobulin, T cell receptor or HLA molecule.

A "conserved residue" is a conserved amino acid occupying a particular position in a peptide motif typically one where the MHC structure may provide a contact point with the immunogenic peptide. One to three, typically two, conserved residues within a peptide of defined length defines a motif for an immunogenic peptide. These residues are typically in close contact with the peptide binding groove, with their side chains buried in specific pockets of the groove itself.

The term "motif' refers to the pattern of residues in a peptide of defined length, usually a peptide of from about 8 to about 13 amino acids for a class I HLA motif and from about 6 to about 25 amino acids for a class II HLA motif, which is recognized by a particular HLA molecule. Peptide motifs are typically different for each protein encoded by each human HLA allele and differ in the pattern of the primary and secondary anchor residues.

The term "supermotif" refers to motifs that, when present in an immunogenic peptide, allow the peptide to bind more than one HLA antigen. The supermotif preferably is recognized by at least one HLA allele having a wide distribution in the human population, preferably recognized by at least two alleles, more preferably recognized by at least three alleles, and most preferably recognized by more than three alleles.

A "primary anchor residue" is an amino acid at a specific position along a peptide sequence which is understood to provide a contact point between the immunogenic peptide and the HLA molecule. One to three primary anchor residues within a peptide of defined length generally defines a "motif' for an immunogenic peptide. These residues are understood to fit in close contact with peptide binding grooves of an HLA molecule, with their side chains buried in specific pockets of the binding grooves themselves. For example, analog peptides can be created by altering the presence or absence of particular residues in these primary anchor positions. Such analogs are used to modulate the binding affinity of a peptide comprising a particular motif or supermotif.

A "secondary anchor residue" is an amino acid at a position other than a primary anchor position in a peptide which may influence peptide binding. A secondary anchor residue occurs at a significantly higher frequency amongst bound peptides than would be expected by random distribution of amino acids at one position. The secondary anchor residues are said to occur at "secondary anchor positions." A secondary anchor residue can be identified as a residue which is present at a higher frequency among high or intermediate affinity binding peptides, or a residue otherwise associated with high or intermediate affinity binding. For example, analog peptides can be created by altering the presence or absence of particular residues in these secondary anchor positions. Such analogs are used to finely modulate the binding affinity of a peptide comprising a particular motif or supermotif.

A "negative binding residue" is an amino acid which if present at certain positions (typically not primary anchor positions) of peptide epitope results in decreased binding affinity of the peptide for the peptide's corresponding HLA molecule.

Throughout this disclosure, results are expressed in terms of "IC₅₀'s." IC₅₀ is the concentration of peptide in a binding assay at which 50% inhibition of binding of a reference peptide is observed. Given the conditions in which the assays are run *(i.e.,* limiting HLA proteins and labeled peptide concentrations), these values approximate K_{D} values. It should be noted that IC₅₀ values can change, often dramatically, if the assay conditions are varied, and depending on the particular reagents used *(e.g.,* HLA preparation, *etc.).* For example, excessive concentrations of HLA molecules will increase the apparent measured IC₅₀ of a given ligand. Assays for determining binding are described in detail in PCT publications WO 94/20127 and WO 94/03205. Alternatively, binding is expressed relative to a reference peptide. As a particular assay becomes more, or less, sensitive, the IC₅₀'s of the peptides tested may change somewhat. However, the binding relative to the reference peptide will not significantly change. For example, in an assay run under conditions such that the IC₅₀ of the reference peptide increases 10-fold, the IC₅₀ values of the test peptides will also shift approximately 10-fold. Therefore, to avoid ambiguities, the assessment of whether a peptide is a good, intermediate, weak, or negative binder is generally based on its IC₅₀, relative to the IC₅₀ of a standard peptide.

As used herein, "high affinity" with respect to HLA class II molecules is defined as binding with an IC₅₀ or K_{D} of less than 100 nM. "Intermediate affinity" is binding with an IC₅₀ or K_{D} of between about 100 and about 1000 nM.

"Cross-reactive binding" indicates that a peptide is bound by more than one HLA molecule; a synonym is degenerate binding.

The phrases "isolated" or "biologically pure" refer to material which is substantially or essentially free from components which normally accompany it as found in its native state. Thus, the peptides of this invention do not contain materials normally associated with their *in situ* environment, e.g., MHC I molecules on antigen presenting cells. Even where a protein has been isolated to a homogenous or dominant band, there are trace contaminants in the range of 5-10% of native protein which co-purify with the desired protein. Isolated peptides of this invention do not contain such endogenous co-purified protein.

The term "residue" refers to an amino acid or amino acid mimetic incorporated in an oligopeptide by an amide bond or amide bond mimetic.

### BRIEF DESCRIPTION OF THE DRAWINGS

Figure 1 presents a map of the positive or negative effect of each of the 20 naturally occurring amino acids on DR4w4 binding capacity when occupying a particular position, relative to the main P1-P6 anchors.
Figure 2A presents a map of the positive or negative effect of each of the 20 naturally occurring amino acids on DR1 binding capacity when occupying a particular position, relative to the main P1-P6 anchors.
Figure 2B presents a map of the positive or negative effect of each of the 20 naturally occurring amino acids on DR7 binding capacity when occupying a particular position, relative to the main P1-P6 anchors.

### DESCRIPTION OF THE PREFERRED EMBODIMENT

The present invention relates to compositions and methods for preventing, treating or diagnosing a number of pathological states such as viral, fungal, bacterial and parasitic diseases and cancers. In particular, it provides novel peptides capable of binding selected major histocompatibility complex (MHC) class II molecules at an IC₅₀ of less than or equal to 1000 nM and inducing an immune response.

Peptide binding to MHC molecules is determined by the allelic type of the MHC molecule and the amino acid sequence of the peptide. MHC class II-binding peptides usually contain within their sequence two conserved ("anchor") residues that interact with corresponding binding pockets in the MHC molecule. Specific combination of anchor residues (usually referred to as "MHC motifs") required for binding by several allelic forms of human MHC (HLA, histocompatibility leukocyte antigens) are described in International Applications WO 94/03205 and WO 94/20127. Definition of specific MHC motifs allows one to predict from the amino acid sequence of an individual protein, which peptides have the potential of being immunogenic for HTL. These applications describe methods for preparation and use of immunogenic peptides in the treatment of disease.

An affinity threshold strongly correlated with immunogenicity in the context of HLA class II DR molecules has been delineated as disclosed herein. In order to define a biologically significant threshold of DR binding affinity, a database of the binding affinities of 32 DR-restricted epitopes for their restricting element *(i.e.,* the HLA molecule that binds the motif) was compiled. In approximately half of the cases (15 of 32 epitopes), DR restriction was associated with high binding affinities, i.e. binding affinities of less than 100 nM. In the other half of the cases (16 of 32), DR restriction was associated with intermediate affinity (binding affinities in the 100-1000 nM range). In only one of 32 cases was DR restriction associated with an IC₅₀ of 1000 nM or greater. Thus, 1000 nM, preferably 100 nM, can be defined as an affinity threshold associated with immunogenicity in the context of DR molecules.

The peptide epitopes described here can also be used in combination with peptide epitopes which induce a CTL response. See, also, WO 95/07077.

The peptide epitopes of the invention may also include analogs of the epitopes. Although the peptide epitopes may exhibit cross-reactive binding with multiple DR alleles, cross-reactivity is not always complete and in such cases procedures to further increase cross-reactivity of peptides can be useful; such procedures can also be used to modify other properties of the peptide epitopes. Having established the general rules that govern cross-reactivity of peptides for HLA alleles within a given motif or supermotif, modification *(i.e.,* analoging) of the structure of peptides of particular interest in order to achieve broader (or otherwise modified) HLA binding capacity can be performed. More specifically, peptides which exhibit the broadest cross-reactivity patterns, (both amongst the known T cell epitopes, as well as the more extended set of peptides that contain the appropriate supermotifs), can be produced in accordance with the teachings herein.

The strategy employed utilizes the motifs or supermotifs which correlate with binding to certain HLA molecules. The motifs or supermotifs are defined by having primary anchors, though secondary anchors can also be modified. Analog peptides can be created by substituting amino acids residues at primary anchor, secondary anchor, or at primary and secondary anchor positions. Generally, analogs are made for peptides that already bear a motif or supermotif. Preferred secondary anchor residues of supermotifs and motifs that have been defined for HLA class II binding peptides are shown in Table IX.

For a number of the motifs or supermotifs in accordance with the invention, residues are defined which are deleterious to binding to allele-specific HLA molecules or members of HLA supertypes that bind to the respective motif or supermotif. Accordingly, removal of residues that are detrimental to binding can be performed in accordance with the present invention. For example, in the case of the A3 supertype, when all peptides that have such deleterious residues are removed from the population of analyzed peptides, the incidence of cross-reactivity increases from 22% to 37% (see, e.g., Sidney, J. *et al., Hu. Immunol.* 45:79, 1996). Thus, one strategy to improve the cross-reactivity of peptides within a given supermotif is simply to delete one or more of the deleterious residues present within a peptide and substitute a small "neutral" residue such as Ala (that may not influence T cell recognition of the peptide). An enhanced likelihood of cross-reactivity is expected if, together with elimination of detrimental residues within a peptide, residues associated with high affinity binding to multiple alleles within a superfamily are inserted.

To ensure that changes in the native or original epitope recognized by T cells do not lead to a failure to elicit helper T cells that cross-react with the wild type peptides, the variant peptide may be used to immunize T cells *in vitro* from individuals of the appropriate HLA allele, and the cells' capacity to induce lysis of wild type peptide sensitized target cells is evaluated. In both class I and class II systems it will be desirable to use as targets, cells that have been either infected or transfected with the appropriate genes to establish whether endogenously produced antigen is also recognized by the relevant T cells.

Another embodiment of the invention to ensure adequate numbers of cross-reactive cellular binders is to create analogs of weak binding peptides. Class II peptides exhibiting binding affinities of above 1000 nM, and carrying an acceptable but suboptimal primary anchor residue at one or both positions can be "fixed" by substituting preferred anchor residues in accordance with the respective supertype. The analog peptides can then be tested for crossbinding activity.

Another embodiment for generating effective peptide analogs involves the substitution of residues that have an adverse impact on peptide stability or solubility in a liquid environment. This substitution may occur at any position of the peptide epitope. For example, a cysteine (C) can be substituted out in favor of α-amino butyric acid. Due to its chemical nature, cysteine has the propensity to form disulfide bridges and sufficiently alter the peptide structurally so as to reduce binding capacity. Substituting α-amino butyric acid for C not only alleviates this problem, but actually improves binding and crossbinding capability in certain instances (Review: A. Sette et al, In: Persistent Viral Infections, Eds. R. Ahmed and I. Chen, John Wiley & Sons, England, in press, 1998). Substitution of cysteine with α-amino butyric acid may occur at any residue of a peptide epitope, i.e. at either anchor or non-anchor positions.

The DR-binding peptides of the present invention or nucleic acids encoding them can be administered to mammals, particularly humans, for prophylactic and/or therapeutic purposes. The DR peptide epitopes can be used to enhance immune responses against other immunogens administered with the peptides. For instance, CTLepitope/DR epitope mixtures may be used to treat and/or prevent viral infection and cancer, Alternatively, immunogens which induce antibody responses can be used. Examples of diseases which can be treated using the immunogenic mixtures of DR peptides and other immunogens include prostate cancer, hepatitis B, hepatitis C, AIDS, renal carcinoma, cervical carcinoma, lymphoma, CMV and condyloma acuminatum.

The DR-binding peptides or nucleic acids encoding them may also be used to treat a variety of conditions involving unwanted T cell reactivity. Examples of diseases which can be treated using DR-binding peptides include autoimmune diseases (e.g., rheumatoid arthritis, multiple sclerosis, and myasthenia gravis), allograft rejection, allergies (e.g., pollen allergies), lyme disease, hepatitis, LCMV, post-streptococcal endocarditis, or glomerulonephritis, and food hypersensitivities.

In therapeutic applications, the immunogenic compositions or the DR-binding peptides or nucleic acids of the invention are administered to an individual already suffering from cancer, autoimmune disease, or infected with the virus of interest. Those in the incubation phase or the acute phase of the disease may be treated with the DR-binding peptides or immunogenic conjugates separately or in conjunction with other treatments, as appropriate.

In therapeutic applications, compositions comprising immunogenic compositions are administered to a patient in an amount sufficient to cure or at least partially arrest the symptoms of the disease and its complications. An amount adequate to accomplish this is defined as "therapeutically effective dose." Amounts effective for this use will depend on, e.g., the peptide composition, the manner of administration, the stage and severity of the disease being treated, the weight and general state of health of the patient, and the judgment of the prescribing physician.

Therapeutically effective amounts of the immunogenic compositions of the present invention are in t;he general range of immunogenically effective dosages described below. These doses may be followed by boosting dosages pursuant to a boosting regimen over weeks to months depending upon the patient's response and condition by measuring specific HTL activity in the patient's blood.

It must be kept in mind that the compositions of the present invention may generally be employed in serious disease states, that is, life-threatening or potentially life-threatening situations. In such cases, in view of the minimization of extraneous substances and the relative nontoxic nature of the conjugates, it is possible and may be felt desirable by the treating physician to administer substantial excesses of these compositions.

For prophylactic use, administration should be given to risk groups. For example, protection against malaria, hepatitis, or AIDS may be accomplished by prophylactically administering compositions of the invention, thereby increasing immune capacity. Therapeutic administration may begin at the first sign of disease or the detection or surgical removal of tumors or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide mixtures or conjugates can also be used for the treatment of chronic infection and to stimulate the immune system to eliminate virus-infected cells in carriers. It is important to provide an amount of immuno-potentiating peptide in a formulation and mode of administration sufficient to effectively stimulate helper T cell response. Immunizing doses followed by boosting doses at established intervals, e.g., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic or prophylactic treatment are intended for parenteral, topical, oral or local administration. Typically, the pharmaceutical compositions are administered parenterally, e.g., intravenously, intrathecally, subcutaneously, intradermally, or intramuscularly. Because of the ease of administration, the vaccine compositions of the invention are particularly suitable for oral administration. Thus, the invention provides compositions for parenteral administration which comprise a solution of the peptides or conjugates dissolved or suspended in an acceptable carrier, preferably an aqueous carrier. A variety of aqueous carriers may be used, e.g., water, buffered water, 0.9% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of DR and/or CTL stimulatory peptides of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

The peptides or conjugates of the invention may also be administered via liposomes, which serve to target the conjugates to a particular tissue, such as lymphoid tissue, or targeted selectively to infected cells, as well as increase the half-life of the peptide composition. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes filled with a desired peptide or conjugate of the invention can be directed to the site of lymphoid cells, where the liposomes then deliver the selected therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka, *et al., Ann. Rev. Biophys. Bioeng.* 9, 467 (1980), U.S. Patent Nos. 4,235,871, 4,501,728, 4,837,028, and 5,019,369, incorporated herein by reference.

For targeting to the immune cells, a ligand to be incorporated into the liposome can include, e.g., antibodies or fragments thereof specific for cell surface determinants of the desired immune system cells. A liposome suspension containing a peptide or conjugate may be administered intravenously, locally, topically, etc. in a dose which varies according to, inter alia, the manner of administration, the conjugate being delivered, and the stage of the disease being treated.

Alternatively, DNA or RNA encoding one or more DR peptides (and optionally, a polypeptide containing one or more CTL epitopes or antibody inducing epitopes) may be introduced into patients to obtain an immune response to the polypeptides which the nucleic acid encodes. This approach is described, for instance, in Wolff *et. al., Science* 247:1465 (1990) as well as U.S. Patent Nos. 5,580,859; 5,589,466; 5,804,566; 5,739,118; 5,736,524; 5,679,647; WO 98/04720; and in more detail below. Examples of DNA-based delivery technologies include "naked DNA", facilitated (bupivicaine, polymers, peptide-mediated) delivery, cationic lipid complexes, and particle-mediated ("gene gun") delivery.

A preferred means of administering nucleic acids encoding the peptides of the invention uses minigene constructs encoding one or multiple epitopes of the invention. The use of multi-epitope minigenes is described below and in, *e.g.* An, L. and Whitton, J. L., *J*. *Virol.* 71:2292, 1997; Thomson, S. A. *et al, J*. *Immunol.* 157:822, 1996; Whitton, J. L. *et al., J. Virol.* 67:348, 1993; Hanke, R. *et al., Vaccine* 16:426, 1998. For example, a multi-epitope DNA plasmid encoding nine dominant HLA-A*0201- and A11-restricted epitopes derived from the polymerase, envelope, and core proteins of HBV and HIV, the PADRE^{TM} universal helper T cell (HTL) epitope, and an ER-translocating signal sequence was engineered. Immunization of HLA transgenic mice with this plasmid construct resulted in strong CTL induction responses against the nine epitopes tested, similar to those observed with a lipopeptide of known immunogenicity in humans, and significantly greater than immunization in oil-based adjuvants. Moreover, the immunogenicity of DNA-encoded epitopes *in vivo* correlated with the *in vitro* responses of specific CTL lines against target cells transfected with the DNA plasmid.

For example, to create a DNA sequence encoding the selected epitopes (minigene) for expression in human cells, the amino acid sequences of the epitopes may be reverse translated. A human codon usage table can be used to guide the codon choice for each amino acid. These epitope-encoding DNA sequences may be directly adjoined, so that when translated, a continuous polypeptide sequence is created. To optimize expression and/or immunogenicity, additional elements can be incorporated into the minigene design. Examples of amino acid sequences that could be reverse translated and included in the minigene sequence include: HLA class I epitopes, HLA class II epitopes, a ubiquitination signal sequence, a leader sequence, and/or an endoplasmic reticulum targeting signal. In addition, HLA presentation of CTL and HTL epitopes may be improved by including synthetic *(e.g.* poly-alanine) or naturally-occurring flanking sequences adjacent to the CTL or HTL epitopes.

The minigene sequence may be converted to DNA by assembling oligonucleotides that encode the plus and minus strands of the minigene. Overlapping oligonucleotides (30-100 bases long) may be synthesized, phosphorylated, purified and annealed under appropriate conditions using well known techniques. The ends of the oligonucleotides can be joined, for example, using T4 DNA ligase. This synthetic minigene, encoding the epitope polypeptide, can then be cloned into a desired expression vector.

Standard regulatory sequences well known to those of skill in the art are preferably included in the vector to ensure expression in the target cells. Several vector elements are desirable: a promoter with a down-stream cloning site for minigene insertion; a polyadenylation signal for efficient transcription termination; an *E. coli* origin of replication; and an *E. coli* selectable marker *(e.g.* ampicillin or kanamycin resistance). Numerous promoters can be used for this purpose, *eg.,* the human cytomegalovirus (hCMV) promoter. *See, e.g.,* U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences and sequences for replication in mammalian cells may also be considered for increasing minigene expression.

Once an expression vector is selected, the minigene is cloned into the polylinker region downstream of the promoter. This plasmid is transformed into an appropriate *E. coli* strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

In addition, immunostimulatory sequences (ISSs or CpGs) appear to play a role in the immunogenicity of DNA vaccines. These sequences may be included in the vector, outside the minigene coding sequence, if desired to enhance immunogenicity.

In some embodiments, a bi-cistronic expression vector which allows production of both the minigene-encoded epitopes and a second protein (included to enhance or decrease immunogenicity) can be used. Examples of proteins or polypeptides that could beneficially enhance the immune response if co-expressed include cytokines (*e.g*., IL-2, IL-12, GM-CSF), cytokine-inducing molecules *(e.g.,* LeIF) or costimulatory molecules. Helper (HTL) epitopes can be joined to intracellular targeting signals and expressed separately from expressed CTL epitopes; this allows direction of the HTL epitopes to a cell compartment different than that of the CTL epitopes. If required, this could facilitate more efficient entry of HTL epitopes into the HLA class II pathway, thereby improving CTL induction. In contrast to HTL or CTL induction, specifically decreasing the immune response by co-expression of immunosuppressive molecules *(e.g.* TGF-β) may be beneficial in certain diseases).

Therapeutic quantities of plasmid DNA can be produced for example, by fermentation in *E. coli,* followed by purification. Aliquots from the working cell bank are used to inoculate growth medium, and grown to saturation in shaker flasks or a bioreactor according to well known techniques. Plasmid DNA can be purified using standard bioseparation technologies such as solid phase anion-exchange resins supplied by QIAGEN, Inc. (Valencia, California). If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). This approach, known as "naked DNA," is currently being used for intramuscular (IM) administration in clinical trials. To maximize the immunotherapeutic effects of minigene DNA vaccines, an alternative method for formulating purified plasmid DNA may be desirable. A variety of methods have been described, and new techniques may become available. Cationic lipids can also be used in the formulation (see, *e.g.,* as described by WO 93/24640; Mannino & Gould-Fogerite, *BioTechniques* 6(7): 682 (1988); U.S. Pat No. 5,279,833; WO 91/06309; and Felgner, *et al., Proc. Nat'l Acad. Sci. USA* 84:7413 (1987). In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

*In vitro* assays can be used as functional assays for expression of HTL epitopes. For example, the plasmid DNA is introduced into a mammalian cell line that is a suitable presenter of HTL epitopes.. The transfection method used will be dependent on the final formulation. Electroporation can be used for "naked" DNA, whereas cationic lipids allow direct in *vitro* transfection. A plasmid expressing green fluorescent protein (GFP) can be co-transfected to allow enrichment of transfected cells using fluorescence activated cell sorting (FACS). The cells may then be assayed for the ability to elicit an HTL response using methods known in the art *(see, e.g.,* Alexander *et al., Immunity* 1:751-761, 1994)

*In vivo* immunogenicity is a second approach for functional testing of minigene DNA formulations which include both CTL and HTL epitopes. Transgenic mice expressing appropriate human HLA proteins are immunized with the DNA product. The dose and route of administration are formulation dependent (*e.g*., IM for DNA in PBS, IP for lipid-complexed DNA). Twenty-one days after immunization, splenocytes are harvested and restimulated for 1 week in the presence of peptides encoding each epitope being tested. For CTL effector cells, assays are conducted for cytolysis of peptide-loaded, chromium-51 labeled target cells using standard techniques. Lysis of target cells sensitized by HLA loading of peptides corresponding to minigene-encoded epitopes demonstrates DNA vaccine function for *in vivo* induction of CTLs.

Alternatively, the nucleic acids can be administered using ballistic delivery as described, for instance, in U.S. Patent No. 5,204,253. Using this technique, particles comprised solely of DNA are administered. In a further alternative embodiment, DNA can be adhered to particles, such as gold particles.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more conjugates of the invention, and more preferably at a concentration of 25%-75%.

For aerosol administration, the peptides are preferably supplied in finely divided form along with a surfactant and propellant. Typical percentages of conjugates are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1 %-20% by weight of the composition, preferably 0.25-5%. The balance of the composition is ordinarily propellant. A carrier can also be included, as desired, as with, e.g., lecithin for intranasal delivery.

In another aspect the present invention is directed to vaccines which contain as an active ingredient an immunogenically effective amount of an immunogenic DR peptide or a CTL\DR peptide conjugate or nucleic acid encoding them as described herein. The conjugate(s) may be introduced into a host, including humans, linked to its own carrier or as a homopolymer or heteropolymer of active peptide units. Such a polymer has the advantage of increased immunological reaction and, where different peptides are used to make up the polymer, the additional ability to induce antibodies and/or CTLs that react with different antigenic determinants of the virus or tumor cells. Useful carriers are well known in the art, and include, e.g., thyroglobulin, albumins such as bovine serum albumin, tetanus toxoid, polyamino acids such as poly(lysine:glutamic acid), hepatitis B virus core protein, hepatitis B virus recombinant vaccine and the like. The vaccines can also contain a physiologically tolerable (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art. And, as mentioned above, CTL responses can be primed by conjugating peptides of the invention to lipids, such as P₃CSS. Upon immunization with a peptide composition as described herein, via injection, aerosol, oral, transdermal or other route, the immune system of the host responds to the vaccine by producing large amounts of CTLs and/or HTLs specific for the desired antigen, and the host becomes at least partially immune to later infection, or resistant to developing chronic infection.

Vaccine compositions containing the DR peptides of the invention are administered to a patient susceptible to or otherwise at risk of disease, such as viral infection or cancer in an amount that will elicit an immune response against the antigen and thus enhance the patient's own immune response capabilities.

A therapeutically effective amount and an amount used for vaccine of a peptide disclosed herein is defined to be an "immunogenically effective dose." In this use, the precise amounts again depend on the patient's state of health and weight, the mode of administration, the nature of the formulation, etc., but generally occur in a unit dosage range where the lower value is about 1, 5, 50, 500, or 1000 µg and the higher value is about 10,000; 20,000; 30,000; or 50,000 µg. Dosage values for a human typically range from about 500 µg to about 50,000 µg per 70 kilogram patient.

In some instances it may be desirable to combine the peptide vaccines of the invention with vaccines which induce neutralizing antibody responses to the virus of interest, particularly to viral envelope antigens. For instance, PADRE peptides can be combined with hepatitis vaccines to increase potency or broaden population coverage. Suitable hepatitis vaccines that can be used in this manner include, Recombivax HB® (Merck) and Engerix-B (Smith-Kline).

For therapeutic or immunization purposes, the peptides of the invention can also be expressed by attenuated viral hosts, such as vaccinia or fowlpox. This approach involves the use of vaccinia virus as a vector to express nucleotide sequences that encode the peptides of the invention. Upon introduction into an acutely or chronically infected host or into a non-infected host, the recombinant vaccinia virus expresses the immunogenic peptide, and thereby elicits a host CTL response. Vaccinia vectors and methods useful in immunization protocols are described in, e.g., U.S. Patent No. 4,722,848, incorporated herein by reference. Another vector is BCG (Bacille Calmette Guerin). BCG vectors are described in Stover *et al., Nature* 351, 456-460 (1991)) which is incorporated herein by reference. A wide variety of other vectors useful for therapeutic administration or immunization of the peptides of the invention, e.g., Salmonella typhi vectors and the like, will be apparent to those skilled in the art from the description herein.

The peptide epitopes of the invention may be administered to antigen presenting cells (APCs), preferably dendritic cells, *ex vivo,* as well. In a preferred embodiment, responses to a particular pathogen (infectious agent or tumor antigen) are induced by pulsing APCs with the peptide epitope and subsequently administering the pulsed APC, wherein the cells then present the peptide *in* vivo. The pulsed APCs may be administered in vivo as described above for the peptides.

Peptides epitopes of the invention may also be used in conjunction with CTL epitopes to elicit CTL *ex vivo* as well. The resulting CTL can be used to treat infections or tumors. Ex vivo CTL responses to a particular pathogen are induced by incubating in tissue culture the patient's CTL precursor cells together with a source of antigen-presenting cells and the appropriate immunogenic peptide epitopes. After an appropriate incubation time *typically 1-4 weeks) in which the CTL precursor cells are activated and expanded into effector CTL, the cells are infused back into the patient, where they will destroy their specific target cell (an infected cell or a tumor cell).

The peptides of this invention may also be used to make monoclonal antibodies. Such antibodies may be useful as potential diagnostic or therapeutic agents.

The peptides may also find use as diagnostic reagents. For example, a peptide of the invention may be used to determine the susceptibility of a particular individual to a treatment regimen which employs the peptide or related peptides, and thus may be helpful in modifying an existing treatment protocol or in determining a prognosis for an affected individual. In addition, the peptides may also be used to predict which individuals will be at substantial risk for developing chronic infection.

### Examples

### Materials and Methods

Cells. The following Epstein-Barr virus (EBV) transformed homozygous cell lines were used as sources of human HLA class II molecules: LG2 [DRB1c0101 (DR1)1; GM3107 [DRB50101 (DR2w2a)]; MAT (DRB10301 (DR3)1; PREISS [DRB10401 (DR4w4)1; BIN40 [DRB10404 (DR4w14)1; SWEIG [DRB11101 (DR5w11)]; PITOUT [DRB10701 (DR7)] (a); KT3 [DRB10405 (DR4w15)]; Herluf [DRB11201 (DR5w12)]; H0301 [DRB11302 (DR6w 19)]; OLL [DRB10802 (DR8w2)]; and HTC9074 [DRB10901 (DR9), supplied as a kind gift by Dr. Paul Harris, Columbia University]. In some instances, transfected fibroblasts were used: L466.1 [DRB11501 (DR2w2b)]; TR81.19 [DRB30101 (DR52a)]; and L257.6 [DRB40101 (DRw53)]. (Valli, *et al.* J *Clin. Invest.* 91:616 (1993). Cells were maintained *in vitro* by culture in RPMI 1640 medium supplemented with 2mM L-glutamine [GIBCO, Grand Island, NY], 50µM 2-ME, and 10% heat-inactivated FCS [Irvine Scientific, Santa Ana, CA]. Cells were also supplemented with 100 ug/ml of streptomycin and 100U/ml of penicillin [Irvine Scientific]. Large quantities of cells were grown in spinner cultures.

Cells were lysed at a concentration of 10⁸ cells/ml in PBS containing 1% NP-40 [Fluka Biochemika, Buchs, Switzerland], 1mM PMSF [CalBioChem, La Jolla, CA], 5mM Na-orthovanadate, and 25mM iodoacetamide [Sigma Chemical, St. Louis, Mo]. The lysates were cleared of debris and nuclei by centrifugation at 10,000 x *g* for 20 min.

Affinity purification of HLA-DR molecules. Class II molecules were purified by affinity chromatography as previously described (Sette, *et al. J. Immunol.* 142:35 (1989) and Gorga, *et al. J. Biol. Chem.* 262:16087 (1987)) using the mAb LB3.1 coupled to Sepharose 4B beads. Lysates were filtered through 0.8 and 0.4 µM filters and then passed over the anti-DR column, which were then washed with 15-column volumes of 10mM TRIS in 1% NP-40, PBS and 2-column volumes of PBS containing 0.4% n-octylglucoside. Finally, the DR was eluted with 50mM diethylamine in 0.15M NaCI containing 0.4% n-octylglucoside, pH 11.5. A 1/25 volume of 2.0M Tris, pH 6.8, was added to the eluate to reduce the pH to ∼8.0, and then concentrated by centrifugation in Centriprep 30 concentrators at 2000 rpm (Amicon, Beverly, MA).

Class II peptide-binding assays. A panel of 13 different specific DR-peptide assays were utilized in the present study. These assays were chosen as to be representative of the most common DR alleles. Table I lists for each DR antigen, the representative allelic product utilized, the cell line utilized as a source of DR, and the radiolabled probe utilized in the assay. Purified human class II molecules [5 to 500 nM] were incubated with various unlabeled peptide inhibitors and 1-10 nM ¹²⁵I-radiolabeled probe peptides for 48h in PBS containing 5% DMSO in the presence of a protease inhibitor cocktail. The radiolabeled probes used were HA Y307-319 (DR1), Tetanus Toxoid[TT] 830-843 (DR2w2a, DR5w111, DR7, DR8w2, DR8w3, DR9), MBP Y85-100 (DR2w2b), TT1272-1284 (DR52a), MT 65 kD Y3-13 with Y7 substituted with F for DR3, a non-natural peptide with the sequence YARFQSQTTLKQKT (DR4w4, DR4w15, DRw53) (Valli, *et al. supra),* and for DR5w12, a naturally processed peptide eluted from the cell line C1R, EALIHQLINPYVLS (DR5w12) and 650.22 peptide, (TT 830-843 A ® S836 analog), for DR6w19.

Radiolabeled peptides were iodinated using the chloramine-T method. Peptide inhibitors were typically tested at concentrations ranging from 1201 ug/ml to 1.2 ng/ml. The data were then plotted and the dose yielding 50% inhibition (IC50) was measured. In appropriate stoichiometric conditions, the IC50 of an unlabeled test peptide to the purified DR is a reasonable approximation of the affinity of interaction (Kd). Peptides were tested in two to four completely independent experiments. The final concentrations of protease inhibitors were: 1 mM PMSF, 1.3nM 1.10 phenanthroline, 73 µM pepstatin A, 8mM EDTA, and 200 µM N alpha-p-tosyl-L-lysine chloromethyl ketone (TLCK) [All protease inhibitors from CalBioChem, La Jolla, CA]. Final detergent concentration in the incubation mixture was 0.05% Nonidet P-40. Assays were performed at pH 7.0 with the exception of DR3, which was performed at pH 4.5, and DRw53, which was performed at pH 5.0. The pH was adjusted as previously described (Sette, *et al. J. Immunol.* 148:844 (1992)).

Class II peptide complexes were separated from free peptide by gel filtration on TSK2000 columns (TosoHaas 16215, Montgomeryville, PA), and the fraction of bound peptide calculated as previously described (Sette, *et al.,* (1989) *supra).* In preliminary experiments, the DR prep was titered in the presence of fixed amounts of radiolabeled peptides to determine the concentration of class II molecules necessary to bind 10-20% of the total radioactivity. All subsequent inhibition and direct binding assays were the performed using these class II concentrations.

### DRB1 specificity of DR4w15, DR6w19, DR8w2, DR8w3, and DR9 assays.

Because the antibody used for purification is α-chain specific, β1 molecules are not separated from β3 (and/or β4 and β5) molecules. Development and validation of assays in regard with DRβ chain specificity has been described in detail elsewhere for many of the DR alleles listed above (108). Herein we describe for the first time DR4w15, DR6w19, DR8w2, DR8w3, and DR9 assays. Experiments addressing the β chain specificity of these new assays are described in the present section.

DR4w15. The β4 product DRw53 is co-expressed with DR4w15 and the determination of the specificity of the DR4w1 binding assay is complicated in that the same radiolabeled ligand is used for both the DR4w15 and DRw53 binding assays. Since typically β1 chains are expressed at 5-10 fold higher levels than other β chains, and all binding assays are performed utilizing limiting DR amounts, it would be predicted that the dominant specificity detected in the assay would be DR4w15. To verify that this was indeed the case, the binding pattern of a panel of 58 different synthetic peptides in the putative DR4w15 specific assay with that obtained in a DRw53 specific assay (which uses a DRw53 fibroblast as the source of class II molecules). Two very distinct binding patterns were noted, and in several instances, a peptide bound to one DR molecule with high affinity, and did not bind to the other (data not shown).

DR6w19. The DR6w19 assay utilizes as the source of class II molecules the EBV transformed homozygous cell line H0301, which co-expresses DRB30301 (DR52a). While the radiolabeled ligand used in the DR6w19 assay is different than that used for the DR52a assay, the ligand is related (i.e., is a single substitution analog) to a high affinity DR52a binder. As was done in the case of DR4w15, the specificity of the assay was investigated by analyzing the binding capacity of a panel of naturally occurring peptides for DR6w19 and DR52a. The two assays demonstrated completely different binding specificities. For example, in terms of relative binding, TT 1272-1284 binds 63-fold better in the DR52a assay than in the DR6w19 assay. Conversely, the Invariant chain peptide binds 189-fold better in the DR6w19 assay. In conclusion, these data demonstrated that the binding of the radiolabeled peptide 650.22 to purified class II MHC from the H0301 cell line is specific for DR6w19.

DR8w2 and DR8w3. The β1 specificity of the DR8w2 and DR8w3 assays is obvious in that no β3 (and/or B4 and β5) molecule is expressed.

DR9. The specificity of DR9 assay is inferred from previous studies which have shown that the TT 830-843 radiolabeled probe peptide does not bind to DRw53 molecules (Alexander, *et al., Immunity* 1:751 (1994)).

### Results

### DR binding affinity of antigenic peptides recognized by DR restricted T cells

To define a threshold DR binding affinity, to be considered as biologically significant, we compiled the affinities of a panel of 32 reported instances of DR restriction of a given T cell epitope. In approximately half of the cases, DR restriction was associated with affinities of less than 100 nM, and in the other half of the instances, with 1C50% in the 100-1000 nM range. Only in 1 out of 32 cases (3.1%) DR restriction was associated with IC50% of 1000 nM or greater. It was noted that this distribution of affinities differs from what was previously reported for HLA class I epitopes, where a vast majority of epitopes bound with IC50% of 50 nM or less (Sette, *et al.,* JI, 1994). This relatively lower affinity of class II restricted epitope interactions might explain why activation of class II restricted T cells in general requires more antigen relative to class I restricted T cells.

In conclusion, this analysis suggested that 1000 nM may be defined as an affinity threshold associated with immunogenicity in the context of DR molecules, and for this reason a suitable target for our studies.

### P1 and P6 anchors are necessary but not sufficient for DRB 10401 binding

Several independent studies have pointed to a crucial role in DRB 10401 binding of a large aromatic or hydrophobic residue in position 1, near the N-terminus of the peptide and of a 9-residue core region (residues 1 through 9). In addition, an important role has been demonstrated for the residue in position six (P6) of this 9-residues core region. Short and/or hydrophobic residues were in general preferred in this position (O'Sullivan, *et al.,* JI 147:2663, 1991; Sette, *et al.,* JI 151:3163, 1993; Hammer, *et al.,* Cell 74:197, 1993 and Marshall, *et al.,* JI 154:5927, 1995).

In the present set of experiments, a library of 384 peptides was analyzed for DRB 10401 binding capacity and screened for the presence of the P1-P6 motif (that is, F, W, Y, L, I, V or M in P1 and S, T, C, A, P, V, I, L or M in P6, at least 9 residues apart from the peptide C-terminus. This set of 384 peptides contained a total of 80 DR4w4 binders (specifically 27 good binders [IC50 of 100 nM or less], and 53 intermediate binders [IC50 of the 100-1000 range]. Seventy-seven out of the 80 DR4w4 binders (96%) carried the P1-P6 motif. However, it should be noted that most non-DR4w4 binding peptides also contained the P1-P6 motif. Of 384 peptides included in our database, only 125 were "P1-P6 negative." Only three of them (6%) bound appreciably to purified DR4w4 as opposed to 77/259 (30%) of the "P1-P6 positive" peptides. Therefore, these results demonstrate that presence of suitable P1 and P6 anchors are necessary but not sufficient for DRB10401 binding. A detailed map of DRB 10401 peptide interactions

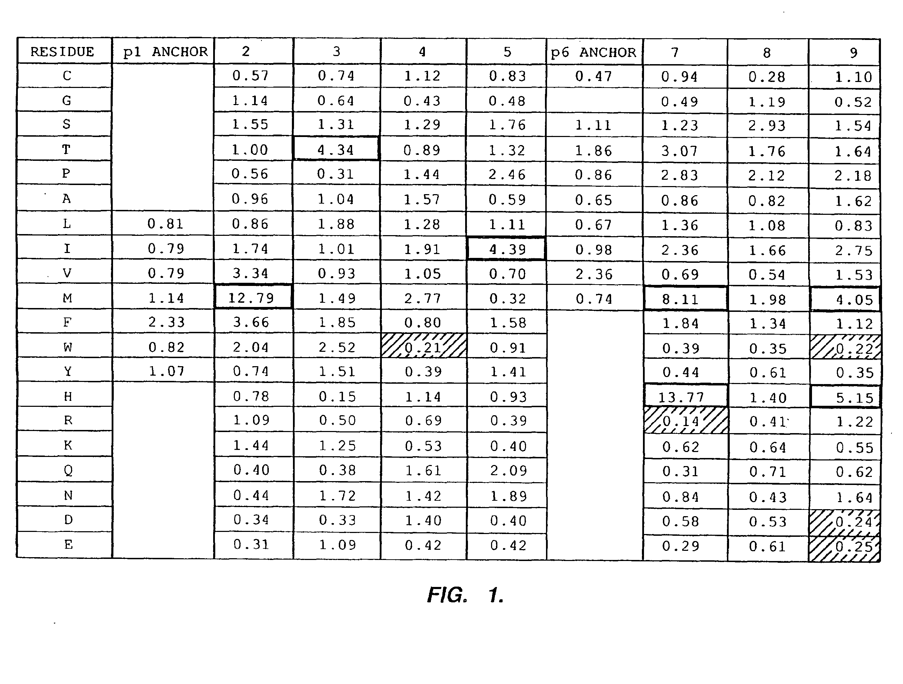

Next, for each P1-P6 aligned core region, in analogy with what the strategy previously utilized to detail peptide class I interactions the average binding affinity of peptides carrying a particular residue, relative to the remainder of the group, were calculated for each position. Following this method a table of average relative binding (ARB) values was compiled. This table also represents a map of the positive or negative effect of each of the 20 naturally occurring amino acids on DRB 10401 binding capacity when occupying a particular position, relative to the main P1-P6 anchors (Figure 1).

Variations in ARB values greater than four fold (ARB³ 4 or £ 0.25) were arbitrarily considered significant and indicative of secondary effects of a given residue on DR-peptide interactions. Most secondary effects were associated with positions 4, 7, and 9. These positions correspond to secondary anchors engaging shallow pockets on the DR molecule. In addition, significant secondary effects were detected for M in position 3 (ARB = 12.8) T in position 3 (ARB = 4.34) and I in position 5 (ARB = 4.4).

### Development of a DRB 10401 specific algorithm

Next, the ARB table was utilized to develop a DRB 10401 specific algorithm. In order to predict 0401 binding propensity, each aligned P1-P6 sequence was scored by multiplying, for each position, the ARB value of the appropriate amino acid. According to this procedure, a numerical "algorithm score" was derived. If multiple P1-P6 alignments were possible, binding scores were calculated for each one and the best score was selected. The efficacy of this method in predicting 0401 binding capacity is shown in Table IIa

Considering only peptides with algorithm scores above -17.00 narrowed the set of predicted peptides to 156. This set still contained 72 out of 80 (90%) of the total high or intermediate DR binders. Raising the cut-off to an algorithm score of -16.44 or higher still allowed identification of 60 out of 80 (75%) of the DR4w4 binding peptides. Of the whole 107 peptide set, twenty-five of them were either good or intermediate binders. In other words, as expected, increasing the algorithm score stringency predicted a smaller fraction of the total binders present in the set, but at the same time less false positive peptides were identified.

### Blind test of the predictive power of the DRB 10401 specific algorithm

To verify that the predictive capacity of our algorithm was not merely a reflection of having utilized the same data set to test and define the algorithm itself, we further examined its efficacy in a blind prediction test. For this scope we utilized data from an independent set of 50 peptides, whose binding affinities were known, but that had not been utilized in the derivation of the algorithm. As shown in Table IIb, the algorithm was effective in predicting DR4w4 binding capacity of this independent peptide set. The algorithm score of -17.00 identified a total 18 peptides. This set contained 3/3 (100%) of all good binders, and 8/11 (70%) of all intermediate binders in the entire test set of 50 peptides. Increasing the cut-off value to -16.44, identified a set of nine peptides. Seven of them (78%) were either good or intermediate binders. This set contained 7 out of 14 (50%) of the binders contained in the blind prediction peptide set. In conclusion, these data supports the validity of the DR4w4 specific algorithm described above.

### Detailed maps ofDRB10401, DRB10101, and DRB10701 peptide binding specificities

Next, we analyzed the binding to purified DR1 and DR7 molecules for the same set of 384 peptides utilized to define the DR4w4 algorithm. It was found that this set contained 120 and 59 binders for the DR1 and DR7 alleles, respectively. A total of 158 peptides were capable of binding either DR1, DR4w4 or DR7. A large fraction of them (73/158; 46%) were also degenerate binders, which bound two or more of the three alleles thus far considered. Furthermore, we also found that more than 90% of the DR1 or DR7 good and intermediate binders carried the P1-P6 motif. Most importantly, 72 out of 73 (99%) degenerate DR binders carried this motif (data not shown). In conclusion, this analysis suggests that P1-P6 based algorithms might be utilized to effectively predict degenerate DR binders.

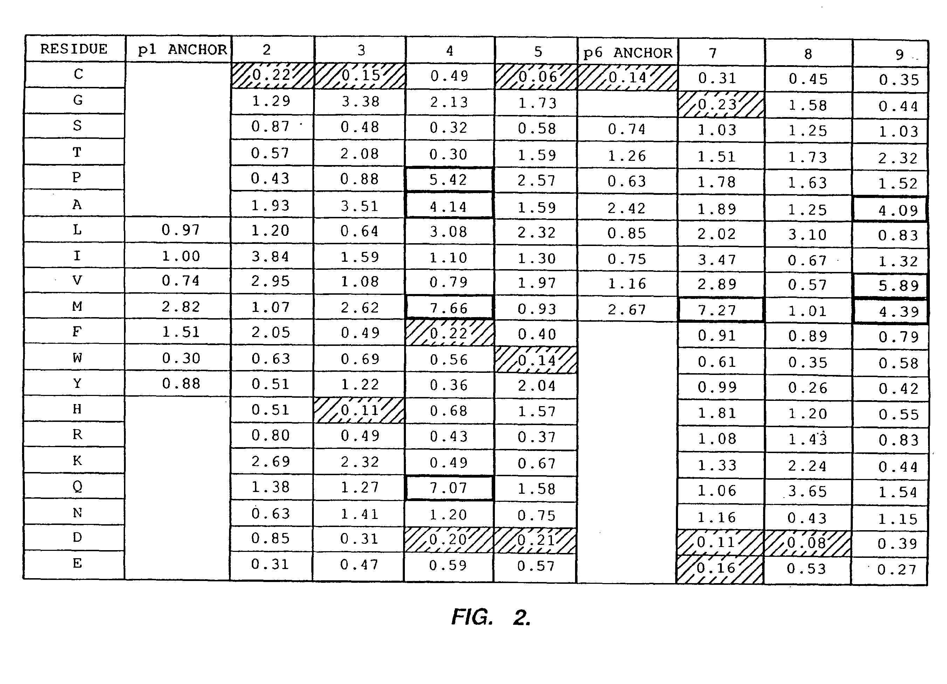

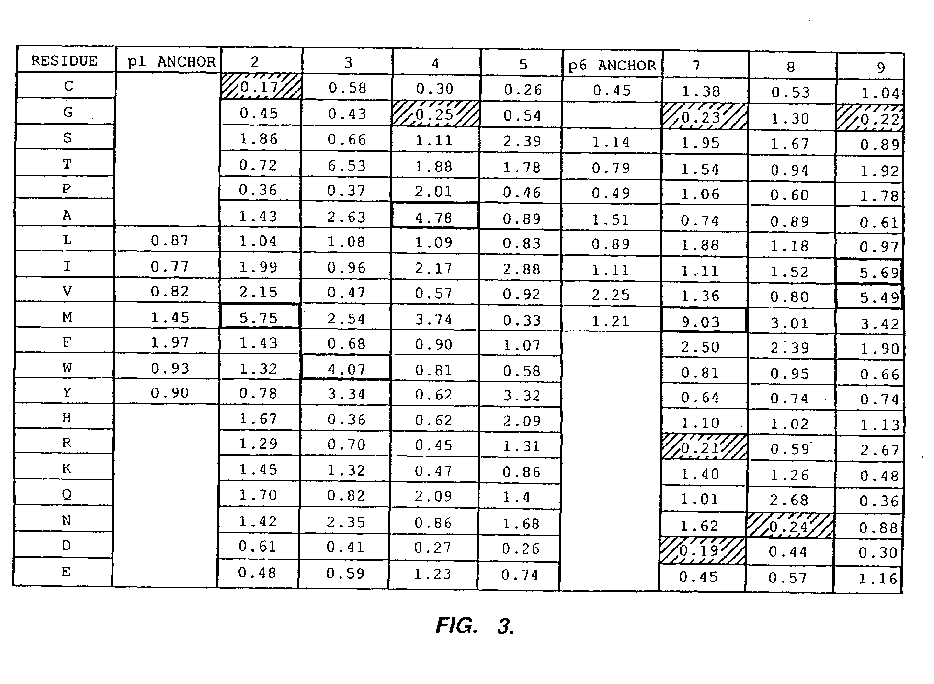

In analogy with what was described above for DR4w4 molecules, specific algorithms were designed for the DR1 and DR7 alleles. Figures 2A and 2B detail the allele specific maps defined according to this method.

As in the case of DRB 10401, most secondary effects were concentrated in positions 4, 7 and 9. Position 4 was especially prominent in the case of DR1, while position 7 was the most prominent secondary anchor for DR7. Specific algorithms were developed based on these maps, and it was found that the cut-off values necessary to predict 75% or 90% of the binders were -19.32 and -20.28 for DR1, and 20.91 and -21.63 for DR7, respectively. Depending on the particular allele or cut off value selected, 40 to 60% of the predicted peptides were in fact good or intermediate binders (data not shown).

### Development of a DR1-4-7 combined algorithm

Finally, we examined whether a combined algorithm would allow to predict degenerate binders. For this purpose, the sequences of the 384 peptides in our database were simultaneously screened with the three (DR1, 4w4, and 7) specific algorithms. It was found that an even 100 peptides were predicted (using the 75% cut off) to bind either two or three of the alleles considered. This set contained 59 out of 73 (81%) of the peptides which were in fact capable of degenerate 1-4-7 binding (defined as the capacity to bind to more than one of the DR1, 4w4 or 7 alleles) (Table III).

### Definition of a target set of DR specificities, representative of the world population

The data presented in the preceding sections illustrates how peptides capable of binding multiple DR alleles can be identified by the use of a combined "1-4-7" algorithm. Next, we wished to examine whether the peptides exhibiting degenerate 1-4-7 binding behavior would also bind other common DR types as well. As a first step in our experimental strategy, we sought to define a set of target DR types representative of a large (³ 80%) fraction of the world population, irrespective of the ethnic population of origin. For this purpose, seven additional DR antigens were considered. For each one of the DR antigens considered in this study, (including DR1,4 and 7), the estimated frequency in various ethnicities, according to the most recent HLA workshop (11th, 1991) is shown in Table IVa, together with the main subtypes thus far identified.

For the purpose of measuring peptide binding affinity to the various DR molecules, one representative subtype for each DR antigen was chosen (Table I). It should be noted that for most antigens, either one subtype is by far the most abundant, or alternatively a significant degree of similarity in the binding pattern displayed by the different, most abundant subtypes of each DR antigen is likely to exist (see comments column of Table IVb). One exception to this general trend is represented by the DR4 antigen, for which significant differences in peptide specificity between the 0401 and 0405 have been reported. Since both alleles are quite frequent (in Caucasians and Orientals, respectively) we included both DR 0401 and 0405 in the set of representative DR binding assays.

Our set of representative assays is mostly focused on allelic products of the gene, because these molecules appear to be the most abundantly expressed, serve as the dominant restricting element of most human class III responses analyzed thus far, and accurate methods for serologic and DNA typing most readily available. However, we have also considered in our analysis assays representative of DRB3/4/5 molecules (Table IVc). These molecules serve as a functional restriction element, and their peptide binding specificity has been previously shown to have certain similarities to the specificity of several common DRβ₁ allelic products.

A general strategy for prediction of DR-degenerate binders.

To test whether the 1-4-7 combined algorithm would also predict degenerate binding to other common DR types, we measured the capacity of three different groups of synthetic peptides to bind the panel of purified HLA DR molecules. The three different peptide sets were: A) 36 peptides which did not score positive in the combined 1-4-7 algorithm (non-predictions), B) 36 peptides which did score positive for the 1-4-7 algorithm, at the 75% cut off level, but had been found upon actual testing not to be degenerate 1-4-7 binders ("wrong" predictions), and C) 29 peptides which scored positive in the 1-4-7 algorithm, and also proved upon experimental testing, to be actual 1-4-7 degenerate binders (correct predictions). The results of this analysis are shown in Table V.

Within the set of "non-predictions" peptides (Table Va) only 3 out of 34 (9%) bound at least two of the DR1, 4w4 or 7 molecules. Interestingly, 2 (1136.04 and 1136.29) out of 3 of these peptides were also rather crossreactive, and bound additional DR types (DR2w2 β2, DR4w15, 5w11 and 8w2 in the case of 1136.04, and 2w2 β2, 4w15,9 and 5w12 in the case of 1136.29). Peptides from the "wrong predictions" peptide set (Table V5), by definition bound at the most only one of the DR1, 4w4 or DR7 molecules, and were also poorly degenerate or other DR types with only two peptides (1136.22 and 1188.35) binding a total of three DR molecules. Within this peptide set, no peptide bound four or more of the DR molecules tested (data not shown).

These results are contrasted by data obtained with the peptide set corresponding to peptides which were first predicted by the use of the combined 1, 4, 7 algorithm, and then experimentally found to be degenerate DR1-4-7 binding. Fourteen out of 29 peptides tested (48%) bound a total of five or more alleles. Four of them were remarkably degenerate (1188.16, 1188.32, 1188.34 and F107.09) and bound a total of nine out of the 11 DR molecules tested. In conclusion, these results suggest that a strategy based on the sequential use of a combined DR1, 4, 7 algorithm and quantitative DR1,4, 7 binding assays can be utilized to identify broadly crossreactive DR binding peptides.

### Definition of the HLA-DR 1-4-7 supertype

The data presented above also suggested that several common DR types are characterized by largely overlapping peptide binding repertoires. When this issue was analyzed in more detail, by analyzing the binding pattern of the thirty-two peptides from Table Va and b which were actual DR1-4-7 degenerate binders. Thirty-one of them (97%) bound DR1, 22 (69%) DR4w4 and 21 (66%) DR7. These files are contrasted with the low percentages of binding observed amongst the remainder non-degenerate binding peptides (17/67 (25%), 8/67 (12%) and 7/67 (10%), for DR1, 4w4 and 7, respectively) (Table VII).

Interestingly, a large fraction of the 1-4-7 degenerate binders also bound certain other common DR types. Sixteen (50%) bound DR2w2a, 18 (56%) DR6w19, 18 (56%) DR2w2b and 20 (62%) DR9. In all cases, the frequency of binding in the non-1-4-7 degenerate peptide set was much lower (Table VIII).

Significant, albeit lower, frequencies of cross reactivity were noted also for DR4w15, DR5w11, and DR8w2 (in the 28 to 37% range). Finally, negligible levels of cross reactivity were observed in the case of DR3 and 5w12 and DR53. Further studies will address whether either of these two group of molecules (DR4w15, 5w1 1, and 8w2 on one hand, and DR3, DR53 and 5w12 on the other) might belong to different DR supertypes.

In conclusion, these data demonstrates that a large set of DR molecules encompassing DR1,4w4, 2w2a, 2w2b, 7, 9 and 6w19 is characterized by largely overlapping peptide binding repertoires.

### Discussion

In the present report we have analyzed the peptide binding specificity of a set of 13 different DR molecules, representative of DR types common among the worldwide population. Detailed maps of secondary anchors and secondary interactions have been derived for three of them (DR4w4, DR1 and DR7). Furthermore, we demonstrated that a set of at least seven different DR types share overlapping peptide binding repertoires; and consequently that broadly degenerate HLA DR binding peptides are a relatively common occurrence. This study also describes computerized procedures which should greatly assist in the task of identification of such degenerate peptides.

We would like to discuss the data in the context of our current understanding of peptide-class II interactions, as well as in the context of the recently described class I supermotifs. Finally, the potential implications of broadly degenerate class II epitopes for epitope based vaccine design should also be considered.

Firstly, our studies illustrate how the vast majority of the peptides binding with good affinity to DR4w4, DR1, DR7 and most of the other DR types analyzed in the current study (data not shown), are all characterized by a P1-P6 motif consistent with the one originally proposed by O'Sullivan, *et al.* Crystallographic analysis of DR1-peptide complexes revealed that the residues occupying these positions engage two complementary pockets on the DR1 molecule, with the P1 position corresponding to the most crucial anchor residue and the deepest hydrophobic pocket. Our analysis also illustrates how other "secondary anchor" positions drastically influence in an allele-specific manner peptide binding capacity. Position 4 was found to be particularly crucial for DR1 binding, position 9 for DR4w4, and position 7 for DR7. These data are consistent with previous results which originally described such allele-specific anchors, and with crystallographic data which illustrates how these residues engage shallow pockets on the DR molecule.

Secondly, our studies illustrate how an approach based on alignment and calculation of average relative binding values of large peptide libraries allows definition of quantitative algorithms to predict binding capacity. The present study extends those observations to two other common HLA-DR types, and also illustrates how the combined use of the 1-4-7 algorithms can be of aid in identifying broadly degenerate DR binding peptides.

The data presented herein suggest that a group of common DR alleles, including at least DR1, DR2w2a, DR2w2b, DR4w4, DR6w19, DR7 and DR9 share a largely overlapping peptide repertoire. Degenerate peptide binding to multiple DR alleles, and recognition of the same epitope in the context of multiple DR types was originally described by Lanzavechia, Sinigallia's and Rothbard's groups. The present study provides a classification of alleles belonging to a main HLA-DR supertype (DR1-4-7-like) which includes DR1, DR2w2a, DR2w2b, DR4w4, DR7, DR9, DR6w19. On the basis of the data presented herein, at least two additional groups of alleles exist. The first group encodes for molecules with significant, albeit much reduced overlap with the 1-4-7-like supertype (DR4w15, 8w2, 5 1). The second group of alleles (5w12, 3w17, and w53) clearly has little repertoire association with the 1-4-7 supertype. In this context it is interesting to note that Hammer, *et al.* noted that good DR5w 11 binding peptides are frequently characterized by positively charged P6 anchor (which would be poorly compatible) with the herein proposed 1-4-7 supermotif. It is also interesting to note that Sidney, *et al.* proposed that DR3w17 binds a set of peptides largely distinct from those bound by other common DR types. Future studies will have to determine whether any of the molecules listed above can be grouped in additional DR supertypes. Our group is currently investigating whether analysis of polymorphic residues lining the peptide binding pockets of DR can be utilized to aid in the classification and prediction of HLA DR supertypes.

We would like to comment on similarities and differences between the HLA DR supertype described herein and the recently described HLA class I supermotifs. Class I supermotifs are clear-cut and, as a rule, non-overlapping. Four of them have been described all approximately equally frequent amongst the worldwide population. By contrast, the repertoire defining the HLA DR supertype herein described is not clear-cut and overlaps, at least in part, with the repertoire of other alleles. It also appears that on the basis of the data presented in Tables I and IV, even if other DR supertypes exist, the DR1-4-7 is going to be by far the most abundantly represented worldwide.

Finally, we would like to point out the possible relevance of these data in terms of development of epitope based vaccines. Class II restricted HTL have been implicated in protection from, and termination of many important diseases. Inclusion of well defined class II epitopes in prophylactic or therapeutic vaccines may allow the immune response to focus towards conserved or subdominant epitopes, and avoid suppressive determinants. Based on the data presented herein (Table IV), the DR1-4-7 supertype would allow coverage in the 50 to 80% range, depending on the ethnicities considered. It is thus possible that broad and not ethnically biased population coverage could be achieved by considering a very limited number of peptide binding specificities.

Based on the results present above, the sequences of various antigens of interest were scanned for the presence of the DR 1-4-7 motifs. Peptides identified using this approach are broadly cross reactive, class II restricted T cell epitopes. Table VIII presents a listing of such peptides derived from various antigens and includes representive epitopes that bind one or more DR alleles at an IC₅₀ of 1000 nM or less. The information in Table VIII includes the antigen from which the peptide was derived, and binding data expressed as IC₅₀ values for the designated DR alleles as shown.

The above examples are provided to illustrate the invention but not to limit its scope. Other variants of the invention will be readily apparent to one of ordinary skill in the art. All publications, patents, and patent applications cited herein are hereby incorporated by reference for all purposes.

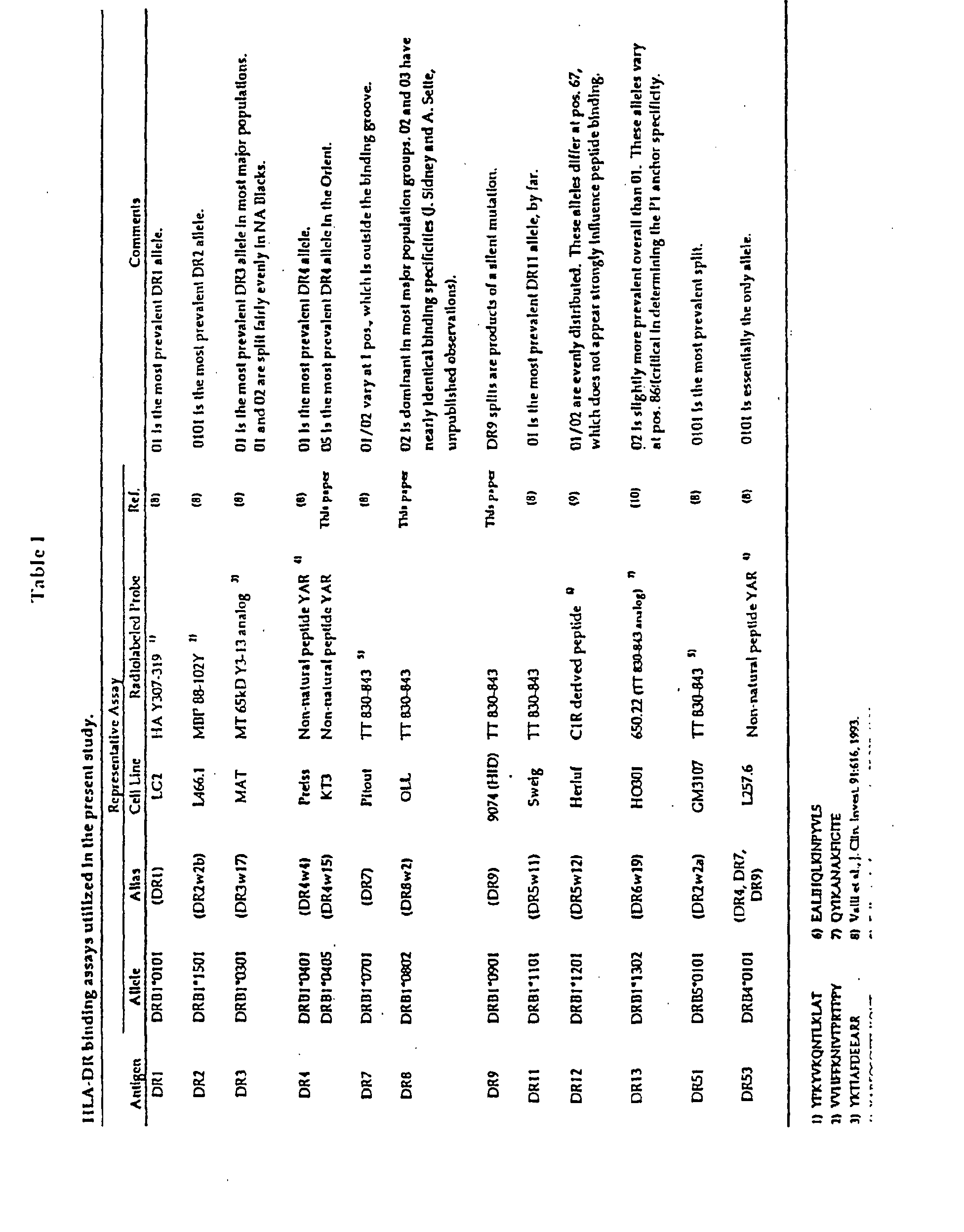

**Table II**

| An algorithm to predict DRB1*0401 binding capacity. a) Original peptide set. | | | | |
|---|---|---|---|---|
| | No. of peptides (Binding nM) | | | |
| Selection Criteria | High ≤100 | Inter. 100-1000 | Non >1000 | Total |
| None | 27 | 53 | 304 | 384 |
| P1-P6 | 27 | 50 | 182 | 259 |
| -17.00 ¹⁾ | 27 | 45 | 84 | 156 |
| -16.44 ²⁾ | 25 | 35 | 47 | 107 |

| | | | | |
|---|---|---|---|---|
| 1) Algorithm score which predicts 90% of all binders. | | | | |
| 2) Algorithm score which predicts 75% of all binders. | | | | |

**Table II**

| b) Blind test of the predictive power of the DRB1*0401 algorithm. | | | | |
|---|---|---|---|---|
| | No. of peptides (Binding nM) | | | |
| Selection Criteria | High ≤100 | Inter. 100-1000 | Non >1000 | Total |
| None | 3 | 11 | 36 | 50 |
| P1-P6 | 3 | 9 | 28 | 40 |
| - 17.00 | 3 | 8 | 7 | 18 |
| - 16.44 | 3 | 4 | 2 | 9 |

**Table III**

| A combined "1-4-7" algorithm. | | |
|---|---|---|
| Selection Criteria | Degenerate Binders ¹⁾ | Percent of Total Degenerate Binders |
| None | 73/384 | 100% |
| P1-P6 | 72/259 | 99% |
| Combined Algorithms (90% Cutoff Value) | 67/147 | 92% |
| Combined Algorithms (75% Cutoff Value) | 59/100 | 81% |

| | | |
|---|---|---|
| 1) Degenerate binders are defined as peprides binding at least two out of the three DR1, 4w4, and 7 molecules with an IC50 of 1 µM or less. | | |

**Table IV**

| Phenotypic frequencies of 10 prevalent HLA-DR antigens | | | | | | | |
|---|---|---|---|---|---|---|---|
| | | Phenotypic Frequencies | | | | | |
| Antigen | Alleles | Cauc. | Blk | Jpn. | Chn. | Hisp. | Avg. |
| DR1 | DRB1*0101-03 | 18.5 | 8.4 | 10.7 | 4.5 | 10.1 | 10.4 |
| DR2 | DRB1*150103 | 19.9 | 14.8 | 30.9 | 22.0 | 15.0 | 20.5 |
| DR3 | DRB1*0301-2 | 17.7 | 19.5 | 0.4 | 7.3 | 14.4 | 11.9 |
| DR4 | DRB1*0401-12 | 23.6 | 6.1 | 40.4 | 21.9 | 29.8 | 24.4 |
| DR7 | DRB1*0701-02 | 26.2 | 11.1 | 1.0 | 15.0 | 16.6 | 14.0 |
| DR8 | DRB1*0801-5 | 5.5 | 10.9 | 25.0 | 10.7 | 23.3 | 15.1 |
| DR9 | DRB1*09011,09012 | 3.6 | 4.7 | 24.5 | 19.9 | 6.7 | 11.9 |
| DR11 | DRBT1*1101-05 | 17.0 | 18.0 | 4.9 | 19.4 | 18.1 | 15.5 |
| DR12 | DRB1*1201-02 | 2-8 | 5.5 | 13.1 | 17.6 | 5.7 | 8.9 |
| DR13 | DRB1*1301-06 | 21.7 | 16.5 | 14.6 | 12.2 | 10.5 | 15.1 |
| Total | | 97.0 | 83.9 | 98.8 | 95.5 | 95.6 | 94.7 |

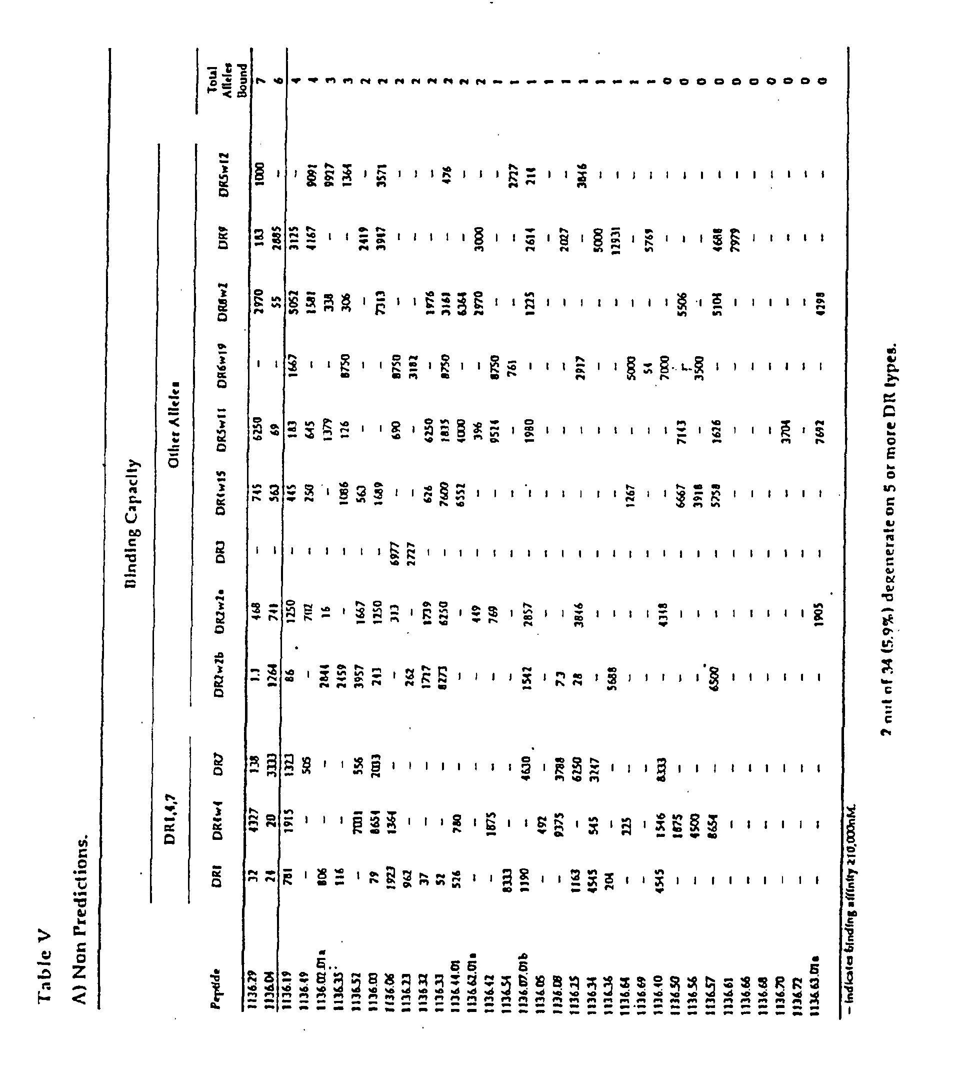

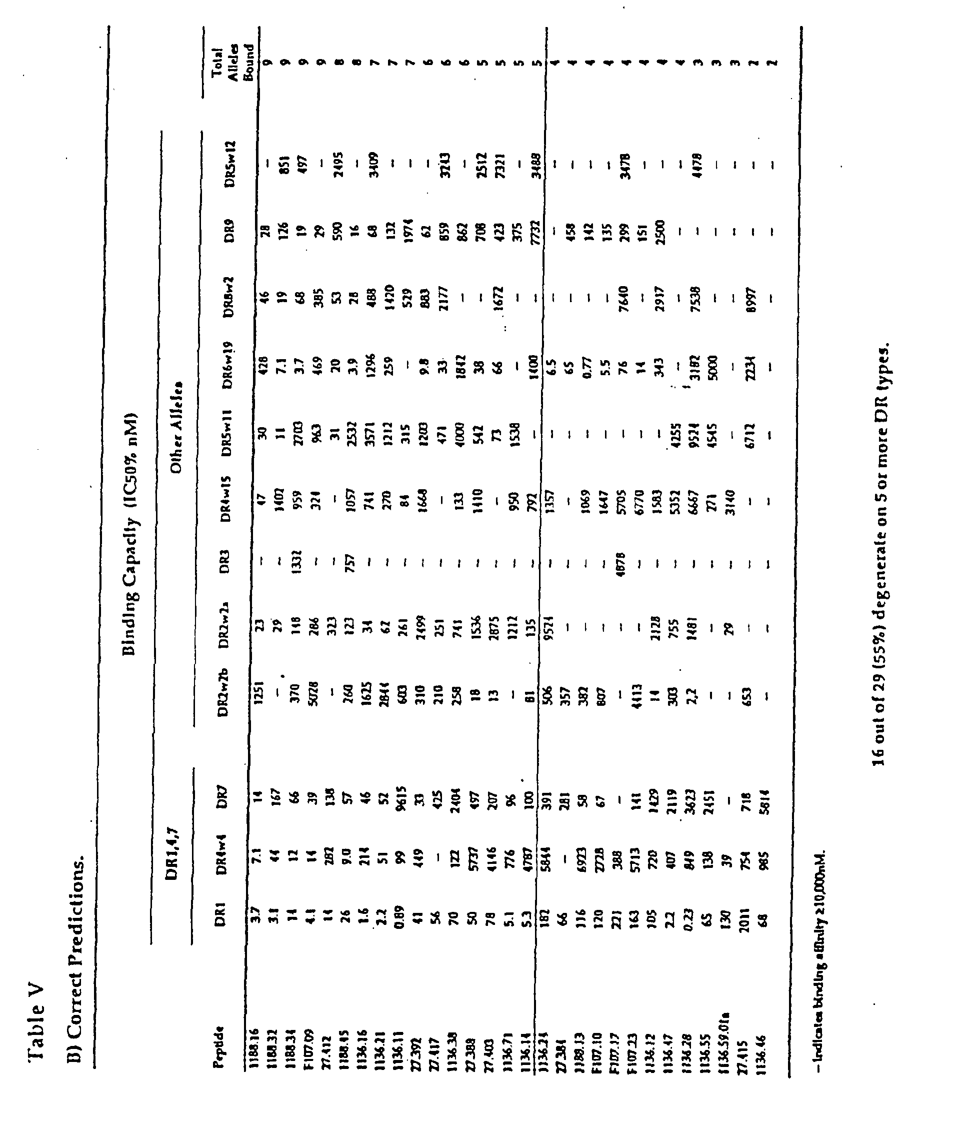

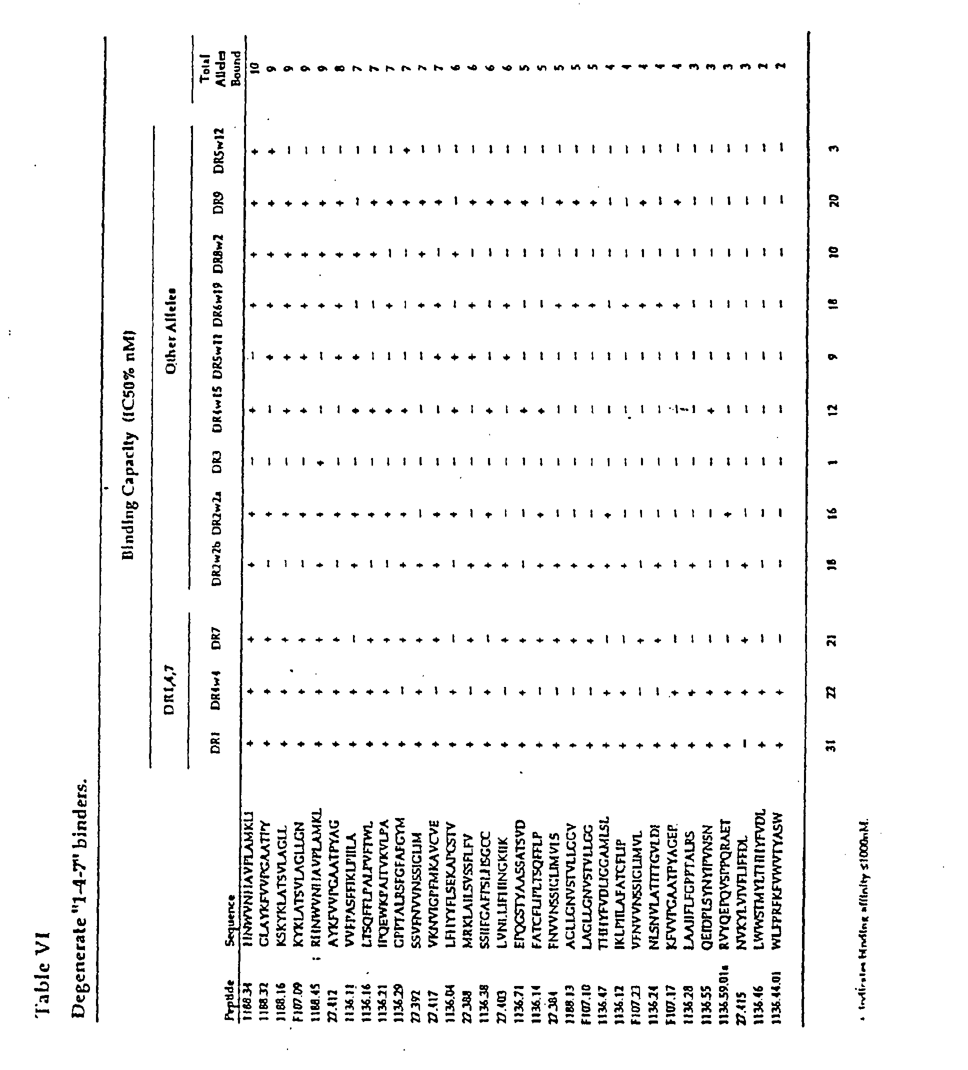

**Table VII**

| | Frequency of Binders | |
|---|---|---|
| DR Type | 1-4-7 Degenerate Binders (%) | Non 1-4-7 Degenerate Binders (%) |
| 1 | 31/32 (97) | 17/67 (25) |
| 4w4 | 22/32 (69) | 8/67 (12) |
| 7 | 21/32 (66) | 7/67 (10) |
| 9 | 20/32 (62) | 2/67 (3.0) |
| 6w19 | 18/32 (56) | 6/67 (8.9) |
| 2w2βb | 18/32 (56) | 16/67 (24) |
| 2w2βa | 16/32 (50) | 10/67 (15) |
| 4w15 | 12/32 (37) | 4/67 (6.0) |
| 8w2 | 10/32 (31) | 3/67 (45) |
| 5w11 | 9/32 (28) | 6/67 (8.9) |
| 5w12 | 3/32 (9.4) | 4/67 (6.0) |
| 3w17 | 1/32 (3.1) | 0/67 (0) |
| w53 | 2/16 (13) | 7/43 (16) |

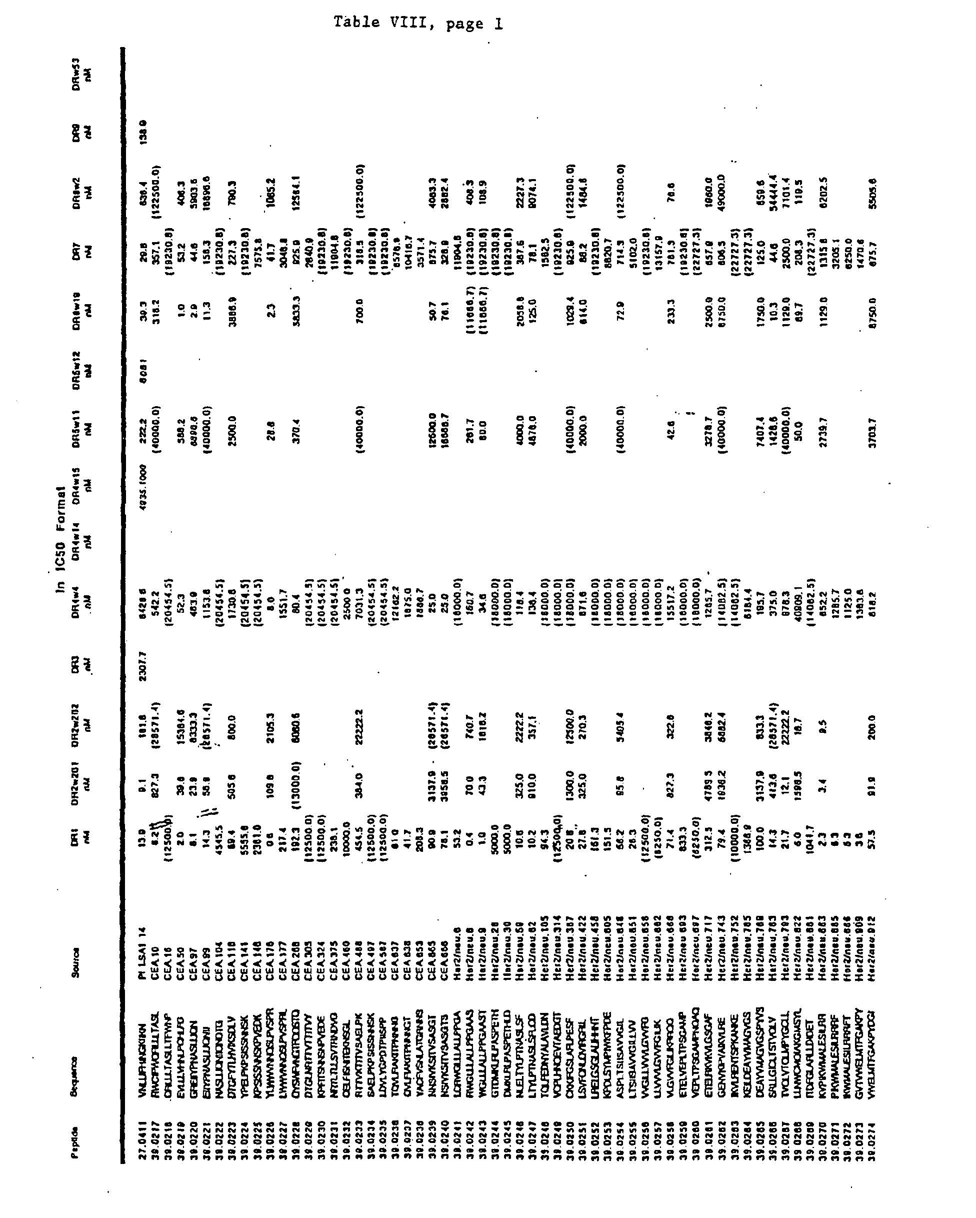

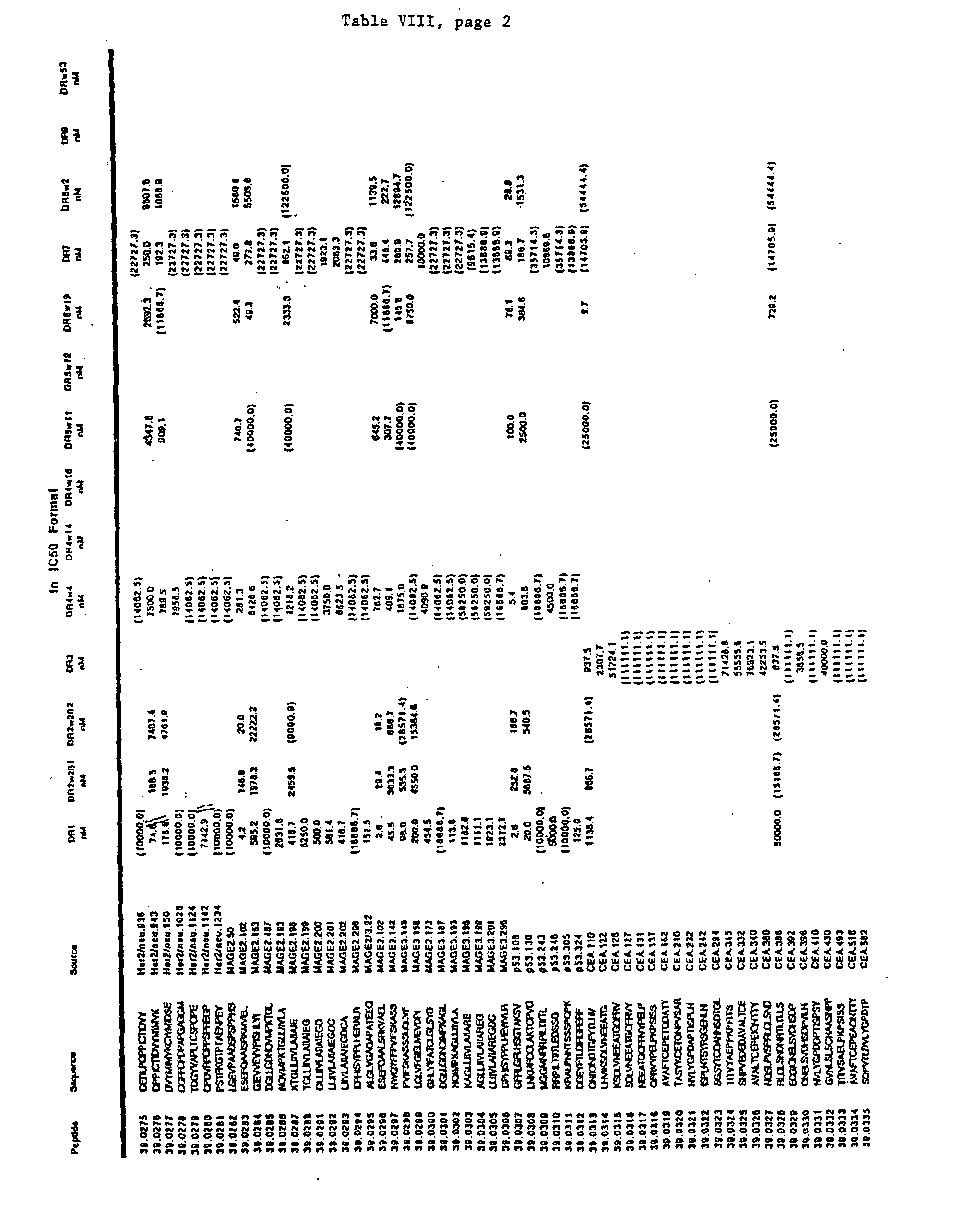

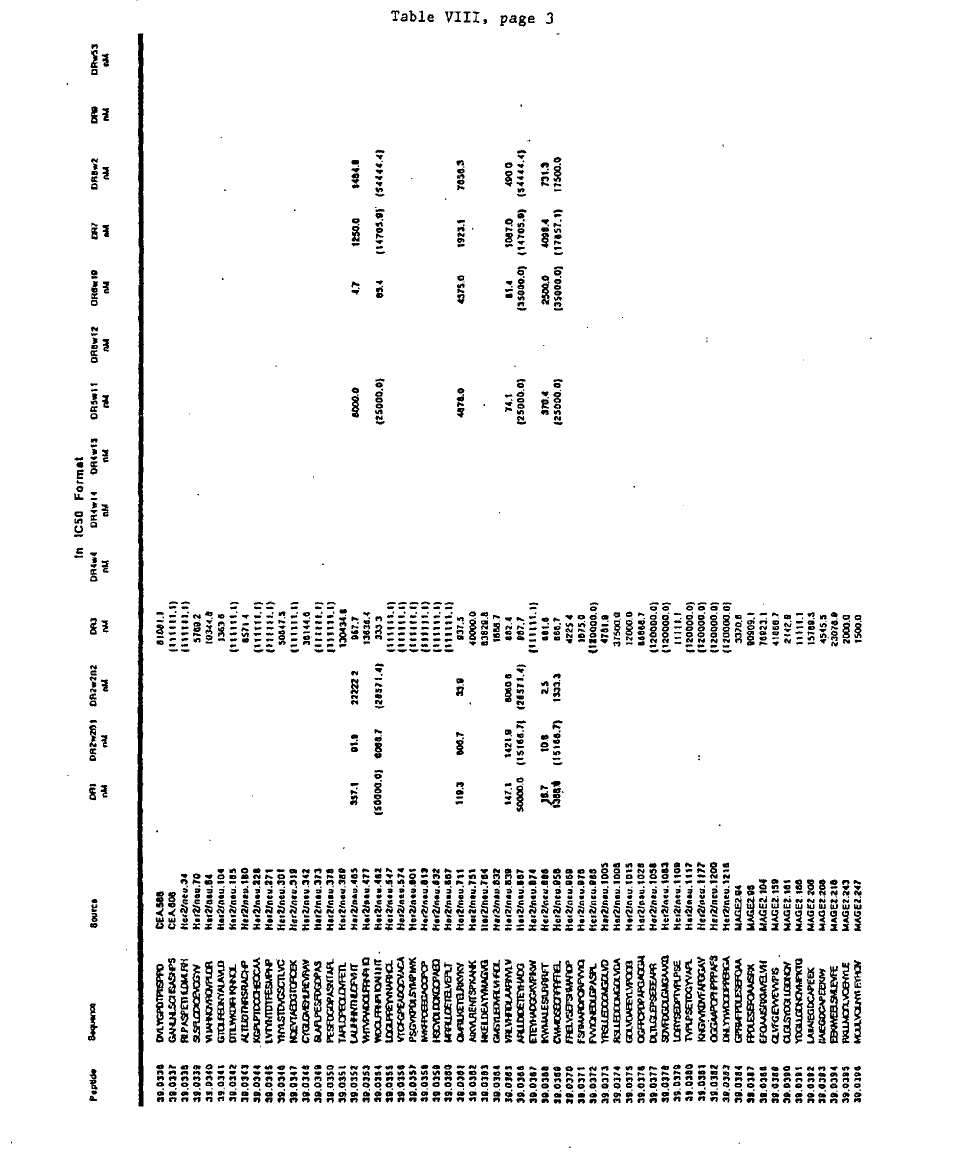

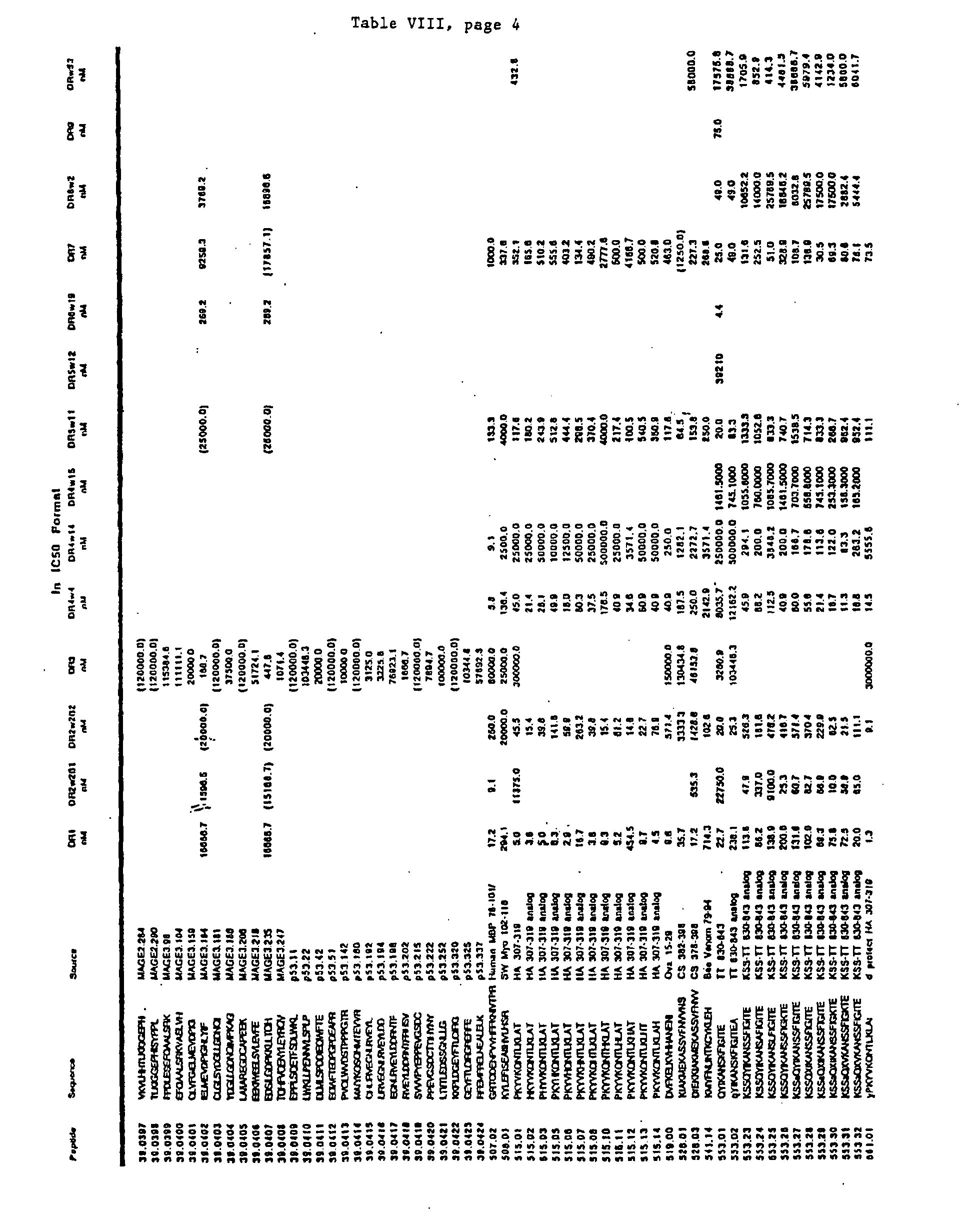

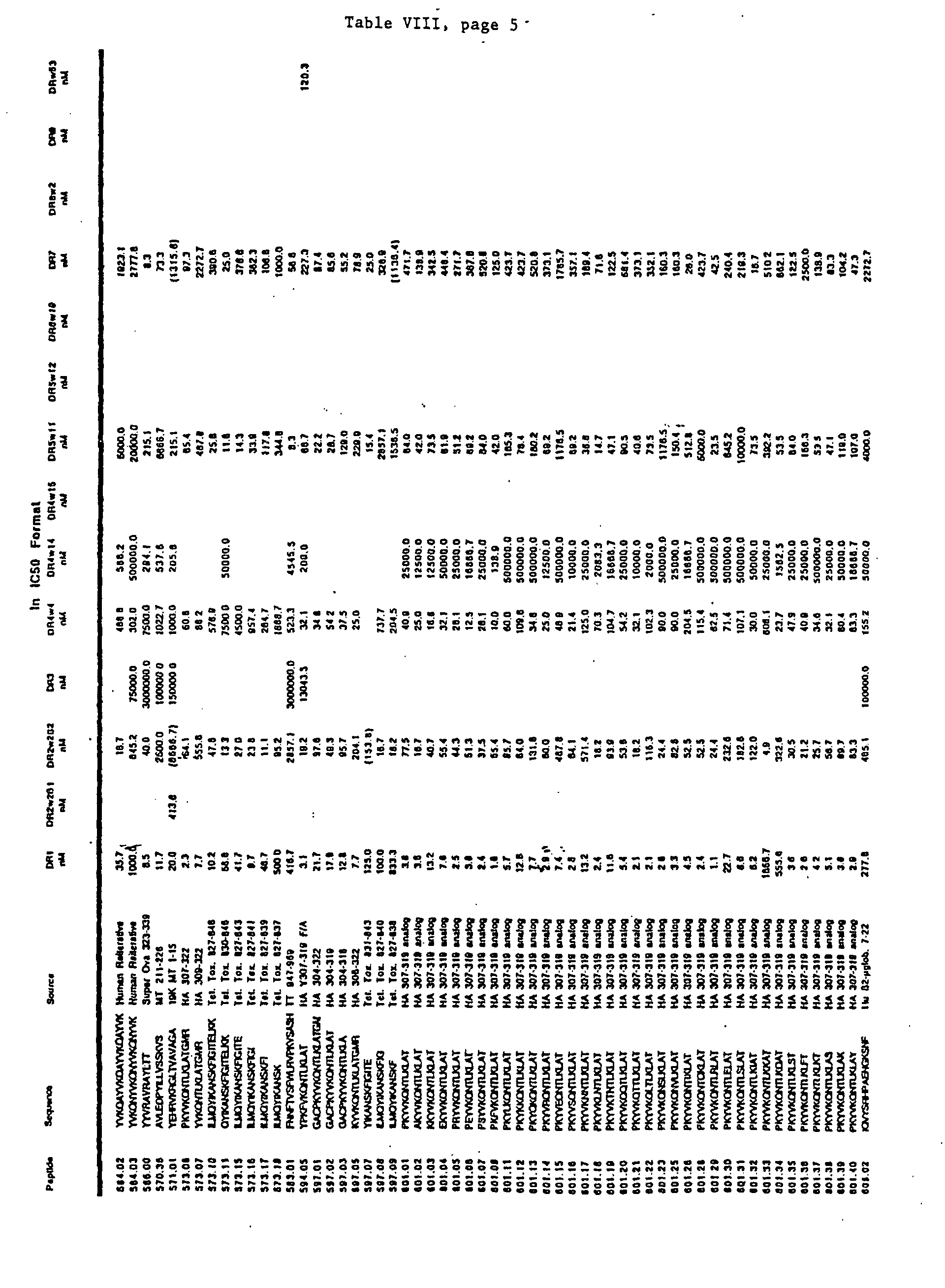

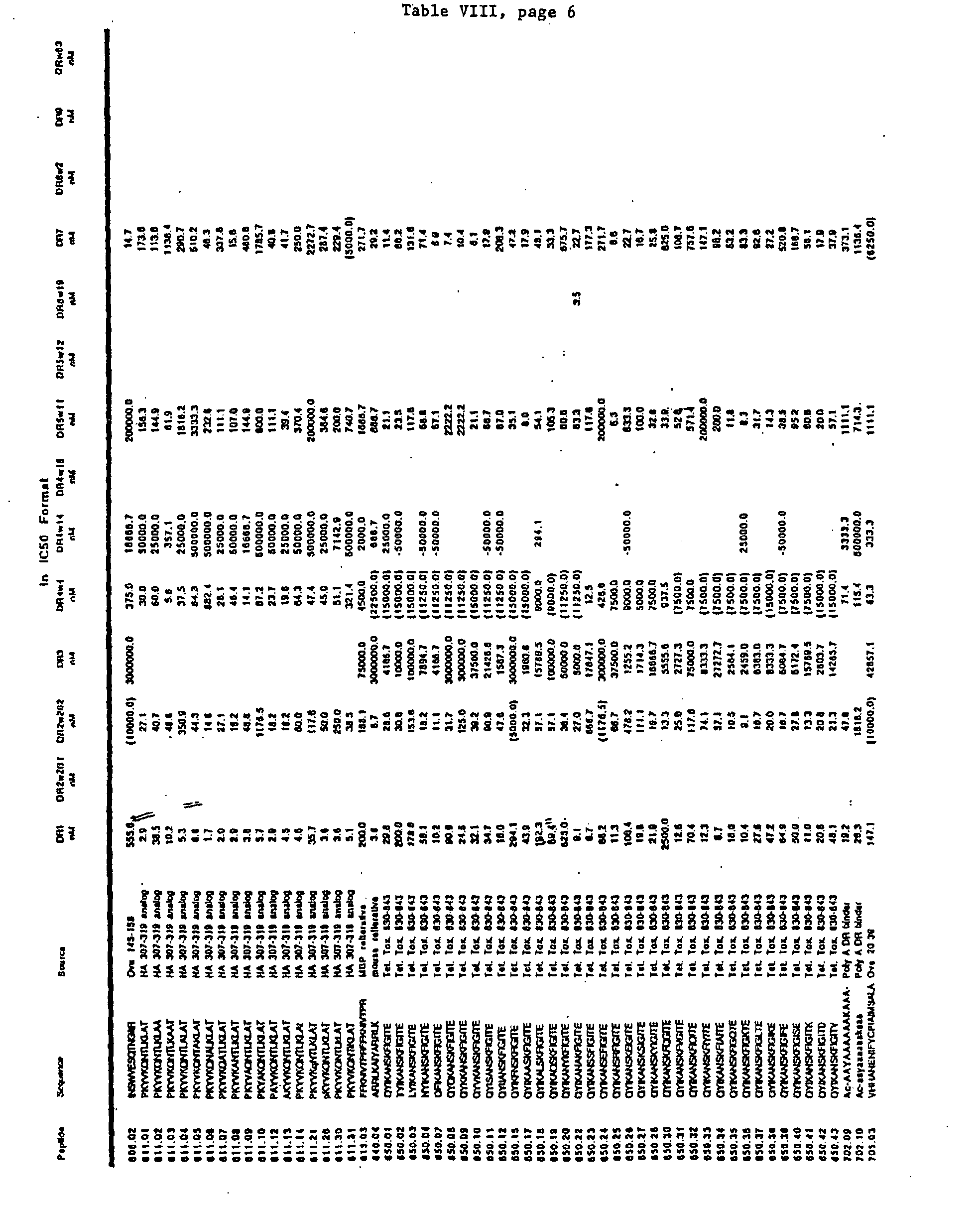

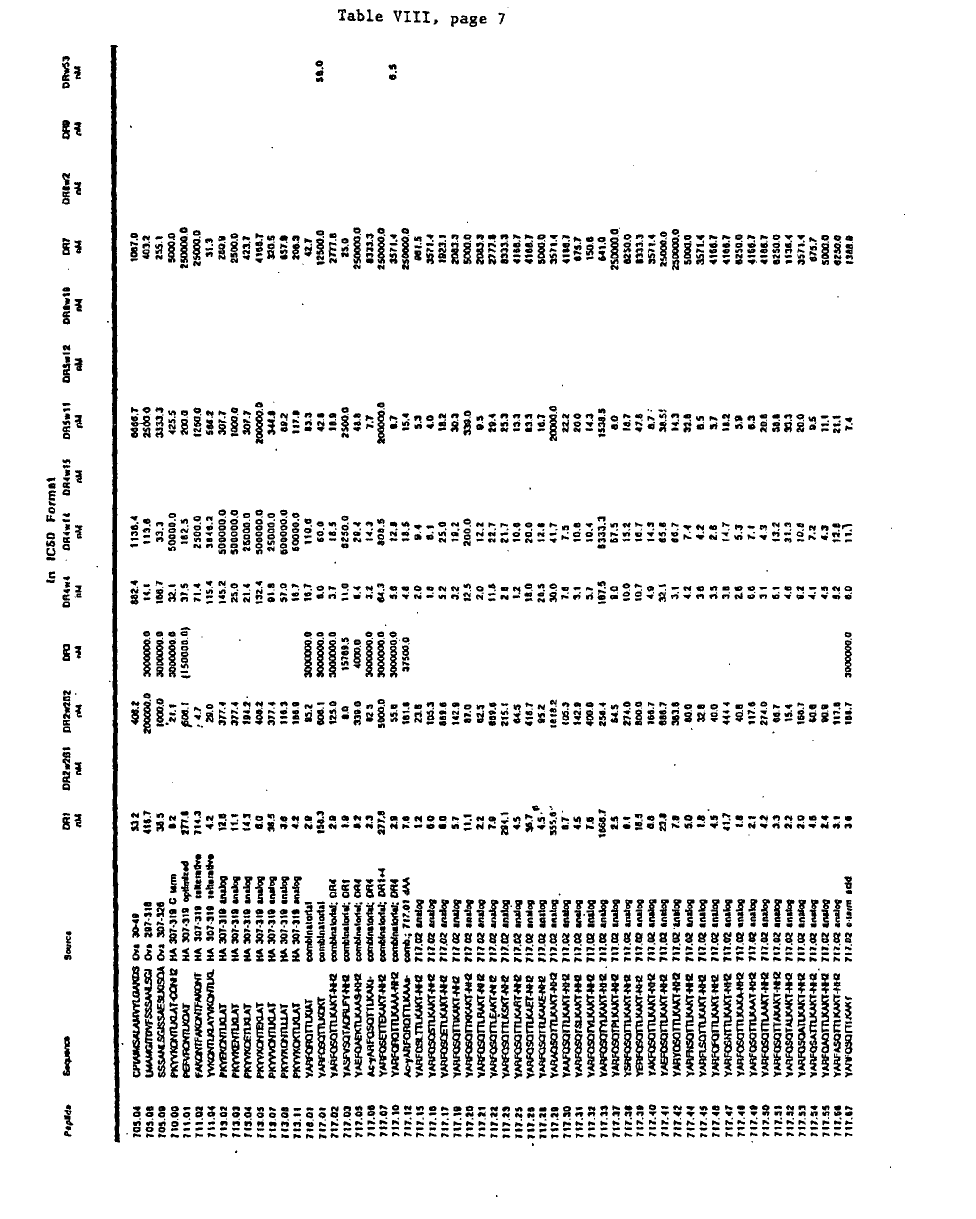

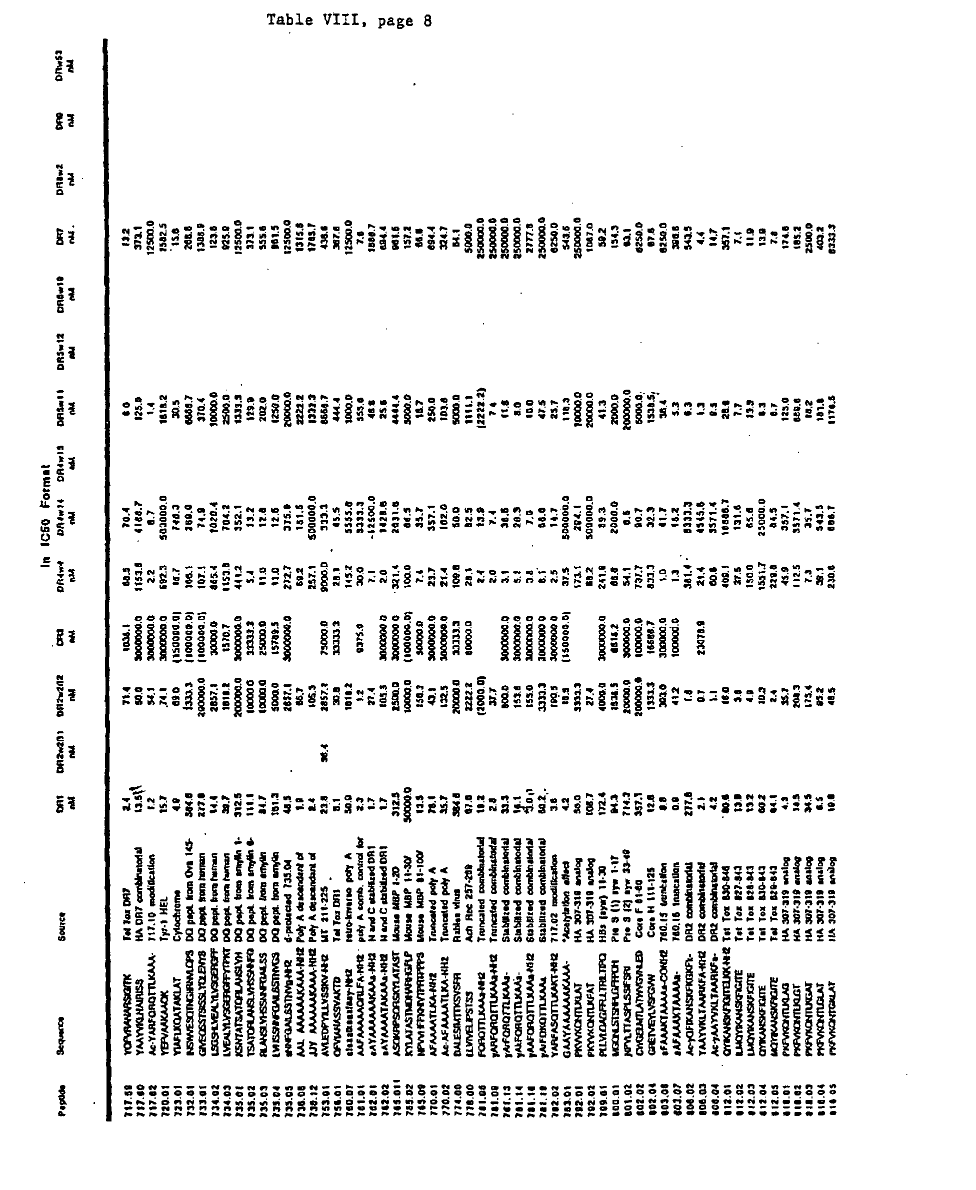

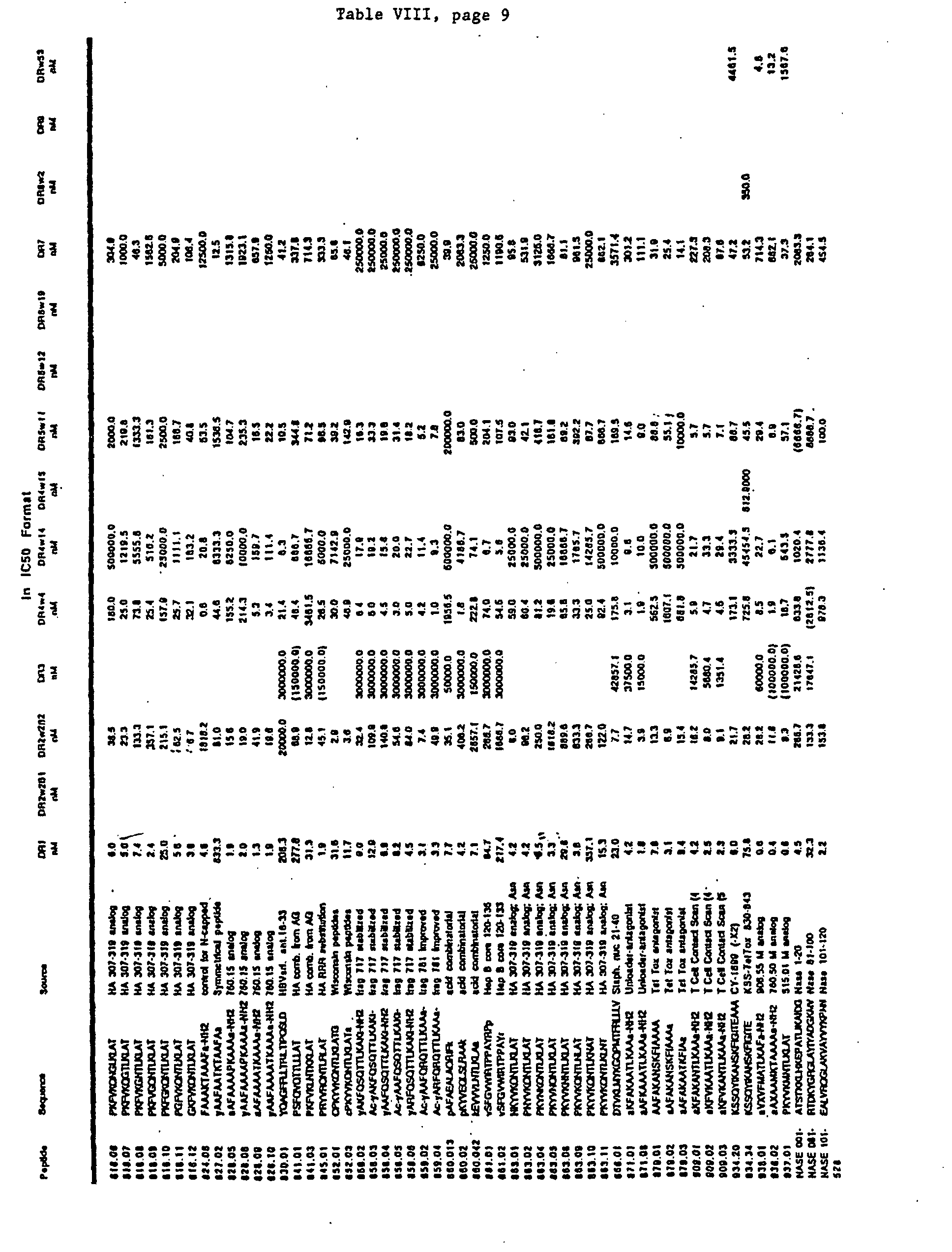

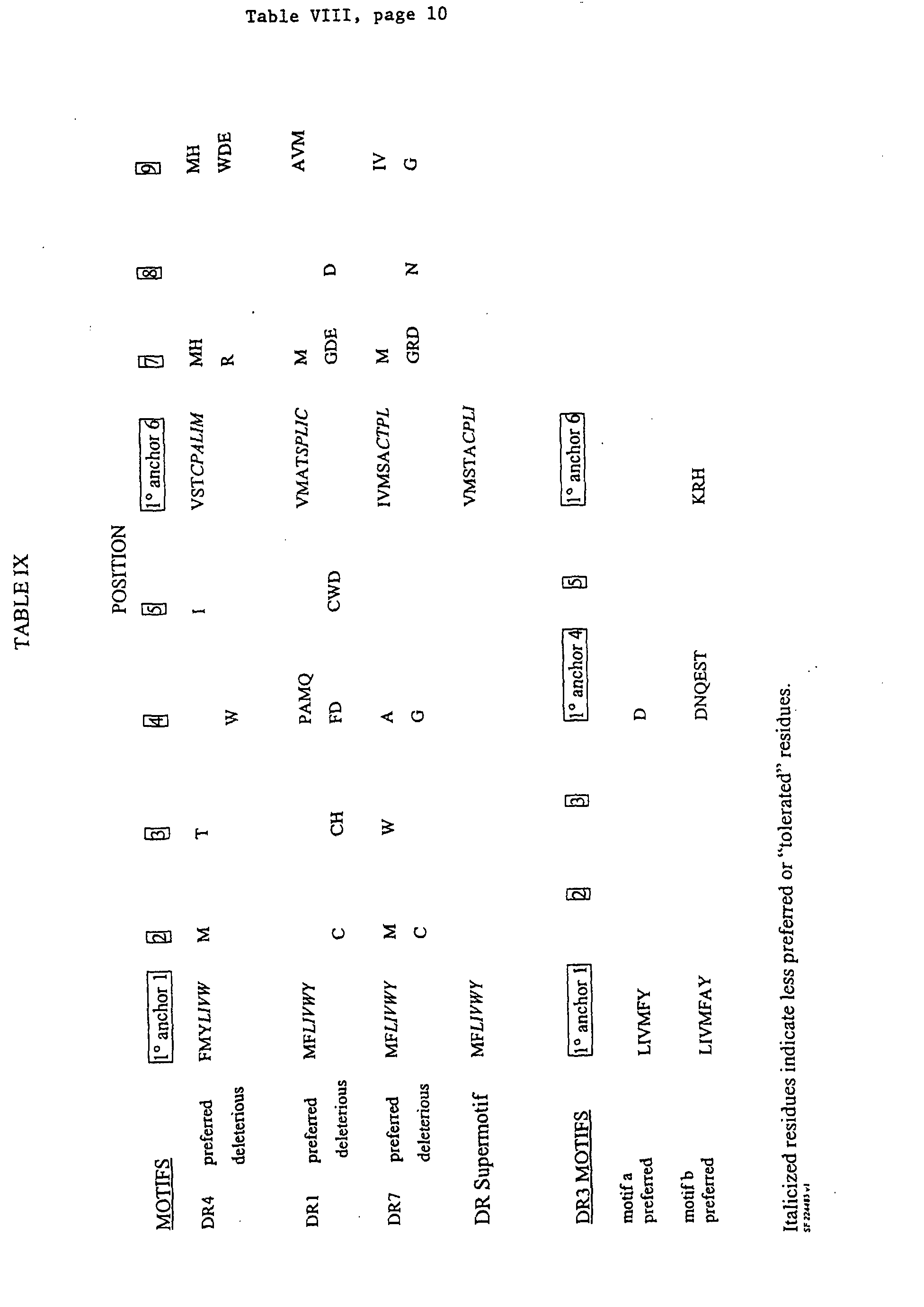

## Claims

1. A composition comprising a peptide less than 50 amino acids in length, wherein the peptide comprises the amino acid sequence EVIILVHNLPQHLFG.

2. A composition comprising a peptide consisting of the amino acid sequence EVIILVHNLPQHLFG.

3. A composition comprising a peptide less than 50 amino acids in length, wherein the peptide comprises an amino acid sequence from Table VIII derived from carcinoembryonic antigen (CEA).

4. A composition comprising a peptide consisting of an amino acid sequence from Table VIII derived from carcinoembryonic antigen (CEA).

5. A composition comprising a peptide of any one of claims 1 to 4 bound to an HLA class II molecule.

6. A composition comprising a nucleic acid that encodes a peptide of any one of claims 1 to 4.

7. The composition of claim 3 or 4, wherein the peptide binds to an HLA class II molecule at an IC₅₀ less than or equal to 1,000nM.

8. The composition of any one of claims 1 to 4, wherein the composition comprises the peptide in an amount between 500 micrograms to 50,000 micrograms.

9. The composition of claim 1 or 3, wherein the peptide is about 12 to about 25 amino acids in length.

10. The composition of claim 9, wherein the peptide is about 15 to about 20 amino acids in length.

11. A method for inducing a helper T cell response in a subject, which comprises contacting a helper T cell with a composition of any one of claims 1 to 10.

12. A pharmaceutical composition comprising a unit dose form or a peptide comprising an epitope from Table VIII or analog thereof which binds to an HLA class II molecule at an IC₅₀ of less than or equal to 1,000 nM.

13. The composition of claim 12, wherein the peptide is derived from a tumor antigen which is selected from the group consisting of carcinoembryonic antigen (CEA), p53, MAGE-2, MAGE-3, or Her2/neu.

14. The composition of claim 12, wherein the immunogenic peptide is derived from a viral antigen.

15. The composition of claim 14, wherein the viral antigen is from HIV, HBV, or HCV.

16. The composition of claim 15, wherein the antigen is *Plasmodium falciparum.*

17. A composition of claim 12 wherein the epitope comprises an amino acid that is Y, F, W, L, I, V, or M at the first position from the N-terminus of the epitope and an amino acid of S, T, C, A, P, V, I, L, or M at the sixth position from the N-terminus of the epitope.

18. A composition of claim 12 wherein the composition is a nucleic acid that encodes the peptide.

19. A method of inducing a helper T cell response in a patient, the method comprising contacting a helper T cell with a composition of claim 12.

20. The method of claim 19, wherein the composition is a nucleic acid that encodes the peptide.

21. A composition comprising an epitope from Table VIII or analog thereof which binds to an HLA class II molecule at an IC₅₀ of less than or equal to 1,000 nM wherein the epitope is bound to an HLA class II molecule present on an antigen presenting cell.

22. A composition that comprises at least two peptides of claim 12.

23. A composition that comprises at least three peptide of claim 12.

24. A composition of claim 12, wherein a unit dose form of the peptide is in the range of between 500 µg and 50,000 µg.


SEQUENCE LISTING

<110> EPIMMUNE, INC.

<120> Identification of broadly reactive DR restricted
      epitopes

<130> JEC/FP5895636

<140> EP 99926084.7
<141> 1999-05-28

<150> PCT/US99/12066
<151> 1999-05-28

<150> US 60/087,192
<151> 1998-05-29

<160> 564

<170> FastSEQ for Windows Version 4.0

<210> 1
<211> 15
<212> PRT
<213> Homo sapiens

<400> 1
His Asn Trp Val Asn His Ala Val Pro Leu Ala Met Lys Leu Ile
 1               5                   10                  15

<210> 2
<211> 15
<212> PRT
<213> Homo sapiens

<400> 2

Gly Leu Ala Tyr Lys Phe Val Val Pro Gly Ala Ala Thr Pro Tyr
1               5                   10                  15

<210> 3
<211> 15
<212> PRT
<213> Homo sapiens

<400> 3

Lys Ser Lys Tyr Lys Leu Ala Thr Ser Val Leu Ala Gly Leu Leu
1               5                   10                  15

<210> 4
<211> 15
<212> PRT
<213> Homo sapiens

<400> 4

Lys Tyr Lys Leu Ala Thr Ser Val Leu Ala Gly Leu Leu Gly Asn
1               5                   10                  15

<210> 5
<211> 15
<212> PRT
<213> Homo sapiens

<400> 5

Arg His Asn Trp Val Asn His Ala Val Pro Leu Ala Met Lys Leu
1               5                   10                  15

<210> 6
<211> 15
<212> PRT
<213> Homo sapiens

<400> 6

Ala Tyr Lys Phe Val Val Pro Gly Ala Ala Thr Pro Tyr Ala Gly

1               5                   10                  15

<210> 7

<211> 16

<212> PRT

<213> Homo sapiens

<400> 7

Val Val Phe Pro Ala Ser Phe Phe Ile Lys Leu Pro Ile Ile Leu Ala

1               5                   10                  15

<210> 8

<211> 16

<212> PRT

<213> Homo sapiens

<400> 8

Leu Thr Ser Gln Phe Phe Leu Pro Ala Leu Pro Val Phe Thr Trp Leu

1               5                   10                  15

<210> 9

<211> 16

<212> PRT

<213> Homo sapiens

<400> 9

Ile Pro Gln Glu Trp Lys Pro Ala Ile Thr Val Lys Val Leu Pro Ala

1               5                   10                  15

<210> 10

<211> 16

<212> PRT

<213> Homo sapiens

<400> 10

Gly Pro Pro Thr Ala Leu Arg Ser Phe Gly Phe Ala Phe Gly Tyr Met

1               5                   10                  15

<210> 11

<211> 15

<212> PRT

<213> Homo sapiens

<400> 11

Ser Ser Val Phe Asn Val Val Asn Ser Ser Ile Gly Leu Ile Met

1               5                   10                  15

<210> 12

<211> 15

<212> PRT

<213> Homo sapiens

<400> 12

Val Lys Asn Val Ile Gly Pro Phe Met Lys Ala Val Cys Val Glu

1               5                   10                  15

<210> 13

<211> 16

<212> PRT

<213> Homo sapiens

<400> 13

Leu Phe His Tyr Tyr Phe Leu Ser Glu Lys Ala Pro Gly Ser Thr Val

1               5                   10                  15

<210> 14

<211> 15

<212> PRT

<213> Homo sapiens

<400> 14

Met Arg Lys Leu Ala Ile Leu Ser Val Ser Ser Phe Leu Phe Val
1               5                   10                  15

<210> 15

<211> 16

<212> PRT

<213> Homo sapiens

<400> 15

Ser Ser Ile Ile Phe Gly Ala Phe Pro Ser Leu His Ser Gly Cys Cys
1               5                   10                  15

<210> 16

<211> 15

<212> PRT

<213> Homo sapiens

<400> 16

Leu Val Asn Leu Leu Ile Phe His Ile Asn Gly Lys Ile Ile Lys
1               5                   10                  15

<210> 17

<211> 16

<212> PRT

<213> Homo sapiens

<400> 17

Glu Pro Gln Gly Ser Thr Tyr Ala Ala Ser Ser Ala Thr Ser Val Asp
1               5                   10                  15

<210> 18
<211> 16
<212> PRT
<213> Homo sapiens

<400> 18
Phe Ala Thr Cys Phe Leu Ile Pro Leu Thr Ser Gln Phe Phe Leu Pro
1               5                   10                  15

<210> 19
<211> 15
<212> PRT
<213> Homo sapiens

<400> 19
Phe Asn Val Val Asn Ser Ser Ile Gly Leu Ile Met Val Leu Ser
1               5                   10                  15

<210> 20
<211> 15
<212> PRT
<213> Homo sapiens

<400> 20
Ala Gly Leu Leu Gly Asn Val Ser Thr Val Leu Leu Gly Gly Val
1               5                   10                  15

<210> 21
<211> 15
<212> PRT
<213> Homo sapiens

<400> 21
Leu Ala Gly Leu Leu Gly Asn Val Ser Thr Val Leu Leu Gly Gly
1               5                   10                  15

<210> 22

<211> 16

<212> PRT

<213> Homo sapiens

<400> 22

Thr His His Tyr Phe Val Asp Leu Ile Gly Gly Ala Met Leu Ser Leu
1               5                   10                  15

<210> 23

<211> 16

<212> PRT

<213> Homo sapiens

<400> 23

Ile Lys Leu Pro Ile Ile Leu Ala Phe Ala Thr Cys Phe Leu Ile Pro
1               5                   10                  15

<210> 24

<211> 15

<212> PRT

<213> Homo sapiens

<400> 24

Val Phe Asn Val Val Asn Ser Ser Ile Gly Leu Ile Met Val Leu
1               5                   10                  15

<210> 25

<211> 16

<212> PRT

<213> Homo sapiens

<400> 25

Asn Leu Ser Asn Val Leu Ala Thr Ile Thr Thr Gly Val Leu Asp Ile
1               5                   10                  15

<210> 26

<211> 15

<212> PRT

<213> Homo sapiens

<400> 26

Lys Phe Val Val Pro Gly Ala Ala Thr Pro Tyr Ala Gly Glu Pro
1               5                   10                  15

<210> 27

<211> 16

<212> PRT

<213> Homo sapiens

<400> 27

Leu Ala Ala Ile Ile Phe Leu Phe Gly Pro Pro Thr Ala Leu Arg Ser
1               5                   10                  15

<210> 28

<211> 16

<212> PRT

<213> Homo sapiens

<400> 28

Gln Glu Ile Asp Pro Leu Ser Tyr Asn Tyr Ile Pro Val Asn Ser Asn
1               5                   10                  15

<210> 29

<211> 16

<212> PRT

<213> Homo sapiens

<400> 29

Arg Val Tyr Gln Glu Pro Gln Val Ser Pro Pro Gln Arg Ala Glu Thr
1               5                   10                  15

<210> 30

<211> 15

<212> PRT

<213> Homo sapiens

<400> 30

Asn Val Lys Tyr Leu Val Ile Val Phe Leu Ile Phe Phe Asp Leu
1               5                   10                  15

<210> 31

<211> 16

<212> PRT

<213> Homo sapiens

<400> 31

Leu Trp Trp Ser Thr Met Tyr Leu Thr His His Tyr Phe Val Asp Leu
1               5                   10                  15

<210> 32

<211> 16

<212> PRT

<213> Homo sapiens

<400> 32

Trp Leu Phe Pro Arg Phe Lys Phe Val Trp Val Thr Tyr Ala Ser Trp
1               5                   10                  15

<210> 33

<211> 15

<212> PRT

<213> Homo sapiens

<400> 33

Val Asn Leu Leu Ile Phe His Ile Asn Gly Lys Ile Ile Lys Asn
1               5                   10                  15

<210> 34

<211> 15

<212> PRT

<213> Homo sapiens

<400> 34

Arg Trp Cys Ile Pro Trp Gln Arg Leu Leu Leu Thr Ala Ser Leu
1               5                   10                  15

<210> 35

<211> 15

<212> PRT

<213> Homo sapiens

<400> 35

Gln Arg Leu Leu Leu Thr Ala Ser Leu Leu Thr Phe Trp Asn Pro
1               5                   10                  15

<210> 36

<211> 15

<212> PRT

<213> Homo sapiens

<400> 36

Glu Val Leu Leu Leu Val His Asn Leu Pro Gln His Leu Phe Gly
1               5                   10                  15

<210> 37

<211> 15

<212> PRT

<213> Homo sapiens

<400> 37

Gly Arg Glu Ile Ile Tyr Pro Asn Ala Ser Leu Leu Ile Gln Asn
1               5                   10                  15

<210> 38

<211> 15

<212> PRT

<213> Homo sapiens

<400> 38

Glu Ile Ile Tyr Pro Asn Ala Ser Leu Leu Ile Gln Asn Ile Ile
1               5                   10                  15

<210> 39

<211> 15

<212> PRT

<213> Homo sapiens

<400> 39

Asn Ala Ser Leu Leu Ile Gln Asn Ile Ile Gln Asn Asp Thr Gly
1               5                   10                  15

<210> 40

<211> 15

<212> PRT

<213> Homo sapiens

<400> 40

Asp Thr Gly Phe Tyr Thr Leu His Val Ile Lys Ser Asp Leu Val
1               5                   10                  15

<210> 41

<211> 15

<212> PRT

<213> Homo sapiens

<400> 41

Tyr Pro Glu Leu Pro Lys Pro Ser Ile Ser Ser Asn Asn Ser Lys
1               5                   10                  15

<210> 42

<211> 15

<212> PRT

<213> Homo sapiens

<400> 42

Lys Pro Ser Ile Ser Ser Asn Asn Ser Lys Pro Val Glu Asp Lys
1               5                   10                  15

<210> 43

<211> 15

<212> PRT

<213> Homo sapiens

<400> 43

Tyr Leu Trp Trp Val Asn Asn Gln Ser Leu Pro Val Ser Pro Arg
1               5                   10                  15

<210> 44

<211> 15

<212> PRT

<213> Homo sapiens

<400> 44

Leu Trp Trp Val Asn Asn Gln Ser Leu Pro Val Ser Pro Arg Leu
1               5                   10                  15

<210> 45

<211> 15

<212> PRT

<213> Homo sapiens

<400> 45

Gln Tyr Ser Trp Phe Val Asn Gly Thr Phe Gln Gln Ser Thr Gln
1               5                   10                  15

<210> 46

<211> 15

<212> PRT

<213> Homo sapiens

<400> 46

Asp Thr Gly Leu Asn Arg Thr Thr Val Thr Thr Ile Thr Val Tyr
1               5                   10                  15

<210> 47

<211> 15

<212> PRT

<213> Homo sapiens

<400> 47

Lys Pro Phe Ile Thr Ser Asn Asn Ser Asn Pro Val Glu Asp Glu
1               5                   10                  15

<210> 48

<211> 15

<212> PRT

<213> Homo sapiens

<400> 48

Asn Arg Thr Leu Thr Leu Leu Ser Val Thr Arg Asn Asp Val Gly
1               5                   10                  15

<210> 49

<211> 15

<212> PRT

<213> Homo sapiens

<400> 49

Gln Glu Leu Phe Ile Ser Asn Ile Thr Glu Lys Asn Ser Gly Leu
1               5                   10                  15

<210> 50

<211> 15

<212> PRT

<213> Homo sapiens

<400> 50

Arg Thr Thr Val Lys Thr Ile Thr Val Ser Ala Glu Leu Pro Lys
1               5                   10                  15

<210> 51

<211> 15

<212> PRT

<213> Homo sapiens

<400> 51

Ser Ala Glu Leu Pro Lys Pro Ser Ile Ser Ser Asn Asn Ser Lys
1               5                   10                  15

<210> 52

<211> 15

<212> PRT

<213> Homo sapiens

<400> 52

Leu Asp Val Leu Tyr Gly Pro Asp Thr Pro Ile Ile Ser Pro Pro
1               5                   10                  15

<210> 53

<211> 15

<212> PRT

<213> Homo sapiens

<400> 53

Thr Gln Val Leu Phe Ile Ala Lys Ile Thr Pro Asn Asn Asn Gly
1               5                   10                  15

<210> 54

<211> 15

<212> PRT

<213> Homo sapiens

<400> 54

Gln Val Leu Phe Ile Ala Lys Ile Thr Pro Asn Asn Asn Gly Thr
1               5                   10                  15

<210> 55

<211> 15

<212> PRT

<213> Homo sapiens

<400> 55

Tyr Ala Cys Phe Val Ser Asn Leu Ala Thr Gly Arg Asn Asn Ser
1               5                   10                  15

<210> 56

<211> 15

<212> PRT

<213> Homo sapiens

<400> 56

Asn Asn Ser Ile Val Lys Ser Ile Thr Val Ser Ala Ser Gly Thr
1               5                   10                  15

<210> 57

<211> 15

<212> PRT

<213> Homo sapiens

<400> 57

Asn Ser Ile Val Lys Ser Ile Thr Val Ser Ala Ser Gly Thr Ser
1               5                   10                  15

<210> 58

<211> 15

<212> PRT

<213> Homo sapiens

<400> 58

Leu Cys Arg Trp Gly Leu Leu Leu Ala Leu Leu Pro Pro Gly Ala
1               5                   10                  15

<210> 59

<211> 15

<212> PRT

<213> Homo sapiens

<400> 59

Arg Trp Gly Leu Leu Leu Ala Leu Leu Pro Pro Gly Ala Ala Ser
1               5                   10                  15

<210> 60

<211> 15

<212> PRT

<213> Homo sapiens

<400> 60

Trp Gly Leu Leu Leu Ala Leu Leu Pro Pro Gly Ala Ala Ser Thr
1               5                   10                  15

<210> 61

<211> 15

<212> PRT

<213> Homo sapiens

<400> 61

Gly Thr Asp Met Lys Leu Arg Leu Pro Ala Ser Pro Glu Thr His
1               5                   10                  15

<210> 62

<211> 15

<212> PRT

<213> Homo sapiens

<400> 62

Asp Met Lys Leu Arg Leu Pro Ala Ser Pro Glu Thr His Leu Asp
1               5                   10                  15

<210> 63

<211> 15

<212> PRT

<213> Homo sapiens

<400> 63

Asn Leu Glu Leu Thr Tyr Leu Pro Thr Asn Ala Ser Leu Ser Phe
1               5                   10                  15

<210> 64

<211> 15

<212> PRT

<213> Homo sapiens

<400> 64

Leu Thr Tyr Leu Pro Thr Asn Ala Ser Leu Ser Phe Leu Gln Asp
1               5                   10                  15

<210> 65

<211> 15

<212> PRT

<213> Homo sapiens

<400> 65

Thr Gln Leu Phe Glu Asp Asn Tyr Ala Leu Ala Val Leu Asp Asn
1               5                   10                  15

<210> 66

<211> 15

<212> PRT

<213> Homo sapiens

<400> 66

Val Cys Pro Leu His Asn Gln Glu Val Thr Ala Glu Asp Gly Thr
1               5                   10                  15

<210> 67

<211> 15

<212> PRT

<213> Homo sapiens

<400> 67

Cys Lys Lys Ile Phe Gly Ser Leu Ala Phe Leu Pro Glu Ser Phe
1               5                   10                  15

<210> 68

<211> 15

<212> PRT

<213> Homo sapiens

<400> 68

Leu Ser Val Phe Gln Asn Leu Gln Val Ile Arg Gly Arg Ile Leu
1               5                   10                  15

<210> 69

<211> 15

<212> PRT

<213> Homo sapiens

<400> 69

Leu Arg Glu Leu Gly Ser Gly Leu Ala Leu Ile His His Asn Thr
1               5                   10                  15

<210> 70

<211> 15

<212> PRT

<213> Homo sapiens

<400> 70

Lys Pro Asp Leu Ser Tyr Met Pro Ile Trp Lys Phe Pro Asp Glu

1               5                   10                  15

<210> 71

<211> 15

<212> PRT

<213> Homo sapiens

<400> 71

Ala Ser Pro Leu Thr Ser Ile Ile Ser Ala Val Val Gly Ile Leu

1               5                   10                  15

<210> 72

<211> 15

<212> PRT

<213> Homo sapiens

<400> 72

Leu Thr Ser Ile Ile Ser Ala Val Val Gly Ile Leu Leu Val Val

1               5                   10                  15

<210> 73

<211> 15

<212> PRT

<213> Homo sapiens

<400> 73

Val Val Gly Ile Leu Leu Val Val Val Leu Gly Val Val Phe Gly

1               5                   10                  15

<210> 74

<211> 15

<212> PRT

<213> Homo sapiens

<400> 74

Leu Leu Val Val Val Leu Gly Val Val Phe Gly Ile Leu Ile Lys
1               5                   10                  15

<210> 75

<211> 15

<212> PRT

<213> Homo sapiens

<400> 75

Val Leu Gly Val Val Phe Gly Ile Leu Ile Lys Arg Arg Gln Gln
1               5                   10                  15

<210> 76

<211> 15

<212> PRT

<213> Homo sapiens

<400> 76

Glu Thr Glu Leu Val Glu Pro Leu Thr Pro Ser Gly Ala Met Pro
1               5                   10                  15

<210> 77

<211> 15

<212> PRT

<213> Homo sapiens

<400> 77

Val Glu Pro Leu Thr Pro Ser Gly Ala Met Pro Asn Gln Ala Gln
1               5                   10                  15

<210> 78

<211> 15

<212> PRT

<213> Homo sapiens

<400> 78

Glu Thr Glu Leu Arg Lys Val Lys Val Leu Gly Ser Gly Ala Phe
1               5                   10                  15

<210> 79

<211> 15

<212> PRT

<213> Homo sapiens

<400> 79

Gly Glu Asn Val Lys Ile Pro Val Ala Ile Lys Val Leu Arg Glu
1               5                   10                  15

<210> 80

<211> 15

<212> PRT

<213> Homo sapiens

<400> 80

Ile Lys Val Leu Arg Glu Asn Thr Ser Pro Lys Ala Asn Lys Glu
1               5                   10                  15

<210> 81

<211> 15

<212> PRT

<213> Homo sapiens

<400> 81

Lys Glu Ile Leu Asp Glu Ala Tyr Val Met Ala Gly Val Gly Ser
1               5                   10                  15

<210> 82

<211> 15

<212> PRT

<213> Homo sapiens

<400> 82

Asp Glu Ala Tyr Val Met Ala Gly Val Gly Ser Pro Tyr Val Ser
1               5                   10                  15

<210> 83

<211> 15

<212> PRT

<213> Homo sapiens

<400> 83

Ser Arg Leu Leu Gly Ile Cys Leu Thr Ser Thr Val Gln Leu Val
1               5                   10                  15

<210> 84

<211> 15

<212> PRT

<213> Homo sapiens

<400> 84

Thr Val Gln Leu Val Thr Gln Leu Met Pro Tyr Gly Cys Leu Leu
1               5                   10                  15

<210> 85

<211> 15

<212> PRT

<213> Homo sapiens

<400> 85

Leu Leu Asn Trp Cys Met Gln Ile Ala Lys Gly Met Ser Tyr Leu
1               5                   10                  15

<210> 86

<211> 15

<212> PRT

<213> Homo sapiens

<400> 86

Ile Thr Asp Phe Gly Leu Ala Arg Leu Leu Asp Ile Asp Glu Thr
1               5                   10                  15

<210> 87

<211> 15

<212> PRT

<213> Homo sapiens

<400> 87

Lys Val Pro Ile Lys Trp Met Ala Leu Glu Ser Ile Leu Arg Arg
1               5                   10                  15

<210> 88

<211> 15

<212> PRT

<213> Homo sapiens

<400> 88

Pro Ile Lys Trp Met Ala Leu Glu Ser Ile Leu Arg Arg Arg Phe
1               5                   10                  15

<210> 89

<211> 15

<212> PRT

<213> Homo sapiens

<400> 89

Ile Lys Trp Met Ala Leu Glu Ser Ile Leu Arg Arg Arg Phe Thr
1               5                   10                  15

<210> 90

<211> 15

<212> PRT

<213> Homo sapiens

<400> 90

Gly Val Thr Val Trp Glu Leu Met Thr Phe Gly Ala Lys Pro Tyr

1               5                   10                  15

<210> 91

<211> 15

<212> PRT

<213> Homo sapiens

<400> 91

Val Trp Glu Leu Met Thr Phe Gly Ala Lys Pro Tyr Asp Gly Ile

1               5                   10                  15

<210> 92

<211> 15

<212> PRT

<213> Homo sapiens

<400> 92

Gly Glu Arg Leu Pro Gln Pro Pro Ile Cys Thr Ile Asp Val Tyr

1               5                   10                  15

<210> 93

<211> 15

<212> PRT

<213> Homo sapiens

<400> 93

Gln Pro Pro Ile Cys Thr Ile Asp Val Tyr Met Ile Met Val Lys

1               5                   10                  15

<210> 94

<211> 15

<212> PRT

<213> Homo sapiens

<400> 94

Asp Val Tyr Met Ile Met Val Lys Cys Trp Met Ile Asp Ser Glu
1               5                   10                  15

<210> 95

<211> 15

<212> PRT

<213> Homo sapiens

<400> 95

Gln Gly Phe Phe Cys Pro Asp Pro Ala Pro Gly Ala Gly Gly Met
1               5                   10                  15

<210> 96

<211> 15

<212> PRT

<213> Homo sapiens

<400> 96

Thr Asp Gly Tyr Val Ala Pro Leu Thr Cys Ser Pro Gln Pro Glu
1               5                   10                  15

<210> 97

<211> 15

<212> PRT

<213> Homo sapiens

<400> 97

Gln Pro Asp Val Arg Pro Gln Pro Pro Ser Pro Arg Glu Gly Pro
1               5                   10                  15

<210> 98

<211> 15

<212> PRT

<213> Homo sapiens

<400> 98

Pro Ser Thr Phe Lys Gly Thr Pro Thr Ala Glu Asn Pro Glu Tyr
1               5                   10                  15

<210> 99

<211> 15

<212> PRT

<213> Homo sapiens

<400> 99

Leu Gly Glu Val Pro Ala Ala Asp Ser Pro Ser Pro Pro His Ser
1               5                   10                  15

<210> 100

<211> 15

<212> PRT

<213> Homo sapiens

<400> 100

Glu Ser Glu Phe Gln Ala Ala Ile Ser Arg Lys Met Val Glu Leu
1               5                   10                  15

<210> 101

<211> 15

<212> PRT

<213> Homo sapiens

<400> 101

Gly Ile Glu Val Val Glu Val Val Pro Ile Ser His Leu Tyr Ile
1               5                   10                  15

<210> 102

<211> 15

<212> PRT

<213> Homo sapiens

<400> 102

Asp Gly Leu Leu Gly Asp Asn Gln Val Met Pro Lys Thr Gly Leu
1               5                   10                  15

<210> 103

<211> 15

<212> PRT

<213> Homo sapiens

<400> 103

Asn Gln Val Met Pro Lys Thr Gly Leu Leu Ile Ile Val Leu Ala
1               5                   10                  15

<210> 104

<211> 15

<212> PRT

<213> Homo sapiens

<400> 104

Lys Thr Gly Leu Leu Ile Ile Val Leu Ala Ile Ile Ala Ile Glu
1               5                   10                  15

<210> 105

<211> 15

<212> PRT

<213> Homo sapiens

<400> 105

Thr Gly Leu Leu Ile Ile Val Leu Ala Ile Ile Ala Ile Glu Gly
1               5                   10                  15

<210> 106

<211> 15

<212> PRT

<213> Homo sapiens

<400> 106

Gly Leu Leu Ile Ile Val Leu Ala Ile Ile Ala Ile Glu Gly Asp
1               5                   10                  15

<210> 107

<211> 15

<212> PRT

<213> Homo sapiens

<400> 107

Leu Leu Ile Ile Val Leu Ala Ile Ile Ala Ile Glu Gly Asp Cys
1               5                   10                  15

<210> 108

<211> 15

<212> PRT

<213> Homo sapiens

<400> 108

Leu Ile Ile Val Leu Ala Ile Ile Ala Ile Glu Gly Asp Cys Ala
1               5                   10                  15

<210> 109

<211> 15

<212> PRT

<213> Homo sapiens

<400> 109

Glu Pro His Ile Ser Tyr Pro Pro Leu His Glu Arg Ala Leu Arg
1               5                   10                  15

<210> 110

<211> 15

<212> PRT

<213> Homo sapiens

<400> 110

Ala Leu Gly Leu Val Gly Ala Gln Ala Pro Ala Thr Glu Glu Gln
1               5                   10                  15

<210> 111

<211> 15

<212> PRT

<213> Homo sapiens

<400> 111

Glu Ser Glu Phe Gln Ala Ala Leu Ser Arg Lys Val Ala Glu Leu
1               5                   10                  15

<210> 112

<211> 15

<212> PRT

<213> Homo sapiens

<400> 112

Asn Trp Gln Tyr Phe Phe Pro Val Ile Phe Ser Lys Ala Ser Ser
1               5                   10                  15

<210> 113

<211> 15

<212> PRT

<213> Homo sapiens

<400> 113

Pro Val Ile Phe Ser Lys Ala Ser Ser Ser Leu Gln Leu Val Phe
1               5                   10                  15

<210> 114
<211> 15
<212> PRT
<213> Homo sapiens

<400> 114
Leu Gln Leu Val Phe Gly Ile Glu Leu Met Glu Val Asp Pro Ile
1               5                   10                  15

<210> 115
<211> 15
<212> PRT
<213> Homo sapiens

<400> 115
Gly His Leu Tyr Ile Phe Ala Thr Cys Leu Gly Leu Ser Tyr Asp
1               5                   10                  15

<210> 116
<211> 15
<212> PRT
<213> Homo sapiens

<400> 116
Asp Gly Leu Leu Gly Asp Asn Gln Ile Met Pro Lys Ala Gly Leu
1               5                   10                  15

<210> 117
<211> 15
<212> PRT
<213> Homo sapiens

<400> 117
Asn Gln Ile Met Pro Lys Ala Gly Leu Leu Ile Ile Val Leu Ala
1               5                   10                  15

<210> 118
<211> 15
<212> PRT
<213> Homo sapiens

<400> 118
Lys Ala Gly Leu Leu Ile Ile Val Leu Ala Ile Ile Ala Arg Glu
1               5                   10                  15

<210> 119
<211> 15
<212> PRT
<213> Homo sapiens

<400> 119
Ala Gly Leu Leu Ile Ile Val Leu Ala Ile Ile Ala Arg Glu Gly
1               5                   10                  15

<210> 120
<211> 15
<212> PRT
<213> Homo sapiens

<400> 120
Leu Leu Ile Ile Val Leu Ala Ile Ile Ala Arg Glu Gly Asp Cys
1               5                   10                  15

<210> 121
<211> 15
<212> PRT
<213> Homo sapiens

<400> 121
Gly Pro His Ile Ser Tyr Pro Pro Leu His Glu Trp Val Leu Arg
1               5                   10                  15

<210> 122

<211> 15

<212> PRT

<213> Homo sapiens

<400> 122

Gly Phe Arg Leu Gly Phe Leu His Ser Gly Thr Ala Lys Ser Val
1               5                   10                  15

<210> 123

<211> 15

<212> PRT

<213> Homo sapiens

<400> 123

Leu Asn Lys Met Phe Cys Gln Leu Ala Lys Thr Cys Pro Val Gln
1               5                   10                  15

<210> 124

<211> 15

<212> PRT

<213> Homo sapiens

<400> 124

Met Gly Gly Met Asn Arg Arg Pro Ile Leu Thr Ile Ile Thr Leu
1               5                   10                  15

<210> 125

<211> 15

<212> PRT

<213> Homo sapiens

<400> 125

Arg Arg Pro Ile Leu Thr Ile Ile Thr Leu Glu Asp Ser Ser Gly
1               5                   10                  15

<210> 126

<211> 15

<212> PRT

<213> Homo sapiens

<400> 126

Lys Arg Ala Leu Pro Asn Asn Thr Ser Ser Ser Pro Gln Pro Lys
1               5                   10                  15

<210> 127

<211> 15

<212> PRT

<213> Homo sapiens

<400> 127

Asp Gly Glu Tyr Phe Thr Leu Gln Ile Arg Gly Arg Glu Arg Phe
1               5                   10                  15

<210> 128

<211> 15

<212> PRT

<213> Homo sapiens

<400> 128

Gln Asn Ile Ile Gln Asn Asp Thr Gly Phe Tyr Thr Leu His Val
1               5                   10                  15

<210> 129

<211> 15

<212> PRT

<213> Homo sapiens

<400> 129

Leu His Val Ile Lys Ser Asp Leu Val Asn Glu Glu Ala Thr Gly
1               5                   10                  15

<210> 130

<211> 15

<212> PRT

<213> Homo sapiens

<400> 130

Lys Ser Asp Leu Val Asn Glu Glu Ala Thr Gly Gln Phe Arg Val

1               5                   10                  15

<210> 131

<211> 15

<212> PRT

<213> Homo sapiens

<400> 131

Ser Asp Leu Val Asn Glu Glu Ala Thr Gly Gln Phe Arg Val Tyr

1               5                   10                  15

<210> 132

<211> 15

<212> PRT

<213> Homo sapiens

<400> 132

Asn Glu Glu Ala Thr Gly Gln Phe Arg Val Tyr Pro Glu Leu Pro

1               5                   10                  15

<210> 133

<211> 15

<212> PRT

<213> Homo sapiens

<400> 133

Gln Phe Arg Val Tyr Pro Glu Leu Pro Lys Pro Ser Ile Ser Ser

1               5                   10                  15

<210> 134

<211> 15

<212> PRT

<213> Homo sapiens

<400> 134

Ala Val Ala Phe Thr Cys Glu Pro Glu Thr Gln Asp Ala Thr Tyr
1               5                   10                  15

<210> 135

<211> 15

<212> PRT

<213> Homo sapiens

<400> 135

Thr Ala Ser Tyr Lys Cys Glu Thr Gln Asn Pro Val Ser Ala Arg
1               5                   10                  15

<210> 136

<211> 15

<212> PRT

<213> Homo sapiens

<400> 136

Asn Val Leu Tyr Gly Pro Asp Ala Pro Thr Ile Ser Pro Leu Asn
1               5                   10                  15

<210> 137

<211> 15

<212> PRT

<213> Homo sapiens

<400> 137

Ile Ser Pro Leu Asn Thr Ser Tyr Arg Ser Gly Glu Asn Leu Asn
1               5                   10                  15

<210> 138

<211> 15

<212> PRT

<213> Homo sapiens

<400> 138

Ser Gly Ser Tyr Thr Cys Gln Ala His Asn Ser Asp Thr Gly Leu
1               5                   10                  15

<210> 139

<211> 15

<212> PRT

<213> Homo sapiens

<400> 139

Thr Ile Thr Val Tyr Ala Glu Pro Pro Lys Pro Phe Ile Thr Ser
1               5                   10                  15

<210> 140

<211> 15

<212> PRT

<213> Homo sapiens

<400> 140

Ser Asn Pro Val Glu Asp Glu Asp Ala Val Ala Leu Thr Cys Glu
1               5                   10                  15

<210> 141

<211> 15

<212> PRT

<213> Homo sapiens

<400> 141

Ala Val Ala Leu Thr Cys Glu Pro Glu Ile Gln Asn Thr Thr Tyr
1               5                   10                  15

<210> 142

<211> 15

<212> PRT

<213> Homo sapiens

<400> 142

Asn Gln Ser Leu Pro Val Ser Pro Arg Leu Gln Leu Ser Asn Asp
1               5                   10                  15

<210> 143

<211> 15

<212> PRT

<213> Homo sapiens

<400> 143

Arg Leu Gln Leu Ser Asn Asp Asn Arg Thr Leu Thr Leu Leu Ser
1               5                   10                  15

<210> 144

<211> 15

<212> PRT

<213> Homo sapiens

<400> 144

Glu Cys Gly Ile Gln Asn Glu Leu Ser Val Asp His Ser Asp Pro
1               5                   10                  15

<210> 145

<211> 15

<212> PRT

<213> Homo sapiens

<400> 145

Gln Asn Glu Leu Ser Val Asp His Ser Asp Pro Val Ile Leu Asn
1               5                   10                  15

<210> 146

<211> 15

<212> PRT

<213> Homo sapiens

<400> 146

Asn Val Leu Tyr Gly Pro Asp Asp Pro Thr Ile Ser Pro Ser Tyr
1               5                   10                  15

<210> 147

<211> 15

<212> PRT

<213> Homo sapiens

<400> 147

Gly Val Asn Leu Ser Leu Ser Cys His Ala Ala Ser Asn Pro Pro
1               5                   10                  15

<210> 148

<211> 15

<212> PRT

<213> Homo sapiens

<400> 148

Thr Ile Thr Val Ser Ala Glu Leu Pro Lys Pro Ser Ile Ser Ser
1               5                   10                  15

<210> 149

<211> 15

<212> PRT

<213> Homo sapiens

<400> 149

Ala Val Ala Phe Thr Cys Glu Pro Glu Ala Gln Asn Thr Thr Tyr
1               5                   10                  15

<210> 150

<211> 15

<212> PRT

<213> Homo sapiens

<400> 150

Ser Asp Pro Val Thr Leu Asp Val Leu Tyr Gly Pro Asp Thr Pro

1               5                   10                  15

<210> 151

<211> 14

<212> PRT

<213> Homo sapiens

<400> 151

Asp Val Leu Tyr Gly Pro Asp Thr Pro His Ser Pro Pro Asp

1               5                   10

<210> 152

<211> 15

<212> PRT

<213> Homo sapiens

<400> 152

Gly Ala Asn Leu Asn Leu Ser Cys His Ser Ala Ser Asn Pro Ser

1               5                   10                  15

<210> 153

<211> 15

<212> PRT

<213> Homo sapiens

<400> 153

Arg Leu Pro Ala Ser Pro Glu Thr His Leu Asp Met Leu Arg His

1               5                   10                  15

<210> 154

<211> 15

<212> PRT

<213> Homo sapiens

<400> 154

Ser Leu Ser Phe Leu Gln Asp Ile Gln Glu Val Gln Gly Tyr Val
1               5                   10                  15

<210> 155

<211> 15

<212> PRT

<213> Homo sapiens

<400> 155

Val Leu Ile Ala His Asn Gln Val Arg Gln Val Pro Leu Gln Arg
1               5                   10                  15

<210> 156

<211> 15

<212> PRT

<213> Homo sapiens

<400> 156

Gly Thr Gln Leu Phe Glu Asp Asn Tyr Ala Leu Ala Val Leu Asp
1               5                   10                  15

<210> 157

<211> 15

<212> PRT

<213> Homo sapiens

<400> 157

Asp Thr Ile Leu Trp Lys Asp Ile Phe His Lys Asn Asn Gln Leu
1               5                   10                  15

<210> 158

<211> 15

<212> PRT

<213> Homo sapiens

<400> 158

Ala Leu Thr Leu Ile Asp Thr Asn Arg Ser Arg Ala Cys His Pro
1               5                   10                  15

<210> 159

<211> 15

<212> PRT

<213> Homo sapiens

<400> 159

Lys Gly Pro Leu Pro Thr Asp Cys Cys His Glu Gln Cys Ala Ala
1               5                   10                  15

<210> 160

<211> 15

<212> PRT

<213> Homo sapiens

<400> 160

Leu Val Thr Tyr Asn Thr Asp Thr Phe Glu Ser Met Pro Asn Pro
1               5                   10                  15

<210> 161

<211> 15

<212> PRT

<213> Homo sapiens

<400> 161

Tyr Asn Tyr Leu Ser Thr Asp Val Gly Ser Cys Thr Leu Val Cys
1               5                   10                  15

<210> 162

<211> 15

<212> PRT

<213> Homo sapiens

<400> 162

Asn Gln Glu Val Thr Ala Glu Asp Gly Thr Gln Arg Cys Glu Lys
1               5                   10                  15

<210> 163

<211> 15

<212> PRT

<213> Homo sapiens

<400> 163

Cys Tyr Gly Leu Gly Met Glu His Leu Arg Glu Val Arg Ala Val
1               5                   10                  15

<210> 164

<211> 15

<212> PRT

<213> Homo sapiens

<400> 164

Ser Leu Ala Phe Leu Pro Glu Ser Phe Asp Gly Asp Pro Ala Ser
1               5                   10                  15

<210> 165

<211> 15

<212> PRT

<213> Homo sapiens

<400> 165

Pro Glu Ser Phe Asp Gly Asp Pro Ala Ser Asn Thr Ala Pro Leu
1               5                   10                  15

<210> 166

<211> 15

<212> PRT

<213> Homo sapiens

<400> 166

Thr Ala Pro Leu Gln Pro Glu Gln Leu Gln Val Phe Glu Thr Leu
1               5                   10                  15

<210> 167

<211> 15

<212> PRT

<213> Homo sapiens

<400> 167

Leu Ala Leu Ile His His Asn Thr His Leu Cys Phe Val His Thr
1               5                   10                  15

<210> 168

<211> 15

<212> PRT

<213> Homo sapiens

<400> 168

Val His Thr Val Pro Trp Asp Gln Leu Phe Arg Asn Pro His Gln
1               5                   10                  15

<210> 169

<211> 15

<212> PRT

<213> Homo sapiens

<400> 169

Trp Asp Gln Leu Phe Arg Asn Pro His Gln Ala Leu Leu His Thr
1               5                   10                  15

<210> 170

<211> 15

<212> PRT

<213> Homo sapiens

<400> 170

Leu Gln Gly Leu Pro Arg Glu Tyr Val Asn Ala Arg His Cys Leu
1               5                   10                  15

<210> 171

<211> 15

<212> PRT

<213> Homo sapiens

<400> 171

Val Thr Cys Phe Gly Pro Glu Ala Asp Gln Cys Val Ala Cys Ala
1               5                   10                  15

<210> 172

<211> 15

<212> PRT

<213> Homo sapiens

<400> 172

Pro Ser Gly Val Lys Pro Asp Leu Ser Tyr Met Pro Ile Trp Lys
1               5                   10                  15

<210> 173

<211> 15

<212> PRT

<213> Homo sapiens

<400> 173

Ile Trp Lys Phe Pro Asp Glu Glu Gly Ala Cys Gln Pro Cys Pro
1               5                   10                  15

<210> 174

<211> 15

<212> PRT

<213> Homo sapiens

<400> 174

His Ser Cys Val Asp Leu Asp Asp Lys Gly Cys Pro Ala Glu Gln
1               5                   10                  15

<210> 175

<211> 15

<212> PRT

<213> Homo sapiens

<400> 175

Met Arg Arg Leu Leu Gln Glu Thr Glu Leu Val Glu Pro Leu Thr
1               5                   10                  15

<210> 176

<211> 14

<212> PRT

<213> Homo sapiens

<400> 176

Gln Met Arg Ile Lys Glu Thr Glu Leu Arg Lys Val Lys Val
1               5                   10

<210> 177

<211> 15

<212> PRT

<213> Homo sapiens

<400> 177

Ala Ile Lys Val Leu Arg Glu Asn Thr Ser Pro Lys Ala Asn Lys
1               5                   10                  15

<210> 178

<211> 15

<212> PRT

<213> Homo sapiens

<400> 178

Asn Lys Glu Ile Leu Asp Glu Ala Tyr Val Met Ala Gly Val Gly
1               5                   10                  15

<210> 179

<211> 15

<212> PRT

<213> Homo sapiens

<400> 179

Gly Met Ser Tyr Leu Glu Asp Val Arg Leu Val His Arg Asp Leu
1               5                   10                  15

<210> 180

<211> 15

<212> PRT

<213> Homo sapiens

<400> 180

Val Arg Leu Val His Arg Asp Leu Ala Ala Arg Asn Val Leu Val
1               5                   10                  15

<210> 181

<211> 15

<212> PRT

<213> Homo sapiens

<400> 181

Ala Arg Leu Leu Asp Ile Asp Glu Thr Glu Tyr His Ala Asp Gly
1               5                   10                  15

<210> 182
<211> 15
<212> PRT
<213> Homo sapiens

<400> 182
Glu Thr Glu Tyr His Ala Asp Gly Gly Lys Val Pro Ile Lys Trp
1               5                   10                  15

<210> 183
<211> 15
<212> PRT
<213> Homo sapiens

<400> 183
Ile Lys Trp Met Ala Leu Glu Ser Ile Leu Arg Arg Arg Phe Thr
1               5                   10                  15

<210> 184
<211> 15
<212> PRT
<213> Homo sapiens

<400> 184
Cys Trp Met Ile Asp Ser Glu Cys Arg Pro Arg Phe Arg Glu Leu
1               5                   10                  15

<210> 185
<211> 15
<212> PRT
<213> Homo sapiens

<400> 185
Phe Arg Glu Leu Val Ser Glu Phe Ser Arg Met Ala Arg Asp Pro
1               5                   10                  15

<210> 186
<211> 15
<212> PRT
<213> Homo sapiens

<400> 186
Phe Ser Arg Met Ala Arg Asp Pro Gln Arg Phe Val Val Ile Gln
1               5                   10                  15

<210> 187
<211> 15
<212> PRT
<213> Homo sapiens

<400> 187
Phe Val Val Ile Gln Asn Glu Asp Leu Gly Pro Ala Ser Pro Leu
1               5                   10                  15

<210> 188
<211> 15
<212> PRT
<213> Homo sapiens

<400> 188
Tyr Arg Ser Leu Leu Glu Asp Asp Asp Met Gly Asp Leu Val Asp
1               5                   10                  15

<210> 189
<211> 15
<212> PRT
<213> Homo sapiens

<400> 189
Arg Ser Leu Leu Glu Asp Asp Asp Met Gly Asp Leu Val Asp Ala
1               5                   10                  15

<210> 190

<211> 15

<212> PRT

<213> Homo sapiens

<400> 190

Gly Asp Leu Val Asp Ala Glu Glu Tyr Leu Val Pro Gln Gln Gly
1               5                   10                  15

<210> 191

<211> 15

<212> PRT

<213> Homo sapiens

<400> 191

Gln Gly Phe Phe Cys Pro Asp Pro Ala Pro Gly Ala Gly Gly Met
1               5                   10                  15

<210> 192

<211> 15

<212> PRT

<213> Homo sapiens

<400> 192

Asp Leu Thr Leu Gly Leu Glu Pro Ser Glu Glu Glu Ala Pro Arg
1               5                   10                  15

<210> 193

<211> 15

<212> PRT

<213> Homo sapiens

<400> 193

Ser Asp Val Phe Asp Gly Asp Leu Gly Met Gly Ala Ala Lys Gly
1               5                   10                  15

<210> 194

<211> 15

<212> PRT

<213> Homo sapiens

<400> 194

Leu Gln Arg Tyr Ser Glu Asp Pro Thr Val Pro Leu Pro Ser Glu
1               5                   10                  15

<210> 195

<211> 15

<212> PRT

<213> Homo sapiens

<400> 195

Thr Val Pro Leu Pro Ser Glu Thr Asp Gly Tyr Val Ala Pro Leu
1               5                   10                  15

<210> 196

<211> 15

<212> PRT

<213> Homo sapiens

<400> 196

Lys Asn Gly Val Val Lys Asp Val Phe Ala Phe Gly Gly Ala Val
1               5                   10                  15

<210> 197

<211> 15

<212> PRT

<213> Homo sapiens

<400> 197

Gln Gly Gly Ala Ala Pro Gln Pro His Pro Pro Pro Ala Phe Ser
1               5                   10                  15

<210> 198
<211> 15
<212> PRT
<213> Homo sapiens

<400> 198
Asp Asn Leu Tyr Tyr Trp Asp Gln Asp Pro Pro Glu Arg Gly Ala
1               5                   10                  15

<210> 199
<211> 15
<212> PRT
<213> Homo sapiens

<400> 199
Gly Pro Arg Met Phe Pro Asp Leu Glu Ser Glu Phe Gln Ala Ala
1               5                   10                  15

<210> 200
<211> 15
<212> PRT
<213> Homo sapiens

<400> 200
Phe Pro Asp Leu Glu Ser Glu Phe Gln Ala Ala Ile Ser Arg Lys
1               5                   10                  15

<210> 201
<211> 15
<212> PRT
<213> Homo sapiens

<400> 201
Glu Phe Gln Ala Ala Ile Ser Arg Lys Met Val Glu Leu Val His
1               5                   10                  15

<210> 202

<211> 15

<212> PRT

<213> Homo sapiens

<400> 202

Gln Leu Val Phe Gly Ile Glu Val Val Glu Val Val Pro Ile Ser
1               5                   10                  15

<210> 203

<211> 15

<212> PRT

<213> Homo sapiens

<400> 203

Cys Leu Gly Leu Ser Tyr Asp Gly Leu Leu Gly Asp Asn Gln Val
1               5                   10                  15

<210> 204

<211> 15

<212> PRT

<213> Homo sapiens

<400> 204

Tyr Asp Gly Leu Leu Gly Asp Asn Gln Val Met Pro Lys Thr Gly
1               5                   10                  15

<210> 205

<211> 15

<212> PRT

<213> Homo sapiens

<400> 205

Leu Ala Ile Ile Ala Ile Glu Gly Asp Cys Ala Pro Glu Glu Lys
1               5                   10                  15

<210> 206

<211> 15

<212> PRT

<213> Homo sapiens

<400> 206

Ile Ile Ala Ile Glu Gly Asp Cys Ala Pro Glu Glu Lys Ile Trp
1               5                   10                  15

<210> 207

<211> 15

<212> PRT

<213> Homo sapiens

<400> 207

Glu Glu Lys Ile Trp Glu Glu Leu Ser Met Leu Glu Val Phe Glu
1               5                   10                  15

<210> 208

<211> 15

<212> PRT

<213> Homo sapiens

<400> 208

Arg Lys Leu Leu Met Gln Asp Leu Val Gln Glu Asn Tyr Leu Glu
1               5                   10                  15

<210> 209

<211> 15

<212> PRT

<213> Homo sapiens

<400> 209

Met Gln Asp Leu Val Gln Glu Asn Tyr Leu Glu Tyr Arg Gln Val
1               5                   10                  15

<210> 210
<211> 15
<212> PRT
<213> Homo sapiens

<400> 210
Val Lys Val Leu His His Thr Leu Lys Ile Gly Gly Glu Pro His
1               5                   10                  15

<210> 211
<211> 15
<212> PRT
<213> Homo sapiens

<400> 211
Thr Leu Lys Ile Gly Gly Glu Pro His Ile Ser Tyr Pro Pro Leu
1               5                   10                  15

<210> 212
<211> 15
<212> PRT
<213> Homo sapiens

<400> 212
Phe Pro Asp Leu Glu Ser Glu Phe Gln Ala Ala Leu Ser Arg Lys
1               5                   10                  15

<210> 213
<211> 15
<212> PRT
<213> Homo sapiens

<400> 213
Glu Phe Gln Ala Ala Leu Ser Arg Lys Val Ala Glu Leu Val His
1               5                   10                  15

<210> 214

<211> 15

<212> PRT

<213> Homo sapiens

<400> 214

Gln Leu Val Phe Gly Ile Glu Leu Met Glu Val Asp Pro Ile Gly
1               5                   10                  15

<210> 215

<211> 15

<212> PRT

<213> Homo sapiens

<400> 215

Ile Glu Leu Met Glu Val Asp Pro Ile Gly His Leu Tyr Ile Phe
1               5                   10                  15

<210> 216

<211> 15

<212> PRT

<213> Homo sapiens

<400> 216

Cys Leu Gly Leu Ser Tyr Asp Gly Leu Leu Gly Asp Asn Gln Ile
1               5                   10                  15

<210> 217

<211> 14

<212> PRT

<213> Homo sapiens

<400> 217

Tyr Asp Gly Leu Leu Gly Asp Asn Ile Met Pro Lys Ala Gly
1               5                   10

<210> 218

<211> 15

<212> PRT

<213> Homo sapiens

<400> 218

Leu Ala Ile Ile Ala Arg Glu Gly Asp Cys Ala Pro Glu Glu Lys
1               5                   10                  15

<210> 219

<211> 15

<212> PRT

<213> Homo sapiens

<400> 219

Glu Glu Lys Ile Trp Glu Glu Leu Ser Val Leu Glu Val Phe Glu
1               5                   10                  15

<210> 220

<211> 15

<212> PRT

<213> Homo sapiens

<400> 220

Glu Asp Ser Ile Leu Gly Asp Pro Lys Lys Leu Leu Thr Gln His
1               5                   10                  15

<210> 221

<211> 15

<212> PRT

<213> Homo sapiens

<400> 221

Thr Gln His Phe Val Gln Glu Asn Tyr Leu Glu Tyr Arg Gln Val
1               5                   10                  15

<210> 222

<211> 15

<212> PRT

<213> Homo sapiens

<400> 222

Glu Pro Pro Leu Ser Gln Glu Thr Phe Ser Asp Leu Trp Lys Leu
1               5                   10                  15

<210> 223

<211> 15

<212> PRT

<213> Homo sapiens

<400> 223

Leu Trp Lys Leu Leu Pro Glu Asn Asn Val Leu Ser Pro Leu Pro
1               5                   10                  15

<210> 224

<211> 15

<212> PRT

<213> Homo sapiens

<400> 224

Asp Leu Met Leu Ser Pro Asp Asp Ile Glu Gln Trp Phe Thr Glu
1               5                   10                  15

<210> 225

<211> 15

<212> PRT

<213> Homo sapiens

<400> 225

Glu Gln Trp Phe Thr Glu Asp Pro Gly Pro Asp Glu Ala Pro Arg
1               5                   10                  15

<210> 226

<211> 15

<212> PRT

<213> Homo sapiens

<400> 226

Pro Val Gln Leu Trp Val Asp Ser Thr Pro Pro Pro Gly Thr Arg
1               5                   10                  15

<210> 227

<211> 15

<212> PRT

<213> Homo sapiens

<400> 227

Met Ala Ile Tyr Lys Gln Ser Gln His Met Thr Glu Val Val Arg
1               5                   10                  15

<210> 228

<211> 15

<212> PRT

<213> Homo sapiens

<400> 228

Gln His Leu Ile Arg Val Glu Gly Asn Leu Arg Val Glu Tyr Leu
1               5                   10                  15

<210> 229

<211> 15

<212> PRT

<213> Homo sapiens

<400> 229

Leu Ile Arg Val Glu Gly Asn Leu Arg Val Glu Tyr Leu Asp Asp
1               5                   10                  15

<210> 230

<211> 15

<212> PRT

<213> Homo sapiens

<400> 230

Glu Gly Asn Leu Arg Val Glu Tyr Leu Asp Asp Arg Asn Thr Phe
1               5                   10                  15

<210> 231

<211> 15

<212> PRT

<213> Homo sapiens

<400> 231

Arg Val Glu Tyr Leu Asp Asp Arg Asn Thr Phe Arg His Ser Val
1               5                   10                  15

<210> 232

<211> 15

<212> PRT

<213> Homo sapiens

<400> 232

Ser Val Val Val Pro Tyr Glu Pro Pro Glu Val Gly Ser Asp Cys
1               5                   10                  15

<210> 233

<211> 15

<212> PRT

<213> Homo sapiens

<400> 233

Pro Pro Glu Val Gly Ser Asp Cys Thr Thr Ile His Tyr Asn Tyr
1               5                   10                  15

<210> 234

<211> 15

<212> PRT

<213> Homo sapiens

<400> 234

Leu Thr Ile Ile Thr Leu Glu Asp Ser Ser Gly Asn Leu Leu Gly
1               5                   10                  15

<210> 235

<211> 15

<212> PRT

<213> Homo sapiens

<400> 235

Lys Lys Pro Leu Asp Gly Glu Tyr Phe Thr Leu Gln Ile Arg Gly
1               5                   10                  15

<210> 236

<211> 15

<212> PRT

<213> Homo sapiens

<400> 236

Gly Glu Tyr Phe Thr Leu Gln Ile Arg Gly Arg Glu Arg Phe Glu
1               5                   10                  15

<210> 237

<211> 15

<212> PRT

<213> Homo sapiens

<400> 237

Arg Phe Glu Met Phe Arg Glu Leu Asn Glu Ala Leu Glu Leu Lys
1               5                   10                  15

<210> 238

<211> 20

<212> PRT

<213> Homo sapiens

<400> 238

Gly Arg Thr Gln Asp Glu Asn Pro Val Val His Phe Phe Lys Asn Ile
1               5                   10                  15

Val Thr Pro Arg
            20

<210> 239

<211> 17

<212> PRT

<213> Homo sapiens

<400> 239

Lys Tyr Leu Glu Phe Ile Ser Glu Ala Ile Ile His Val Leu His Ser
1               5                   10                  15

Arg

<210> 240

<211> 13

<212> PRT

<213> Homo sapiens

<400> 240

Pro Lys Tyr Val Lys Gln Asn Thr Leu Lys Leu Ala Thr
1               5                   10

<210> 241

<211> 13

<212> PRT

<213> Homo sapiens

<400> 241

His Lys Tyr Val Lys Gln Asn Thr Leu Lys Leu Ala Thr
1               5                   10

<210> 242

<211> 13

<212> PRT

<213> Homo sapiens

<400> 242

Pro His Tyr Val Lys Gln Asn Thr Leu Lys Leu Ala Thr
1               5                   10

<210> 243

<211> 13

<212> PRT

<213> Homo sapiens

<400> 243

Pro Lys Tyr His Lys Gln Asn Thr Leu Lys Leu Ala Thr
1               5                   10

<210> 244

<211> 13

<212> PRT

<213> Homo sapiens

<400> 244

Pro Lys Tyr Val His Gln Asn Thr Leu Lys Leu Ala Thr
1               5                   10

<210> 245

<211> 13

<212> PRT

<213> Homo sapiens

<400> 245

Pro Lys Tyr Val Lys His Asn Thr Leu Lys Leu Ala Thr
 1               5                   10

<210> 246

<211> 13

<212> PRT

<213> Homo sapiens

<400> 246

Pro Lys Tyr Val Lys Gln His Thr Leu Lys Leu Ala Thr
 1               5                   10

<210> 247

<211> 13

<212> PRT

<213> Homo sapiens

<400> 247

Pro Lys Tyr Val Lys Gln Asn Thr His Lys Leu Ala Thr
 1               5                   10

<210> 248

<211> 13

<212> PRT

<213> Homo sapiens

<400> 248

Pro Lys Tyr Val Lys Gln Asn Thr Leu His Leu Ala Thr
 1               5                   10

<210> 249

<211> 13

<212> PRT

<213> Homo sapiens

<400> 249

Pro Lys Tyr Val Lys Gln Asn Thr Leu Lys His Ala Thr
1               5                   10

<210> 250

<211> 13

<212> PRT

<213> Homo sapiens

<400> 250

Pro Lys Tyr Val Lys Gln Asn Thr Leu Lys Leu His Thr
1               5                   10

<210> 251

<211> 13

<212> PRT

<213> Homo sapiens

<400> 251

Pro Lys Tyr Val Lys Gln Asn Thr Leu Lys Leu Ala His
1               5                   10

<210> 252

<211> 15

<212> PRT

<213> Homo sapiens

<400> 252

Asp Val Phe Lys Glu Leu Lys Val His His Ala Asn Glu Asn Ile
1               5                   10                  15

<210> 253
<211> 17
<212> PRT
<213> Homo sapiens

<400> 253
Lys Ile Ala Lys Met Glu Lys Ala Ser Ser Val Phe Asn Val Val Asn
1               5                   10                  15
Ser

<210> 254
<211> 19
<212> PRT
<213> Homo sapiens

<400> 254
Asp Ile Glu Lys Lys Ile Ala Lys Met Glu Lys Ala Ser Ser Val Phe
1               5                   10                  15

Asn Val Val

<210> 255
<211> 16
<212> PRT
<213> Homo sapiens

<400> 255
Lys Met Tyr Phe Asn Leu Ile Asn Thr Lys Cys Tyr Lys Leu Glu His
1               5                   10                  15

<210> 256
<211> 14
<212> PRT
<213> Homo sapiens

<400> 256
Gln Tyr Ile Lys Ala Asn Ser Lys Phe Ile Gly Ile Thr Glu
1               5                   10

<210> 257
<211> 15
<212> PRT
<213> Homo sapiens

<400> 257
Gln Tyr Ile Lys Ala Asn Ser Lys Phe Ile Gly Ile Thr Glu Ala
1               5                   10                  15

<210> 258
<211> 17
<212> PRT
<213> Homo sapiens

<400> 258
Lys Ser Ser Gln Tyr Ile Lys Ala Asn Ser Ser Phe Ile Gly Ile Thr
1               5                   10                  15
Glu

<210> 259
<211> 17
<212> PRT
<213> Homo sapiens

<400> 259
Lys Ser Ser Gln Tyr Ile Lys Ala Asn Ser Ala Phe Ile Gly Ile Thr
1               5                   10                  15
Glu

<210> 260

<211> 17

<212> PRT

<213> Homo sapiens

<400> 260

Lys Ser Ser Gln Tyr Ile Lys Ala Asn Ser Leu Phe Ile Gly Ile Thr
1               5                   10                  15

Glu

<210> 261

<211> 17

<212> PRT

<213> Homo sapiens

<400> 261

Lys Ser Ser Gln Tyr Ile Lys Ala Asn Ser Ser Phe Ile Gly Lys Thr
1               5                   10                  15

Glu

<210> 262

<211> 18

<212> PRT

<213> Homo sapiens

<400> 262

Lys Ser Ser Ala Gln Tyr Ile Lys Ala Asn Ser Ser Phe Ile Gly Ile
1               5                   10                  15

Thr Glu

<210> 263
<211> 17
<212> PRT
<213> Homo sapiens

<220>
<221> SITE
<222> (5)
<223> Xaa is uncertain

<400> 263
Lys Ser Ser Gln Xaa Ile Lys Ala Asn Ser Ser Phe Ile Gly Ile Thr
 1               5                   10                  15
Glu

<210> 264
<211> 18
<212> PRT
<213> Homo sapiens

<220>
<221> SITE
<222> (6)
<223> Xaa is uncertain

<400> 264
Lys Ser Ser Ala Gln Xaa Ile Lys Ala Asn Ser Ser Phe Ile Gly Ile
 1               5                   10                  15
Thr Glu

<210> 265
<211> 18
<212> PRT
<213> Homo sapiens

<220>

<221> SITE

<222> (6)

<223> Xaa is uncertain

<400> 265

Lys Ser Ser Ala Gln Xaa Ile Lys Ala Asn Ser Ser Phe Ile Gly Lys
 1               5                   10                  15

Thr Glu

<210> 266

<211> 18

<212> PRT

<213> Homo sapiens

<220>

<221> SITE

<222> (6)

<223> Xaa is uncertain

<400> 266

Lys Ser Ser Ala Gln Xaa Val Lys Ala Asn Ser Ser Phe Ile Gly Lys
 1               5                   10                  15

Thr Glu

<210> 267

<211> 18

<212> PRT

<213> Homo sapiens

<220>

<221> SITE

<222> (6)

<223> Xaa is uncertain

<400> 267

Lys Ser Ser Ala Gln Xaa Val Lys Ala Asn Ser Ser Phe Ile Gly Ile
1               5                   10                  15

Thr Glu

<210> 268

<211> 14

<212> PRT

<213> Homo sapiens

<400> 268

Tyr Pro Lys Tyr Val Lys Gln Asn Thr Leu Lys Leu Ala Thr
1               5                   10

<210> 269

<211> 18

<212> PRT

<213> Homo sapiens

<400> 269

Tyr Val Lys Gln Ala Tyr Val Lys Gln Ala Tyr Val Lys Gln Ala Tyr
1               5                   10                  15

Val Lys

<210> 270

<211> 18

<212> PRT

<213> Homo sapiens

<400> 270

Tyr Val Lys Gln Asn Tyr Val Lys Gln Asn Tyr Val Lys Gln Asn Tyr
1               5                   10                  15

Val Lys

<210> 271

<211> 12

<212> PRT

<213> Homo sapiens

<400> 271

Tyr Tyr Val Arg Ala Val Arg Ala Tyr Leu Thr Thr
1               5                   10

<210> 272

<211> 16

<212> PRT

<213> Homo sapiens

<400> 272

Ala Val Leu Glu Asp Pro Tyr Ile Leu Leu Val Ser Ser Lys Val Ser
1               5                   10                  15

<210> 273

<211> 16

<212> PRT

<213> Homo sapiens

<400> 273

Tyr Glu His Arg Val Lys Arg Gly Leu Thr Val Ala Val Ala Gly Ala
1               5                   10                  15

<210> 274

<211> 16

<212> PRT

<213> Homo sapiens

<400> 274

Pro Lys Tyr Val Lys Gln Asn Thr Leu Lys Leu Ala Thr Gly Met Arg
1               5                   10                  15

<210> 275

<211> 14

<212> PRT

<213> Homo sapiens

<400> 275

Tyr Val Lys Gln Asn Thr Leu Lys Leu Ala Thr Gly Met Arg
1               5                   10

<210> 276

<211> 20

<212> PRT

<213> Homo sapiens

<400> 276

Ile Leu Met Gln Tyr Ile Lys Ala Asn Ser Lys Phe Ile Gly Ile Thr
1               5                   10                  15

Glu Leu Lys Lys
            20

<210> 277

<211> 17

<212> PRT

<213> Homo sapiens

<400> 277

Gln Tyr Ile Lys Ala Asn Ser Lys Phe Ile Gly Ile Thr Glu Leu Lys
1               5                   10                  15

Lys

<210> 278

<211> 17

<212> PRT

<213> Homo sapiens

<400> 278

Ile Leu Met Gln Tyr Ile Lys Ala Asn Ser Lys Phe Ile Gly Ile Thr

1               5                   10                  15

Glu

<210> 279

<211> 15

<212> PRT

<213> Homo sapiens

<400> 279

Ile Leu Met Gln Tyr Ile Lys Ala Asn Ser Lys Phe Ile Gly Ile

1               5                   10                  15

<210> 280

<211> 13

<212> PRT

<213> Homo sapiens

<400> 280

Ile Leu Met Gln Tyr Ile Lys Ala Asn Ser Lys Phe Ile

1               5                   10

<210> 281

<211> 11

<212> PRT

<213> Homo sapiens

<400> 281

Ile Leu Met Gln Tyr Ile Lys Ala Asn Ser Lys

1               5                   10

<210> 282

<211> 19

<212> PRT

<213> Homo sapiens

<400> 282

Phe Asn Asn Phe Thr Val Ser Phe Trp Leu Arg Val Pro Lys Val Ser
1               5                   10                  15

Ala Ser His

<210> 283

<211> 14

<212> PRT

<213> Homo sapiens

<400> 283

Tyr Pro Lys Phe Val Lys Gln Asn Thr Leu Lys Leu Ala Thr
1               5                   10

<210> 284

<211> 18

<212> PRT

<213> Homo sapiens

<400> 284

Gly Ala Cys Pro Lys Tyr Val Lys Gln Asn Thr Leu Lys Leu Ala Thr
1               5                   10                  15

Gly Met

<210> 285

<211> 16

<212> PRT

<213> Homo sapiens

<400> 285

Gly Ala Cys Pro Lys Tyr Val Lys Gln Asn Thr Leu Lys Leu Ala Thr

1               5                   10                  15

<210> 286

<211> 15

<212> PRT

<213> Homo sapiens

<400> 286

Gly Ala Cys Pro Lys Tyr Val Lys Gln Asn Thr Leu Lys Leu Ala

1               5                   10                  15

<210> 287

<211> 15

<212> PRT

<213> Homo sapiens

<400> 287

Lys Tyr Val Lys Gln Asn Thr Leu Lys Leu Ala Thr Gly Met Arg

1               5                   10                  15

<210> 288

<211> 13

<212> PRT

<213> Homo sapiens

<400> 288

Tyr Ile Lys Ala Asn Ser Lys Phe Ile Gly Ile Thr Glu

1               5                   10

<210> 289

<211> 14

<212> PRT

<213> Homo sapiens

<400> 289

Ile Leu Met Gln Tyr Ile Lys Ala Asn Ser Lys Phe Ile Gly

1               5                   10

<210> 290

<211> 12

<212> PRT

<213> Homo sapiens

<400> 290

Ile Leu Met Gln Tyr Ile Lys Ala Asn Ser Lys Phe

1               5                   10

<210> 291

<211> 13

<212> PRT

<213> Homo sapiens

<400> 291

Pro Lys Tyr Val Lys Gln Asn Thr Leu Lys Leu Ala Thr

1               5                   10

<210> 292

<211> 13

<212> PRT

<213> Homo sapiens

<400> 292

Ala Lys Tyr Val Lys Gln Asn Thr Leu Lys Leu Ala Thr

1               5                   10

<210> 293
<211> 13
<212> PRT
<213> Homo sapiens

<400> 293
Lys Lys Tyr Val Lys Gln Asn Thr Leu Lys Leu Ala Thr
1               5                   10

<210> 294
<211> 13
<212> PRT
<213> Homo sapiens

<400> 294
Glu Lys Tyr Val Lys Gln Asn Thr Leu Lys Leu Ala Thr
1               5                   10

<210> 295
<211> 13
<212> PRT
<213> Homo sapiens

<400> 295
Pro Arg Tyr Val Lys Gln Asn Thr Leu Lys Leu Ala Thr
1               5                   10

<210> 296
<211> 13
<212> PRT
<213> Homo sapiens

<400> 296
Pro Glu Tyr Val Lys Gln Asn Thr Leu Lys Leu Ala Thr
1               5                   10

<210> 297

<211> 13

<212> PRT

<213> Homo sapiens

<400> 297

Pro Ser Tyr Val Lys Gln Asn Thr Leu Lys Leu Ala Thr
1               5                   10

<210> 298

<211> 13

<212> PRT

<213> Homo sapiens

<400> 298

Pro Lys Phe Val Lys Gln Asn Thr Leu Lys Leu Ala Thr
1               5                   10

<210> 299

<211> 13

<212> PRT

<213> Homo sapiens

<400> 299

Pro Lys Tyr Leu Lys Gln Asn Thr Leu Lys Leu Ala Thr
1               5                   10

<210> 300

<211> 13

<212> PRT

<213> Homo sapiens

<400> 300

Pro Lys Tyr Lys Lys Gln Asn Thr Leu Lys Leu Ala Thr
1               5                   10

<210> 301

<211> 13

<212> PRT

<213> Homo sapiens

<400> 301

Pro Lys Tyr Gln Lys Gln Asn Thr Leu Lys Leu Ala Thr
1               5                   10

<210> 302

<211> 13

<212> PRT

<213> Homo sapiens

<400> 302

Pro Lys Tyr Val Arg Gln Asn Thr Leu Lys Leu Ala Thr
1               5                   10

<210> 303

<211> 13

<212> PRT

<213> Homo sapiens

<400> 303

Pro Lys Tyr Val Glu Gln Asn Thr Leu Lys Leu Ala Thr
1               5                   10

<210> 304

<211> 13

<212> PRT

<213> Homo sapiens

<400> 304

Pro Lys Tyr Val Ser Gln Asn Thr Leu Lys Leu Ala Thr
1               5                   10

<210> 305

<211> 13

<212> PRT

<213> Homo sapiens

<400> 305

Pro Lys Tyr Val Lys Asn Asn Thr Leu Lys Leu Ala Thr
1               5                   10

<210> 306

<211> 13

<212> PRT

<213> Homo sapiens

<400> 306

Pro Lys Tyr Val Lys Leu Asn Thr Leu Lys Leu Ala Thr
1               5                   10

<210> 307

<211> 13

<212> PRT

<213> Homo sapiens

<400> 307

Pro Lys Tyr Val Lys Thr Asn Thr Leu Lys Leu Ala Thr
1               5                   10

<210> 308

<211> 13

<212> PRT

<213> Homo sapiens

<400> 308

Pro Lys Tyr Val Lys Gln Gln Thr Leu Lys Leu Ala Thr
1               5                   10

<210> 309

<211> 13

<212> PRT

<213> Homo sapiens

<400> 309

Pro Lys Tyr Val Lys Gln Thr Thr Leu Lys Leu Ala Thr
1               5                   10

<210> 310

<211> 13

<212> PRT

<213> Homo sapiens

<400> 310

Pro Lys Tyr Val Lys Gln Leu Thr Leu Lys Leu Ala Thr
1               5                   10

<210> 311

<211> 13

<212> PRT

<213> Homo sapiens

<400> 311

Pro Lys Tyr Val Lys Gln Asn Ser Leu Lys Leu Ala Thr
1               5                   10

<210> 312

<211> 13

<212> PRT

<213> Homo sapiens

<400> 312

Pro Lys Tyr Val Lys Gln Asn Val Leu Lys Leu Ala Thr
1               5                   10

<210> 313
<211> 13
<212> PRT
<213> Homo sapiens

<400> 313
Pro Lys Tyr Val Lys Gln Asn Thr Ile Lys Leu Ala Thr
1               5                   10

<210> 314
<211> 13
<212> PRT
<213> Homo sapiens

<400> 314
Pro Lys Tyr Val Lys Gln Asn Thr Gln Lys Leu Ala Thr
1               5                   10

<210> 315
<211> 13
<212> PRT
<213> Homo sapiens

<400> 315
Pro Lys Tyr Val Lys Gln Asn Thr Leu Arg Leu Ala Thr
1               5                   10

<210> 316
<211> 13
<212> PRT
<213> Homo sapiens

<400> 316
Pro Lys Tyr Val Lys Gln Asn Thr Leu Glu Leu Ala Thr
1               5                   10

<210> 317
<211> 13
<212> PRT
<213> Homo sapiens

<400> 317
Pro Lys Tyr Val Lys Gln Asn Thr Leu Ser Leu Ala Thr
1               5                   10

<210> 318
<211> 13
<212> PRT
<213> Homo sapiens

<400> 318
Pro Lys Tyr Val Lys Gln Asn Thr Leu Lys Ile Ala Thr
1               5                   10

<210> 319
<211> 13
<212> PRT
<213> Homo sapiens

<400> 319
Pro Lys Tyr Val Lys Gln Asn Thr Leu Lys Lys Ala Thr
1               5                   10

<210> 320
<211> 13
<212> PRT
<213> Homo sapiens

<400> 320
Pro Lys Tyr Val Lys Gln Asn Thr Leu Lys Gln Ala Thr
1               5                   10

<210> 321
<211> 13
<212> PRT
<213> Homo sapiens

<400> 321
Pro Lys Tyr Val Lys Gln Asn Thr Leu Lys Leu Ser Thr
1               5                   10

<210> 322
<211> 13
<212> PRT
<213> Homo sapiens

<400> 322
Pro Lys Tyr Val Lys Gln Asn Thr Leu Lys Leu Phe Thr
1               5                   10

<210> 323
<211> 13
<212> PRT
<213> Homo sapiens

<400> 323
Pro Lys Tyr Val Lys Gln Asn Thr Leu Lys Leu Lys Thr
1               5                   10

<210> 324
<211> 13
<212> PRT
<213> Homo sapiens

<400> 324
Pro Lys Tyr Val Lys Gln Asn Thr Leu Lys Leu Ala Ser
1               5                   10

<210> 325
<211> 13
<212> PRT
<213> Homo sapiens

<400> 325
Pro Lys Tyr Val Lys Gln Asn Thr Leu Lys Leu Ala Lys
1               5                   10

<210> 326
<211> 13
<212> PRT
<213> Homo sapiens

<400> 326
Pro Lys Tyr Val Lys Gln Asn Thr Leu Lys Leu Ala Tyr
1               5                   10

<210> 327
<211> 16
<212> PRT
<213> Homo sapiens

<400> 327
Ile Gln Val Tyr Ser Arg His Pro Ala Glu Asn Gly Lys Ser Asn Phe
1               5                   10                  15

<210> 328
<211> 14
<212> PRT
<213> Homo sapiens

<400> 328
Ile Asn Ser Trp Val Glu Ser Gln Thr Asn Gly Ile Ile Arg
1               5                   10

<210> 329

<211> 13

<212> PRT

<213> Homo sapiens

<400> 329

Pro Lys Tyr Val Lys Gln Asn Thr Leu Lys Leu Ala Ala

1               5                   10

<210> 330

<211> 13

<212> PRT

<213> Homo sapiens

<400> 330

Pro Lys Tyr Val Lys Gln Asn Thr Leu Lys Ala Ala Thr

1               5                   10

<210> 331

<211> 13

<212> PRT

<213> Homo sapiens

<400> 331

Pro Lys Tyr Val Lys Gln Asn Thr Leu Ala Leu Ala Thr

1               5                   10

<210> 332

<211> 13

<212> PRT

<213> Homo sapiens

<400> 332

Pro Lys Tyr Val Lys Gln Asn Thr Ala Lys Leu Ala Thr

1               5                   10

<210> 333

<211> 13

<212> PRT

<213> Homo sapiens

<400> 333

Pro Lys Tyr Val Lys Gln Asn Ala Leu Lys Leu Ala Thr
1               5                   10

<210> 334

<211> 13

<212> PRT

<213> Homo sapiens

<400> 334

Pro Lys Tyr Val Lys Gln Ala Thr Leu Lys Leu Ala Thr
1               5                   10

<210> 335

<211> 13

<212> PRT

<213> Homo sapiens

<400> 335

Pro Lys Tyr Val Lys Ala Asn Thr Leu Lys Leu Ala Thr
1               5                   10

<210> 336

<211> 13

<212> PRT

<213> Homo sapiens

<400> 336

Pro Lys Tyr Val Ala Gln Asn Thr Leu Lys Leu Ala Thr
1               5                   10

<210> 337
<211> 13
<212> PRT
<213> Homo sapiens

<400> 337
Pro Lys Tyr Ala Lys Gln Asn Thr Leu Lys Leu Ala Thr
1               5                   10

<210> 338
<211> 13
<212> PRT
<213> Homo sapiens

<400> 338
Pro Ala Tyr Val Lys Gln Asn Thr Leu Lys Leu Ala Thr
1               5                   10

<210> 339
<211> 13
<212> PRT
<213> Homo sapiens

<400> 339
Ala Lys Tyr Val Lys Gln Asn Thr Leu Lys Leu Ala Thr
1               5                   10

<210> 340
<211> 13
<212> PRT
<213> Homo sapiens

<400> 340
Pro Lys Tyr Val Lys Gln Asn Thr Leu Lys Leu Ala Thr
1               5                   10

<210> 341

<211> 13

<212> PRT

<213> Homo sapiens

<400> 341

Pro Lys Tyr Val Lys Gln Asn Thr Leu Lys Leu Ala Thr
1               5                   10

<210> 342

<211> 13

<212> PRT

<213> Homo sapiens

<400> 342

Pro Lys Tyr Val Lys Gln Asn Thr Leu Lys Leu Ala Thr
1               5                   10

<210> 343

<211> 13

<212> PRT

<213> Homo sapiens

<400> 343

Pro Lys Tyr Val Lys Gln Asn Thr Leu Lys Leu Ala Thr
1               5                   10

<210> 344

<211> 13

<212> PRT

<213> Homo sapiens

<400> 344

Pro Lys Tyr Val Lys Gln Asn Thr Leu Lys Leu Ala Thr
1               5                   10

<210> 345

<400> 345
000

<210> 346
<211> 18
<212> PRT
<213> Homo sapiens

<400> 346
Phe Phe Lys Asn Ile Val Thr Pro Arg Phe Phe Lys Asn Ile Val Thr
1               5                   10                  15
Pro Arg

<210> 347
<211> 13
<212> PRT
<213> Homo sapiens

<400> 347
Ala Arg Arg Leu Lys Ala Ile Tyr Ala Arg Arg Leu Lys
1               5                   10

<210> 348
<211> 14
<212> PRT
<213> Homo sapiens

<400> 348
Gln Tyr Ile Lys Ala Asn Ser Lys Phe Ile Gly Ile Thr Glu
1               5                   10

<210> 349
<211> 14
<212> PRT
<213> Homo sapiens

<400> 349
Thr Tyr Ile Lys Ala Asn Ser Lys Phe Ile Gly Ile Thr Glu
1               5                   10

<210> 350
<211> 14
<212> PRT
<213> Homo sapiens

<400> 350
Leu Tyr Ile Lys Ala Asn Ser Lys Phe Ile Gly Ile Thr Glu
1               5                   10

<210> 351
<211> 14
<212> PRT
<213> Homo sapiens

<400> 351
Asn Tyr Ile Lys Ala Asn Ser Lys Phe Ile Gly Ile Thr Glu
1               5                   10

<210> 352
<211> 14
<212> PRT
<213> Homo sapiens

<400> 352
Gln Phe Ile Lys Ala Asn Ser Lys Phe Ile Gly Ile Thr Glu
1               5                   10

<210> 353

<211> 14

<212> PRT

<213> Homo sapiens

<400> 353

Gln Tyr Gln Lys Ala Asn Ser Lys Phe Ile Gly Ile Thr Glu
1               5                   10

<210> 354

<211> 14

<212> PRT

<213> Homo sapiens

<400> 354

Gln Tyr Lys Lys Ala Asn Ser Lys Phe Ile Gly Ile Thr Glu
1               5                   10

<210> 355

<211> 14

<212> PRT

<213> Homo sapiens

<400> 355

Gln Tyr Val Lys Ala Asn Ser Lys Phe Ile Gly Ile Thr Glu
1               5                   10

<210> 356

<211> 14

<212> PRT

<213> Homo sapiens

<400> 356

Gln Tyr Ile Ser Ala Asn Ser Lys Phe Ile Gly Ile Thr Glu
1               5                   10

<210> 357
<211> 14
<212> PRT
<213> Homo sapiens

<400> 357
Gln Tyr Ile Ile Ala Asn Ser Lys Phe Ile Gly Ile Thr Glu
1               5                   10

<210> 358
<211> 14
<212> PRT
<213> Homo sapiens

<400> 358
Gln Tyr Ile Lys Phe Asn Ser Lys Phe Ile Gly Ile Thr Glu
1               5                   10

<210> 359
<211> 14
<212> PRT
<213> Homo sapiens

<400> 359
Gln Tyr Ile Lys Ala Ala Ser Lys Phe Ile Gly Ile Thr Glu
1               5                   10

<210> 360
<211> 14
<212> PRT
<213> Homo sapiens

<400> 360
Gln Tyr Ile Lys Ala Leu Ser Lys Phe Ile Gly Ile Thr Glu
1               5                   10

<210> 361

<211> 14

<212> PRT

<213> Homo sapiens

<400> 361

Gln Tyr Ile Lys Ala Gln Ser Lys Phe Ile Gly Ile Thr Glu

1               5                   10

<210> 362

<211> 14

<212> PRT

<213> Homo sapiens

<400> 362

Gln Tyr Ile Lys Ala Asn Tyr Lys Phe Ile Gly Ile Thr Glu

1               5                   10

<210> 363

<211> 14

<212> PRT

<213> Homo sapiens

<400> 363

Gln Tyr Ile Lys Ala Asn Ala Lys Phe Ile Gly Ile Thr Glu

1               5                   10

<210> 364

<211> 14

<212> PRT

<213> Homo sapiens

<400> 364

Gln Tyr Ile Lys Ala Asn Ser Ser Phe Ile Gly Ile Thr Glu

1               5                   10

<210> 365
<211> 14
<212> PRT
<213> Homo sapiens

<400> 365
Gln Tyr Ile Lys Ala Asn Ser Glu Phe Ile Gly Ile Thr Glu
1               5                   10

<210> 366
<211> 14
<212> PRT
<213> Homo sapiens

<400> 366
Gln Tyr Ile Lys Ala Asn Ser Arg Phe Ile Gly Ile Thr Glu
1               5                   10

<210> 367
<211> 14
<212> PRT
<213> Homo sapiens

<400> 367
Gln Tyr Ile Lys Ala Asn Ser Lys Glu Ile Gly Ile Thr Glu
1               5                   10

<210> 368
<211> 14
<212> PRT
<213> Homo sapiens

<400> 368
Gln Tyr Ile Lys Ala Asn Ser Lys Ser Ile Gly Ile Thr Glu
1               5                   10

<210> 369

<211> 14

<212> PRT

<213> Homo sapiens

<400> 369

Gln Tyr Ile Lys Ala Asn Ser Lys Tyr Ile Gly Ile Thr Glu
1               5                   10

<210> 370

<211> 14

<212> PRT

<213> Homo sapiens

<400> 370

Gln Tyr Ile Lys Ala Asn Ser Lys Phe Gln Gly Ile Thr Glu
1               5                   10

<210> 371

<211> 14

<212> PRT

<213> Homo sapiens

<400> 371

Gln Tyr Ile Lys Ala Asn Ser Lys Phe Val Gly Ile Thr Glu
1               5                   10

<210> 372

<211> 14

<212> PRT

<213> Homo sapiens

<400> 372

Gln Tyr Ile Lys Ala Asn Ser Lys Phe Ile Gln Ile Thr Glu
1               5                   10

<210> 373
<211> 14
<212> PRT
<213> Homo sapiens

<400> 373
Gln Tyr Ile Lys Ala Asn Ser Lys Phe Ile Tyr Ile Thr Glu
1               5                   10

<210> 374
<211> 14
<212> PRT
<213> Homo sapiens

<400> 374
Gln Tyr Ile Lys Ala Asn Ser Lys Phe Ile Ala Ile Thr Glu
1               5                   10

<210> 375
<211> 14
<212> PRT
<213> Homo sapiens

<400> 375
Gln Tyr Ile Lys Ala Asn Ser Lys Phe Ile Gly Gln Thr Glu
1               5                   10

<210> 376
<211> 14
<212> PRT
<213> Homo sapiens

<400> 376
Gln Tyr Ile Lys Ala Asn Ser Lys Phe Ile Gly Lys Thr Glu
1               5                   10

<210> 377
<211> 14
<212> PRT
<213> Homo sapiens

<400> 377
Gln Tyr Ile Lys Ala Asn Ser Lys Phe Ile Gly Leu Thr Glu
1               5                   10

<210> 378
<211> 14
<212> PRT
<213> Homo sapiens

<400> 378
Gln Tyr Ile Lys Ala Asn Ser Lys Phe Ile Gly Ile Lys Glu
1               5                   10

<210> 379
<211> 14
<212> PRT
<213> Homo sapiens

<400> 379
Gln Tyr Ile Lys Ala Asn Ser Lys Phe Ile Gly Ile Phe Glu
1               5                   10

<210> 380
<211> 14
<212> PRT
<213> Homo sapiens

<400> 380
Gln Tyr Ile Lys Ala Asn Ser Lys Phe Ile Gly Ile Ser Glu
1               5                   10

<210> 381

<211> 14

<212> PRT

<213> Homo sapiens

<400> 381

Gln Tyr Ile Lys Ala Asn Ser Lys Phe Ile Gly Ile Thr Lys

1               5                   10

<210> 382

<211> 14

<212> PRT

<213> Homo sapiens

<400> 382

Gln Tyr Ile Lys Ala Asn Ser Lys Phe Ile Gly Ile Thr Asp

1               5                   10

<210> 383

<211> 14

<212> PRT

<213> Homo sapiens

<400> 383

Gln Tyr Ile Lys Ala Asn Ser Lys Phe Ile Gly Ile Thr Val

1               5                   10

<210> 384

<211> 13

<212> PRT

<213> Homo sapiens

<400> 384

Ala Ala Tyr Ala Ala Ala Ala Ala Ala Lys Ala Ala Ala

1               5                   10

<210> 385
<211> 13
<212> PRT
<213> Homo sapiens

<400> 385
Ala Ala Tyr Ala Ala Ala Ala Ala Ala Lys Ala Ala Ala
1               5                   10

<210> 386
<211> 20
<212> PRT
<213> Homo sapiens

<400> 386
Val His His Ala Asn Glu Asn Ile Phe Tyr Cys Pro Ile Ala Ile Met
1               5                   10                  15
Ser Ala Leu Ala
            20

<210> 387
<211> 19
<212> PRT
<213> Homo sapiens

<400> 387
Cys Pro Ile Ala Ile Met Ser Ala Leu Ala Met Val Tyr Leu Gly Ala
1               5                   10                  15
Lys Asp Ser

<210> 388
<211> 19
<212> PRT
<213> Homo sapiens

<400> 388

Leu Met Ala Met Gly Ile Thr Asp Val Phe Ser Ser Ser Ala Asn Leu
1               5                   10                  15

Ser Gly Ile

<210> 389

<211> 20

<212> PRT

<213> Homo sapiens

<400> 389

Ser Ser Ser Ala Asn Leu Ser Gly Ile Ser Ser Ala Glu Ser Leu Lys
1               5                   10                  15

Ile Ser Gln Ala
            20

<210> 390

<211> 13

<212> PRT

<213> Homo sapiens

<400> 390

Pro Lys Tyr Val Lys Gln Asn Thr Leu Lys Leu Ala Thr
1               5                   10

<210> 391

<211> 13

<212> PRT

<213> Homo sapiens

<400> 391

Pro Glu Phe Val Arg Gln Asn Thr Leu Lys Gln Ala Thr
1               5                   10

<210> 392

<211> 18

<212> PRT

<213> Homo sapiens

<400> 392

Phe Ala Lys Gln Asn Thr Phe Ala Lys Gln Asn Thr Phe Ala Lys Gln
1               5                   10                  15

Asn Thr

<210> 393

<211> 20

<212> PRT

<213> Homo sapiens

<400> 393

Tyr Val Lys Gln Asn Thr Leu Lys Leu Ala Tyr Val Lys Gln Asn Thr
1               5                   10                  15

Leu Lys Leu Ala
            20

<210> 394

<211> 13

<212> PRT

<213> Homo sapiens

<400> 394

Pro Lys Tyr Glu Lys Gln Asn Thr Leu Lys Leu Ala Thr
1               5                   10

<210> 395

<211> 13

<212> PRT

<213> Homo sapiens

<400> 395

Pro Lys Tyr Val Lys Glu Asn Thr Leu Lys Leu Ala Thr

1               5                   10

<210> 396

<211> 13

<212> PRT

<213> Homo sapiens

<400> 396

Pro Lys Tyr Val Lys Gln Glu Thr Leu Lys Leu Ala Thr

1               5                   10

<210> 397

<211> 13

<212> PRT

<213> Homo sapiens

<400> 397

Pro Lys Tyr Val Lys Gln Asn Thr Glu Lys Leu Ala Thr

1               5                   10

<210> 398

<211> 13

<212> PRT

<213> Homo sapiens

<400> 398

Pro Lys Tyr Val Val Gln Asn Thr Leu Lys Leu Ala Thr

1               5                   10

<210> 399

<211> 12

<212> PRT

<213> Homo sapiens

<400> 399

Pro Lys Tyr Val Gln Asn Thr Leu Leu Leu Ala Thr
1               5                   10

<210> 400

<211> 13

<212> PRT

<213> Homo sapiens

<400> 400

Pro Lys Tyr Val Lys Gln Lys Thr Leu Lys Leu Ala Thr
1               5                   10

<210> 401

<211> 14

<212> PRT

<213> Homo sapiens

<400> 401

Tyr Ala Arg Phe Gln Arg Gln Thr Thr Leu Lys Ile Ala Thr
1               5                   10

<210> 402

<211> 14

<212> PRT

<213> Homo sapiens

<400> 402

Tyr Ala Arg Phe Gln Ser Gln Thr Thr Leu Lys Gln Lys Thr
1               5                   10

<210> 403
<211> 14
<212> PRT
<213> Homo sapiens

<400> 403
Tyr Ala Arg Phe Gln Ser Gln Thr Thr Leu Lys Ala Lys Thr
1               5                   10

<210> 404
<211> 14
<212> PRT
<213> Homo sapiens

<400> 404
Tyr Ala Ser Phe Val Ser Gln Thr Ala Gln Arg Leu Phe Tyr
1               5                   10

<210> 405
<211> 14
<212> PRT
<213> Homo sapiens

<400> 405
Tyr Ala Glu Phe Gln Ala Glu Lys Thr Leu Lys Ala Ala Ser
1               5                   10

<210> 406
<211> 14
<212> PRT
<213> Homo sapiens

<400> 406
Tyr Ala Arg Phe Gln Ser Gln Thr Thr Leu Lys Ala Lys Thr
1               5                   10

<210> 407

<211> 14

<212> PRT

<213> Homo sapiens

<400> 407

Tyr Ala Arg Phe Gln Ser Glu Thr Thr Glu Lys Ala Lys Thr

1               5                   10

<210> 408

<211> 14

<212> PRT

<213> Homo sapiens

<400> 408

Tyr Ala Arg Phe Gln Arg Gln Thr Thr Leu Lys Ala Ala Ala

1               5                   10

<210> 409

<211> 14

<212> PRT

<213> Homo sapiens

<400> 409

Tyr Ala Arg Phe Gln Arg Gln Thr Thr Leu Lys Ala Ala Ala

1               5                   10

<210> 410

<211> 14

<212> PRT

<213> Homo sapiens

<400> 410

Tyr Ala Arg Phe Gln Ser Leu Thr Thr Leu Lys Ala Lys Thr

1               5                   10

<210> 411

<211> 14

<212> PRT

<213> Homo sapiens

<400> 411

Tyr Ala Arg Phe Gln Ser Gln Ser Thr Leu Lys Ala Lys Thr
1               5                   10

<210> 412

<211> 14

<212> PRT

<213> Homo sapiens

<400> 412

Tyr Ala Arg Phe Gln Ser Gln Glu Thr Leu Lys Ala Lys Thr
1               5                   10

<210> 413

<211> 14

<212> PRT

<213> Homo sapiens

<400> 413

Tyr Ala Arg Phe Gln Ser Gln Thr Thr Ile Lys Ala Lys Thr
1               5                   10

<210> 414

<211> 14

<212> PRT

<213> Homo sapiens

<400> 414

Tyr Ala Arg Phe Gln Ser Gln Thr Thr Lys Lys Ala Lys Thr
1               5                   10

<210> 415
<211> 14
<212> PRT
<213> Homo sapiens

<400> 415
Tyr Ala Arg Phe Gln Ser Gln Thr Thr Leu Arg Ala Lys Thr
1               5                   10

<210> 416
<211> 14
<212> PRT
<213> Homo sapiens

<400> 416
Tyr Ala Arg Phe Gln Ser Gln Thr Thr Leu Glu Ala Lys Thr
1               5                   10

<210> 417
<211> 14
<212> PRT
<213> Homo sapiens

<400> 417
Tyr Ala Arg Phe Gln Ser Gln Thr Thr Leu Lys Ser Lys Thr
1               5                   10

<210> 418
<211> 14
<212> PRT
<213> Homo sapiens

<400> 418
Tyr Ala Arg Phe Gln Ser Gln Thr Thr Leu Lys Ala Arg Thr
1               5                   10

<210> 419

<211> 14

<212> PRT

<213> Homo sapiens

<400> 419

Tyr Ala Arg Phe Gln Ser Gln Thr Thr Leu Lys Ala Glu Thr
1               5                   10

<210> 420

<211> 14

<212> PRT

<213> Homo sapiens

<400> 420

Tyr Ala Arg Phe Gln Ser Gln Thr Thr Leu Lys Ala Lys Glu
1               5                   10

<210> 421

<211> 14

<212> PRT

<213> Homo sapiens

<400> 421

Tyr Ala Arg Ala Gln Ser Gln Thr Thr Leu Lys Ala Lys Thr
1               5                   10

<210> 422

<211> 14

<212> PRT

<213> Homo sapiens

<400> 422

Tyr Ala Ala Phe Gln Ser Gln Thr Thr Leu Lys Ala Lys Thr
1               5                   10

<210> 423

<211> 14

<212> PRT

<213> Homo sapiens

<400> 423

Tyr Ala Arg Phe Gln Ser Gln Thr Ser Leu Lys Ala Lys Thr
1               5                   10

<210> 424

<211> 14

<212> PRT

<213> Homo sapiens

<400> 424

Tyr Ala Arg Phe Gln Ser Gln Thr Val Leu Lys Ala Lys Thr
1               5                   10

<210> 425

<211> 14

<212> PRT

<213> Homo sapiens

<400> 425

Tyr Ala Arg Phe Gln Ser Gln Thr Tyr Leu Lys Ala Lys Thr
1               5                   10

<210> 426

<211> 14

<212> PRT

<213> Homo sapiens

<400> 426

Tyr Ala Arg Phe Gln Ser Gln Thr Pro Leu Lys Ala Lys Thr
1               5                   10

<210> 427

<211> 14

<212> PRT

<213> Homo sapiens

<400> 427

Tyr Ser Arg Phe Gln Ser Gln Thr Thr Leu Lys Ala Lys Thr
1               5                   10

<210> 428

<211> 14

<212> PRT

<213> Homo sapiens

<400> 428

Tyr Glu Arg Phe Gln Ser Gln Thr Thr Leu Lys Ala Lys Thr
1               5                   10

<210> 429

<211> 14

<212> PRT

<213> Homo sapiens

<400> 429

Tyr Ala Lys Phe Gln Ser Gln Thr Thr Leu Lys Ala Lys Thr
1               5                   10

<210> 430

<211> 14

<212> PRT

<213> Homo sapiens

<400> 430

Tyr Ala Glu Phe Gln Ser Gln Thr Thr Leu Lys Ala Lys Thr
1               5                   10

<210> 431
<211> 14
<212> PRT
<213> Homo sapiens

<400> 431
Tyr Ala Arg Tyr Gln Ser Gln Thr Thr Leu Lys Ala Lys Thr
1               5                   10

<210> 432
<211> 14
<212> PRT
<213> Homo sapiens

<400> 432
Tyr Ala Arg Phe Asn Ser Gln Thr Thr Leu Lys Ala Lys Thr
1               5                   10

<210> 433
<211> 14
<212> PRT
<213> Homo sapiens

<400> 433
Tyr Ala Arg Phe Leu Ser Gln Thr Thr Leu Lys Ala Lys Thr
1               5                   10

<210> 434
<211> 14
<212> PRT
<213> Homo sapiens

<400> 434
Tyr Ala Arg Phe Gln Phe Gln Thr Thr Leu Lys Ala Lys Thr
1               5                   10

<210> 435

<211> 14

<212> PRT

<213> Homo sapiens

<400> 435

Tyr Ala Arg Phe Gln Ser Asn Thr Thr Leu Lys Ala Lys Thr
1               5                   10

<210> 436

<211> 14

<212> PRT

<213> Homo sapiens

<400> 436

Tyr Ala Arg Phe Gln Ser Gln Thr Thr Leu Lys Ala Lys Ala
1               5                   10

<210> 437

<211> 14

<212> PRT

<213> Homo sapiens

<400> 437

Tyr Ala Arg Phe Gln Ser Gln Thr Thr Leu Lys Ala Ala Thr
1               5                   10

<210> 438

<211> 14

<212> PRT

<213> Homo sapiens

<400> 438

Tyr Ala Arg Phe Gln Ser Gln Thr Thr Leu Ala Ala Lys Thr
1               5                   10

<210> 439

<211> 14

<212> PRT

<213> Homo sapiens

<400> 439

Tyr Ala Arg Phe Gln Ser Gln Thr Thr Ala Lys Ala Lys Thr
1               5                   10

<210> 440

<211> 14

<212> PRT

<213> Homo sapiens

<400> 440

Tyr Ala Arg Phe Gln Ser Gln Thr Ala Leu Lys Ala Lys Thr
1               5                   10

<210> 441

<211> 14

<212> PRT

<213> Homo sapiens

<400> 441

Tyr Ala Arg Phe Gln Ser Gln Ala Thr Leu Lys Ala Lys Thr
1               5                   10

<210> 442

<211> 14

<212> PRT

<213> Homo sapiens

<400> 442

Tyr Ala Arg Phe Gln Ser Ala Thr Thr Leu Lys Ala Lys Thr
1               5                   10

<210> 443
<211> 14
<212> PRT
<213> Homo sapiens

<400> 443
Tyr Ala Arg Phe Gln Ala Gln Thr Thr Leu Lys Ala Lys Thr
1               5                   10

<210> 444
<211> 14
<212> PRT
<213> Homo sapiens

<400> 444
Tyr Ala Arg Phe Ala Ser Gln Thr Thr Leu Lys Ala Lys Thr
1               5                   10

<210> 445
<211> 14
<212> PRT
<213> Homo sapiens

<400> 445
Tyr Ala Arg Phe Gln Ser Gln Thr Thr Leu Lys Ala Lys Thr
1               5                   10

<210> 446

<400> 446
000

<210> 447
<211> 15
<212> PRT
<213> Homo sapiens

<400> 447
Tyr Gln Phe Val Arg Ala Asn Ala Arg Ser Ile Gly Ile Thr Lys
1               5                   10                  15

<210> 448
<211> 14
<212> PRT
<213> Homo sapiens

<400> 448
Tyr Ala Ala Tyr Val Lys Leu Asn Ala Ile Arg Ile Ser Ser
1               5                   10

<210> 449
<211> 14
<212> PRT
<213> Homo sapiens

<400> 449
Tyr Ala Arg Phe Gln Arg Gln Thr Thr Leu Lys Ala Ala Ala
1               5                   10

<210> 450
<211> 11
<212> PRT
<213> Homo sapiens

<400> 450
Tyr Glu Phe Val Ala Ala Lys Ala Ala Gln Lys
1               5                   10

<210> 451
<211> 14
<212> PRT
<213> Homo sapiens

<400> 451
Tyr Ile Ala Phe Leu Lys Gln Ala Thr Ala Lys Leu Ala Thr
1               5                   10

<210> 452
<211> 20
<212> PRT
<213> Homo sapiens

<400> 452
Ile Asn Ser Trp Val Glu Ser Gln Thr Asn Gly Ile Ile Arg Asn Val
1               5                   10                  15

Leu Gln Pro Ser
            20

<210> 453
<211> 20
<212> PRT
<213> Homo sapiens

<400> 453
Gly Ile Val Glu Gln Ser Ser Thr Ser Ile Ser Ser Leu Tyr Gln Leu
1               5                   10                  15
Glu Asn Tyr Ser
            20

<210> 454

<211> 20

<212> PRT

<213> Homo sapiens

<400> 454

Leu Ser Gly Ser His Leu Val Glu Ala Leu Tyr Leu Val Ser Gly Glu
1               5                   10                  15

Arg Gly Phe Phe
            20

<210> 455

<211> 20

<212> PRT

<213> Homo sapiens

<400> 455

Leu Val Glu Ala Leu Tyr Leu Val Ser Gly Glu Arg Gly Phe Phe Tyr
1               5                   10                  15

Thr Pro Lys Thr
            20

<210> 456

<211> 18

<212> PRT

<213> Homo sapiens

<400> 456

Lys Ser Asn Thr Ala Thr Ser Ala Thr Gln Arg Leu Ala Asn Ser Leu
1               5                   10                  15

Val His

<210> 457

<211> 19

<212> PRT

<213> Homo sapiens

<400> 457

Thr Ser Ala Thr Gln Arg Leu Ala Asn Ser Leu Val His Ser Ser Asn
1               5                   10                  15

Asn Phe Gly

<210> 458

<211> 19

<212> PRT

<213> Homo sapiens

<400> 458

Arg Leu Ala Asn Ser Leu Val His Ser Ser Asn Asn Phe Gly Ala Ile
1               5                   10                  15

Leu Ser Ser

<210> 459

<211> 19

<212> PRT

<213> Homo sapiens

<400> 459

Leu Val His Ser Ser Asn Asn Phe Gly Ala Ile Leu Ser Ser Thr Asn
1               5                   10                  15

Val Gly Ser

<210> 460

<211> 14

<212> PRT

<213> Homo sapiens

<400> 460

Ser Asn Asn Phe Gly Ala Ile Leu Ser Ser Thr Asn Val Gly
 1               5                   10

<210> 461

<211> 13

<212> PRT

<213> Homo sapiens

<400> 461

Ala Ala Leu Ala Ala Ala Ala Ala Ala Lys Ala Ala Ala
 1               5                   10

<210> 462

<211> 13

<212> PRT

<213> Homo sapiens

<220>

<221> SITE

<222> (1)..(2)

<223> Xaa is disclosed as J

<400> 462

Xaa Xaa Tyr Ala Ala Ala Ala Ala Ala Lys Ala Ala Ala
 1               5                   10

<210> 463

<211> 15

<212> PRT

<213> Homo sapiens

<400> 463

Ala Val Leu Glu Asp Pro Tyr Ile Leu Leu Val Ser Ser Lys Val

1               5                   10                  15

<210> 464

<211> 14

<212> PRT

<213> Homo sapiens

<400> 464

Gln Phe Val Ile Ala Asn Ala Ser Ser Val Ala Lys Thr Asp

1               5                   10

<210> 465

<211> 14

<212> PRT

<213> Homo sapiens

<400> 465

Ala Phe Ala Ala Ala Thr Lys Ala Ala Ala Phe Ala Ala Tyr

1               5                   10

<210> 466

<211> 13

<212> PRT

<213> Homo sapiens

<400> 466

Ala Ala Phe Ala Ala Ala Ala Ala Gln Arg Leu Phe Ala

1               5                   10

<210> 467

<211> 13

<212> PRT

<213> Homo sapiens

<400> 467

Ala Ala Tyr Ala Ala Ala Ala Ala Ala Lys Ala Ala Ala

1               5                   10

<210> 468

<211> 13

<212> PRT

<213> Homo sapiens

<400> 468

Ala Ala Tyr Ala Ala Ala Ala Thr Ala Lys Ala Ala Ala

1               5                   10

<210> 469

<211> 18

<212> PRT

<213> Homo sapiens

<400> 469

Ala Ser Gln Lys Arg Pro Ser Gln Arg Ser Lys Tyr Leu Ala Thr Ala

1               5                   10                  15

Ser Thr

<210> 470

<211> 18

<212> PRT

<213> Homo sapiens

<400> 470

Lys Tyr Leu Ala Thr Ala Ser Thr Met Asp His Ala Arg His Gly Phe
1               5                   10                  15

Leu Pro

<210> 471

<211> 19

<212> PRT

<213> Homo sapiens

<400> 471

Asn Pro Val Val His Phe Phe Lys Asn Ile Val Thr Pro Arg Thr Pro
1               5                   10                  15

Pro Pro Ser

<210> 472

<211> 10

<212> PRT

<213> Homo sapiens

<400> 472

Ala Phe Ala Ala Ala Ala Thr Leu Lys Ala
1               5                   10

<210> 473

<211> 10

<212> PRT

<213> Homo sapiens

<400> 473

Ala Phe Ala Ala Ala Ala Thr Leu Lys Ala
1               5                   10

<210> 474

<211> 15

<212> PRT

<213> Homo sapiens

<400> 474

Asp Ala Leu Glu Ser Ile Met Thr Thr Lys Ser Val Ser Phe Arg
1               5                   10                  15

<210> 475

<211> 13

<212> PRT

<213> Homo sapiens

<400> 475

Leu Leu Val Ile Val Glu Leu Ile Pro Ser Thr Ser Ser
1               5                   10

<210> 476

<211> 11

<212> PRT

<213> Homo sapiens

<400> 476

Phe Gln Arg Gln Thr Thr Leu Lys Ala Ala Ala
1               5                   10

<210> 477

<211> 14

<212> PRT

<213> Homo sapiens

<400> 477

Tyr Ala Arg Phe Gln Arg Gln Thr Thr Leu Lys Ala Ala Ala
1               5                   10

<210> 478

<211> 14

<212> PRT

<213> Homo sapiens

<400> 478

Tyr Ala Arg Phe Gln Arg Gln Thr Thr Leu Lys Ala Ala Ala
1               5                   10

<210> 479

<211> 14

<212> PRT

<213> Homo sapiens

<400> 479

Tyr Ala Lys Phe Gln Arg Gln Thr Thr Leu Lys Ala Ala Ala
1               5                   10

<210> 480

<211> 14

<212> PRT

<213> Homo sapiens

<400> 480

Tyr Ala Ala Phe Gln Arg Gln Thr Thr Leu Lys Ala Ala Ala
1               5                   10

<210> 481

<211> 14

<212> PRT

<213> Homo sapiens

<400> 481

Tyr Ala Arg Phe Gln Lys Gln Thr Thr Leu Lys Ala Ala Ala
1               5                   10

<210> 482

<211> 14

<212> PRT

<213> Homo sapiens

<400> 482

Tyr Ala Arg Phe Ala Ser Gln Thr Thr Leu Lys Ala Lys Thr
1               5                   10

<210> 483

<211> 14

<212> PRT

<213> Homo sapiens

<400> 483

Gly Ala Ala Tyr Ala Ala Ala Ala Ala Ala Lys Ala Ala Ala
1               5                   10

<210> 484

<211> 13

<212> PRT

<213> Homo sapiens

<400> 484

Pro Lys Val Val Lys Gln Asn Thr Leu Lys Leu Ala Thr
1               5                   10

<210> 485

<211> 13

<212> PRT

<213> Homo sapiens

<400> 485

Pro Lys Tyr Val Lys Gln Asn Thr Leu Lys Phe Ala Thr
1               5                   10

<210> 486

<211> 20

<212> PRT

<213> Homo sapiens

<400> 486

Pro Leu Leu Val Leu Gln Ala Gly Phe Phe Leu Leu Thr Arg Ile Leu
1               5                   10                  15

Thr Ile Pro Gln
            20

<210> 487

<211> 17

<212> PRT

<213> Homo sapiens

<400> 487

Met Gly Gln Asn Leu Ser Thr Ser Asn Pro Leu Gly Phe Phe Pro Asp
1               5                   10                  15

His

<210> 488

<211> 17

<212> PRT

<213> Homo sapiens

<400> 488

Asn Pro Val Leu Thr Thr Ala Ser Pro Leu Ser Ser Ile Phe Ser Arg
1               5                   10                  15

Ile

<210> 489
<211> 18
<212> PRT
<213> Homo sapiens

<400> 489
Cys Trp Gly Glu Leu Met Thr Leu Ala Thr Trp Val Gly Val Asn Leu
1               5                   10                  15
Glu Asp

<210> 490
<211> 15
<212> PRT
<213> Homo sapiens

<400> 490
Gly Arg Glu Thr Val Ile Glu Tyr Leu Val Ser Phe Gly Val Trp
1               5                   10                  15

<210> 491
<211> 12
<212> PRT
<213> Homo sapiens

<400> 491
Ala Phe Ala Ala Ala Lys Thr Ala Ala Ala Ala Ala
1               5                   10

<210> 492
<211> 13
<212> PRT
<213> Homo sapiens

<400> 492
Ala Ala Phe Ala Ala Ala Lys Thr Ala Ala Ala Ala Ala
1               5                   10

<210> 493
<211> 15
<212> PRT
<213> Homo sapiens

<400> 493
Tyr Gln Phe Ile Lys Ala Asn Ser Lys Phe Lys Gly Lys Phe Lys
1               5                   10                  15

<210> 494
<211> 14
<212> PRT
<213> Homo sapiens

<400> 494
Tyr Ala Ala Tyr Val Lys Leu Thr Ala Ala Arg Lys Phe Ala
1               5                   10

<210> 495
<211> 14
<212> PRT
<213> Homo sapiens

<400> 495
Tyr Ala Ala Tyr Val Lys Leu Thr Ala Ala Arg Lys Phe Ala
1               5                   10

<210> 496

<211> 17

<212> PRT

<213> Homo sapiens

<400> 496

Gln Tyr Ile Lys Ala Asn Ser Lys Phe Ile Gly Ile Thr Glu Leu Lys
1               5                   10                  15

Lys

<210> 497

<211> 17

<212> PRT

<213> Homo sapiens

<400> 497

Ile Leu Met Gln Tyr Ile Lys Ala Asn Ser Lys Phe Ile Gly Ile Thr
1               5                   10                  15

Glu

<210> 498

<211> 16

<212> PRT

<213> Homo sapiens

<400> 498

Leu Met Gln Tyr Ile Lys Ala Asn Ser Lys Phe Ile Gly Ile Thr Glu
1               5                   10                  15

<210> 499

<211> 14

<212> PRT

<213> Homo sapiens

<400> 499

Gln Tyr Ile Lys Ala Asn Ser Lys Phe Ile Gly Ile Thr Glu
1               5                   10

<210> 500
<211> 15
<212> PRT
<213> Homo sapiens

<400> 500

Met Gln Tyr Ile Lys Ala Asn Ser Lys Phe Ile Gly Ile Thr Glu
1               5                   10                  15

<210> 501
<211> 13
<212> PRT
<213> Homo sapiens

<400> 501

Pro Lys Phe Val Lys Gln Asn Thr Leu Lys Leu Ala Gly
1               5                   10

<210> 502
<211> 12
<212> PRT
<213> Homo sapiens

<400> 502

Pro Lys Phe Val Gln Asn Thr Leu Lys Leu Gly Thr
1               5                   10

<210> 503

<211> 13

<212> PRT

<213> Homo sapiens

<400> 503

Pro Lys Phe Val Lys Gln Asn Thr Leu Lys Gly Ala Thr
1               5                   10

<210> 504

<211> 13

<212> PRT

<213> Homo sapiens

<400> 504

Pro Lys Phe Val Lys Gln Asn Thr Leu Gly Leu Ala Thr
1               5                   10

<210> 505

<211> 13

<212> PRT

<213> Homo sapiens

<400> 505

Pro Lys Phe Val Lys Gln Asn Thr Gly Lys Leu Ala Thr
1               5                   10

<210> 506

<211> 13

<212> PRT

<213> Homo sapiens

<400> 506

Pro Lys Phe Val Lys Gln Asn Gly Leu Lys Leu Ala Thr
1               5                   10

<210> 507
<211> 13
<212> PRT
<213> Homo sapiens

<400> 507
Pro Lys Phe Val Lys Gln Gly Thr Leu Lys Leu Ala Thr
1               5                   10

<210> 508
<211> 13
<212> PRT
<213> Homo sapiens

<400> 508
Pro Lys Phe Val Lys Gly Asn Thr Leu Lys Leu Ala Thr
1               5                   10

<210> 509
<211> 13
<212> PRT
<213> Homo sapiens

<400> 509
Pro Lys Phe Val Gly Gln Asn Thr Leu Lys Leu Ala Thr
1               5                   10

<210> 510
<211> 13
<212> PRT
<213> Homo sapiens

<400> 510
Pro Lys Phe Gly Lys Gln Asn Thr Leu Lys Leu Ala Thr
1               5                   10

<210> 511

<211> 12

<212> PRT

<213> Homo sapiens

<400> 511

Pro Gly Phe Val Lys Gln Asn Thr Lys Leu Ala Thr
1               5                   10

<210> 512

<211> 12

<212> PRT

<213> Homo sapiens

<400> 512

Gly Lys Phe Val Lys Gln Asn Thr Lys Leu Ala Thr
1               5                   10

<210> 513

<211> 11

<212> PRT

<213> Homo sapiens

<400> 513

Phe Ala Ala Ala Lys Thr Ala Ala Ala Phe Ala
1               5                   10

<210> 514

<211> 14

<212> PRT

<213> Homo sapiens

<400> 514

Tyr Ala Ala Phe Ala Ala Thr Lys Thr Ala Ala Phe Ala Ala
1               5                   10

<210> 515

<211> 13

<212> PRT

<213> Homo sapiens

<400> 515

Ala Ala Phe Ala Ala Ala Ala Pro Lys Ala Ala Ala Ala

1               5                   10

<210> 516

<211> 14

<212> PRT

<213> Homo sapiens

<400> 516

Tyr Ala Ala Phe Ala Ala Ala Ala Pro Lys Ala Ala Ala Ala

1               5                   10

<210> 517

<211> 13

<212> PRT

<213> Homo sapiens

<400> 517

Ala Ala Phe Ala Ala Ala Ala Thr Lys Ala Ala Ala Ala

1               5                   10

<210> 518

<211> 14

<212> PRT

<213> Homo sapiens

<400> 518

Tyr Ala Ala Phe Ala Ala Ala Ala Thr Lys Ala Ala Ala Ala

1               5                   10

<210> 519

<211> 19

<212> PRT

<213> Homo sapiens

<400> 519

Tyr Gln Ala Gly Phe Phe Leu Leu Thr Arg Ile Leu Thr Ile Pro Gln
1               5                   10                  15

Ser Leu Asp

<210> 520

<211> 13

<212> PRT

<213> Homo sapiens

<400> 520

Pro Ser Phe Gln Val Gln Thr Thr Leu Leu Leu Ala Thr
1               5                   10

<210> 521

<211> 13

<212> PRT

<213> Homo sapiens

<400> 521

Pro Lys Phe Val Lys Leu Asn Thr Lys Lys Leu Ala Thr
1               5                   10

<210> 522

<211> 13

<212> PRT

<213> Homo sapiens

<400> 522

Pro Arg Tyr Val Arg Gln Asn Thr Leu Arg Leu Ala Thr
1               5                   10

<210> 523

<211> 15

<212> PRT

<213> Homo sapiens

<400> 523

Cys Pro Lys Tyr Val Lys Gln Asn Thr Leu Lys Leu Ala Thr Gly
1               5                   10                  15

<210> 524

<211> 15

<212> PRT

<213> Homo sapiens

<400> 524

Cys Pro Lys Tyr Val Lys Gln Asn Thr Leu Lys Leu Ala Thr Ala
1               5                   10                  15

<210> 525

<211> 14

<212> PRT

<213> Homo sapiens

<400> 525

Tyr Ala Lys Phe Gln Ser Gln Thr Thr Leu Lys Ala Lys Thr
1               5                   10

<210> 526

<211> 14

<212> PRT

<213> Homo sapiens

<400> 526

Tyr Ala Lys Phe Gln Ser Gln Thr Thr Leu Lys Ala Lys Thr
1               5                   10

<210> 527

<211> 14

<212> PRT

<213> Homo sapiens

<400> 527

Tyr Ala Ala Phe Gln Ser Gln Thr Thr Leu Lys Ala Lys Thr
1               5                   10

<210> 528

<211> 14

<212> PRT

<213> Homo sapiens

<400> 528

Tyr Ala Ala Phe Gln Ser Gln Thr Thr Leu Lys Ala Lys Thr
1               5                   10

<210> 529

<211> 14

<212> PRT

<213> Homo sapiens

<400> 529

Tyr Ala Arg Phe Gln Ser Gln Thr Thr Leu Lys Ala Lys Thr
1               5                   10

<210> 530

<211> 14

<212> PRT

<213> Homo sapiens

<400> 530

Tyr Ala Ala Phe Gln Arg Gln Thr Thr Leu Lys Ala Ala Ala

1               5                   10

<210> 531

<211> 14

<212> PRT

<213> Homo sapiens

<400> 531

Tyr Ala Arg Phe Gln Arg Gln Thr Thr Leu Lys Ala Ala Ala

1               5                   10

<210> 532

<211> 12

<212> PRT

<213> Homo sapiens

<400> 532

Pro Ala Phe Glu Ala Leu Ala Gln Arg Ile Phe Lys

1               5                   10

<210> 533

<211> 13

<212> PRT

<213> Homo sapiens

<400> 533

Pro Lys Tyr Val Glu Gln Leu Ser Leu Arg Ala Ala Lys
1               5                   10

<210> 534

<211> 13

<212> PRT

<213> Homo sapiens

<400> 534

Lys Glu Val Val Val Leu Asn Thr Leu Lys Leu Ala Ala
1               5                   10

<210> 535

<211> 16

<212> PRT

<213> Homo sapiens

<400> 535

Val Ser Phe Gly Val Trp Ile Arg Thr Pro Pro Ala Tyr Arg Pro Pro
1               5                   10                  15

<210> 536

<211> 14

<212> PRT

<213> Homo sapiens

<400> 536

Val Ser Phe Gly Val Trp Ile Arg Thr Pro Pro Ala Tyr Arg
1               5                   10

<210> 537

<211> 13

<212> PRT

<213> Homo sapiens

<400> 537

Asn Lys Tyr Val Lys Gln Asn Thr Leu Lys Leu Ala Thr
1               5                   10

<210> 538

<211> 13

<212> PRT

<213> Homo sapiens

<400> 538

Pro Asn Tyr Val Lys Gln Asn Thr Leu Lys Leu Ala Thr
1               5                   10

<210> 539

<211> 13

<212> PRT

<213> Homo sapiens

<400> 539

Pro Lys Tyr Asn Lys Gln Asn Thr Leu Lys Leu Ala Thr
1               5                   10

<210> 540

<211> 13

<212> PRT

<213> Homo sapiens

<400> 540

Pro Lys Tyr Val Asn Gln Asn Thr Leu Lys Leu Ala Thr
1               5                   10

<210> 541
<211> 13
<212> PRT
<213> Homo sapiens

<400> 541
Pro Lys Tyr Val Lys Asn Asn Thr Leu Lys Leu Ala Thr
1               5                   10

<210> 542
<211> 13
<212> PRT
<213> Homo sapiens

<400> 542
Pro Lys Tyr Val Lys Gln Asn Thr Leu Asn Leu Ala Thr
1               5                   10

<210> 543
<211> 13
<212> PRT
<213> Homo sapiens

<400> 543
Pro Lys Tyr Val Lys Gln Asn Thr Leu Lys Asn Ala Thr
1               5                   10

<210> 544
<211> 13
<212> PRT
<213> Homo sapiens

<400> 544
Pro Lys Tyr Val Lys Gln Asn Thr Leu Lys Leu Asn Thr
1               5                   10

<210> 545

<211> 19

<212> PRT

<213> Homo sapiens

<400> 545

Asp Thr Val Lys Leu Met Tyr Lys Gly Gln Pro Met Thr Phe Arg Leu
1               5                   10                  15

Leu Leu Val

<210> 546

<211> 13

<212> PRT

<213> Homo sapiens

<400> 546

Ala Lys Phe Ala Lys Ala Ala Thr Leu Lys Ala Ala Ala
1               5                   10

<210> 547

<211> 13

<212> PRT

<213> Homo sapiens

<400> 547

Ala Ala Phe Lys Ala Ala Ala Thr Leu Lys Ala Ala Ala
1               5                   10

<210> 548

<211> 15

<212> PRT

<213> Homo sapiens

<400> 548

Ala Ala Phe Ala Lys Ala Asn Ser Lys Phe Ile Ala Ala Ala Ala
1               5                   10                  15

<210> 549
<211> 15
<212> PRT
<213> Homo sapiens

<400> 549
Ala Ala Phe Ala Lys Ala Asn Ser Lys Phe Ile Ala Ala Ala Ala
1               5                   10                  15

<210> 550
<211> 13
<212> PRT
<213> Homo sapiens

<400> 550
Ala Ala Phe Ala Lys Ala Ala Thr Lys Phe Ile Ala Ala
1               5                   10

<210> 551
<211> 13
<212> PRT
<213> Homo sapiens

<400> 551
Ala Lys Phe Ala Lys Ala Asn Thr Leu Lys Ala Ala Ala
1               5                   10

<210> 552
<211> 13
<212> PRT
<213> Homo sapiens

<400> 552
Ala Lys Phe Val Lys Ala Ala Thr Leu Lys Ala Ala Ala
1               5                   10

<210> 553

<211> 13

<212> PRT

<213> Homo sapiens

<400> 553

Ala Lys Phe Val Lys Ala Asn Thr Leu Lys Ala Ala Ala
1               5                   10

<210> 554

<211> 20

<212> PRT

<213> Homo sapiens

<400> 554

Lys Ser Ser Gln Tyr Ile Lys Ala Asn Ser Lys Phe Ile Gly Ile Thr
1               5                   10                  15

Glu Ala Ala Ala
            20

<210> 555

<211> 17

<212> PRT

<213> Homo sapiens

<400> 555

Lys Ser Ser Gln Tyr Ile Lys Ala Asn Ser Lys Phe Ile Gly Ile Thr
1               5                   10                  15

Glu

<210> 556

<211> 13

<212> PRT

<213> Homo sapiens

<220>
<221> SITE
<222> (3)
<223> Xaa is uncertain

<400> 556
Ala Val Xaa Val Phe Met Ala Thr Leu Lys Ala Phe Ala
1               5                   10

<210> 557
<211> 13
<212> PRT
<213> Homo sapiens

<220>
<221> SITE
<222> (3)
<223> Xaa is uncertain

<400> 557
Ala Ala Xaa Ala Ala Met Lys Thr Ala Ala Ala Ala Ala
1               5                   10

<210> 558
<211> 13
<212> PRT
<213> Homo sapiens

<400> 558
Pro Lys Tyr Val Lys Met Asn Thr Leu Lys Leu Ala Thr
1               5                   10

<210> 559
<211> 20
<212> PRT
<213> Homo sapiens

<400> 559
Ala Thr Ser Thr Lys Lys Leu His Lys Glu Pro Ala Thr Leu Ile Lys
1               5                   10                  15
Ala Ile Asp Gly
            20

<210> 560
<211> 19
<212> PRT
<213> Homo sapiens

<400> 560
Arg Thr Asp Lys Tyr Gly Arg Gly Leu Ala Tyr Ile Tyr Ala Asp Gly
1               5                   10                  15
Lys Met Val

<210> 561
<211> 19
<212> PRT
<213> Homo sapiens

<400> 561
Glu Ala Leu Val Arg Gln Gly Leu Ala Lys Val Ala Tyr Val Tyr Lys
1               5                   10                  15
Pro Asn Asn

<210> 562
<211> 14
<212> PRT
<213> Artificial Sequence

<220>
<223> Non-natural peptide

<400> 562
Tyr Ala Arg Phe Gln Ser Gln Thr Thr Leu Lys Gln Lys Thr
1               5                   10

<210> 563
<211> 14
<212> PRT
<213> Homo sapiens

<220>
<223> Peptide eluted from the cell line C1R

<400> 563
Glu Ala Leu Ile His Gln Leu Ile Asn Pro Tyr Val Leu Ser
1               5                   10

<210> 564
<211> 15
<212> PRT
<213> Homo sapiens

<400> 564
Val Asn Leu Leu Ile Phe His Ile Asn Gly Lys Ile Ile Lys Asn
1               5                   10                  15